(19) Europäisches Patentamt
European Patent Office
Office européen des brevets

(11) EP 4 223 278 A2

(12) **EUROPEAN PATENT APPLICATION**

(43) Date of publication:
**09.08.2023 Bulletin 2023/32**

(21) Application number: **23166332.9**

(22) Date of filing: **17.07.2019**

(51) International Patent Classification (IPC):
***A61K 8/88*** *(2006.01)* ***A61Q 5/06*** *(2006.01)*
***A61Q 5/08*** *(2006.01)* ***C08G 69/26*** *(2006.01)*
***C08G 69/28*** *(2006.01)* ***C08G 73/10*** *(2006.01)*
***C08L 79/08*** *(2006.01)*

(52) Cooperative Patent Classification (CPC):
**C08G 73/10; A61K 8/84; A61K 8/88; A61Q 5/065; A61Q 5/08; A61Q 5/10; C08G 69/26; C08G 69/28**

(84) Designated Contracting States:
**AL AT BE BG CH CY CZ DE DK EE ES FI FR GR HR HU IE IS IT LI LT LU LV MC MK MT NL NO PL PT RO RS SE SI SK SM TR**

(30) Priority: **24.07.2018 GB 201812032**

(62) Document number(s) of the earlier application(s) in accordance with Art. 76 EPC:
**19742339.5 / 3 826 607**

(71) Applicant: **Henkel AG & Co. KGaA**
**40589 Düsseldorf (DE)**

(72) Inventors:
- **Hodes, Jing**
  **58093 Hagen (DE)**
- **Kessler-Becker, Daniela**
  **51371 Leverkusen (DE)**
- **Beck, Horst**
  **41470 Neuss (DE)**
- **Brandt, Adrian**
  **45219 Essen (DE)**
- **Kux, Alexander**
  **40789 Monheim (DE)**
- **Nemitz, Ralph**
  **41363 Jüchen (DE)**

Remarks:
This application was filed on 03-04-2023 as a divisional application to the application mentioned under INID code 62.

(54) **AMINE-BASED REACTION PRODUCTS AS FUNCTIONAL ADDITIVES**

(57) The present invention relates to an adhesive composition comprising a condensate reaction product obtainable according to a process comprising: (i) reacting itaconic acid or an ester thereof with a diamine of formula $H_2N(CH_2)_nNH_2$, where n is an integer ranging from 2 to 12, wherein when n is an integer ranging from 3 to 12, the alkyl chain is a straight chain, in a molar ratio of diamine to itaconic acid or ester thereof ranging from 1:1.8 to 1:3.0 to form a first reaction mixture; (ii) heating the first reaction mixture to form a first condensate reaction product in a second reaction mixture; and (iii) optionally hydrolysing the second reaction mixture to form a second condensate reaction product, wherein the first condensate reaction product and the second condensate reaction product each independently has a weight average molecular weight ($M_w$) ranging from 200 to 499 g/mol.

Fig. 1

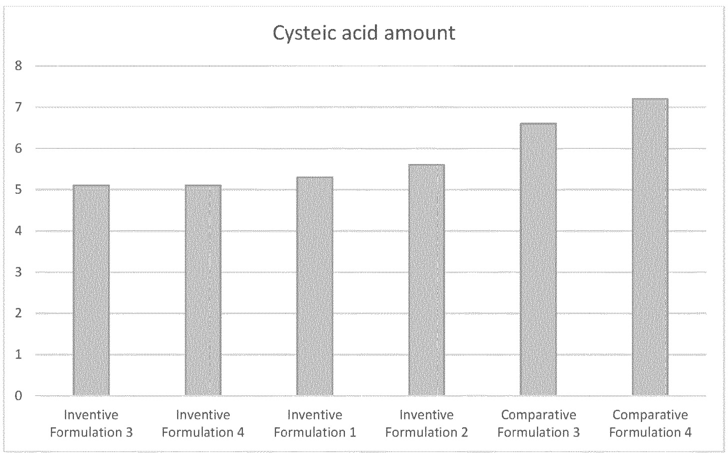


EP 4 223 278 A2

## Description

The present invention relates to adhesive compositions comprising condensate reaction products that are obtainable by two specific processes according to the invention.

Polymers which can be employed as functional additives, e.g., in adhesive compositions, detergent compositions, cosmetic compositions or cleaning compositions are known in the art. Due to the increasing awareness of environmental issues, there exists a need for environmentally friendly, bio-based polymers. The international patent publications WO 2015/164598 A1, WO 2015/164601 A1, WO 2016/040962 A1 and WO 98/22478 A1 disclose such polymers, namely polyimide polymers and pyrrolidinone polymers, respectively.

For certain applications, e.g., adhesive compositions, detergent compositions, cosmetic compositions or cleaning compositions, polymers based on itaconic acid which are water soluble over a broad pH-value range and have a number average molecular weight ($M_n$) of 1.000 to 10.000 g/mol are advantageous. Those polymers - if used as functional additives - have a good stability against other chemicals and different environmental conditions, such as light, heat, exposure to air, especially compared to polymers comprising imide and lactam structures.

For cosmetic applications, it is known to use chelating agents during oxidative hair treatments for dyeing and/or lightening keratinous fibres. In such treatments, the breakdown of melanin pigments and oxidation of hair proteins occurs due to the predominant extremely high alkaline pH value and the presence of oxidizing agents, e.g. hydrogen peroxide or persulfate.

But these processes that take place in and on the hair fibre generally involve the risk of attacking and, in the worst case, partially destroying the hair structure. As a result of the processes, customers are uncertain about the reduced mechanical strength of the hair fibres, a roughening of the surface structure, prevented shine and brittleness of the hair.

The water source used by consumers to wash hair contains calcium and magnesium ions, as well as an undesirable amount of redox metal ions. For example, it is already known that a certain amount of copper and iron is present in human hair. The redox metal ions, especially copper or iron, catalyse the redox reaction with hydrogen peroxide under alkaline conditions and lead to the generation of reactive oxygen species (ROS). These ROS are highly active and react very quickly with hair proteins, which can lead to significant hair damage. Complexing agents such as ethylenediaminetetraacetic acid (EDTA), tetrasodium iminodisuccinate (IDS) and ethylenediamine-N,N'-disuccinic acid (EDDS) are therefore used in blonding agents to mask corresponding metal ions. Today, however, there are repeated discussions about the poor biodegradability of common complexing agents. Another disadvantage of EDTA is that EDTA complex Ca and Mg better than transition metal ions such as Cu or Fe. EDTA is not ideal as a special effective complexing agent for Cu or Fe.

It was an object of the present invention to provide further reaction products based on amine starting materials that are environmentally friendly, sufficiently stable, while retaining good water solubility, and which have advantages when incorporated into products as functional additives.

This object is met by the condensate reaction products of the present invention. The inventors of the present invention have developed two specific processes that result in condensate reaction products according to the present invention. Both processes involve the reaction of itaconic acid or an ester thereof with a diamine. Advantageously, both processes involve the use of a molar excess of the itaconic acid or ester relative to the diamine. The first process involves a condensation reaction. The condensates produced by such a process comprise lactam and/or imide structures. The second process involves the same condensation process followed by a subsequent hydrolysis step. The hydrolysed condensates produced by this second process do not comprise any imide structures.

It has been surprisingly found that, when the condensate reaction products according to the present invention are employed as functional additives in the above-mentioned compositions, they can boost the performance of the respective compositions.

The processes disclosed herein are furthermore advantageous in that they do not employ a catalyst and are thus more cost efficient. Additionally, the methods may either employ no solvent, or a solvent consisting of water, and thus provide the condensate reaction product in a more cost-, time- and labour-efficient manner.

## SUMMARY

In a first aspect, the invention relates to a condensate reaction product obtainable according to a process comprising:

(i) reacting itaconic acid or an ester thereof with a diamine of formula $H_2N(CH_2)_nNH_2$, where n is an integer ranging from 2 to 12, wherein when n is an integer ranging from 3 to 12, the alkyl chain is a straight chain, in a molar ratio of diamine to itaconic acid or ester thereof ranging from 1:1.8 to 1:3.0 to form a first reaction mixture;
(ii) heating the first reaction mixture to form a first condensate reaction product in a second reaction mixture; and
(iii) optionally hydrolysing the second reaction mixture to form a second condensate reaction product,

wherein the first condensate reaction product and the second condensate reaction product each independently has a weight average molecular weight ($M_w$) ranging from 200 to 499 g/mol.

Hereinafter, we interchangeably refer to the first condensate reaction product as the "non-hydrolysed reaction product", and to the second condensate reaction product as the "hydrolysed reaction product". Due to the hydrolysis reaction taking place, the skilled person would understand that the non-hydrolysed reaction product is different to the hydrolysed reaction product.

One of the starting materials in the condensation reaction is itaconic acid or an ester thereof. For ease of reference, we also herein refer to this starting material as the "itaconic-based starting material". The other starting material is a straight-chain diamine of formula $H_2N(CH_2)_nNH_2$, where n is an integer ranging from 2 to 12. Again, for ease of reference, we also herein refer to this starting material as the "diamine starting material".

Itaconic acid may be used as the itaconic-based starting material. Itaconic acid has the following chemical structure:

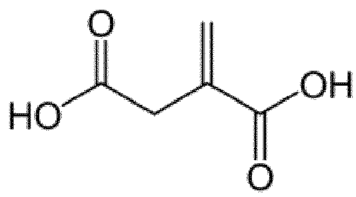

Alternatively, both of the -OH groups on the itaconic acid starting material are esterified, and this ester of itaconic acid is used as the itaconic-based starting material. The ester is preferably the methyl or ethyl ester of itaconic acid, i.e. dimethyl itaconate or diethyl itaconate.

As would be understood by the skilled person, the term "condensate" in the context of the present invention refers to the material resulting from the condensation reaction between the itaconic-based starting material and the diamine starting material. During such a condensation (which typically occurs during the heating step (ii)), either water or an alcohol is produced. Again, as would be understood by the skilled person, when itaconic acid is used as the starting material, water is produced during condensation, whereas when an ester is used, an alcohol is produced (the alcohol corresponding to the ester of the itaconate ester). It is preferable to produce water rather than an alcohol, so for this reason at least, it is preferred that the itaconic-based starting material is itaconic acid.

The diamine used has the formula $H_2N(CH_2)_nNH_2$, where n is an integer ranging from 2 to 12. When n is greater than or equal to 3, the alkyl chain is a straight chain. Preferably, n is an integer ranging from 2 to 8, more preferably from 2 to 6. Thus, the diamine starting material is preferably selected from the group consisting of $H_2N\text{-}CH_2\text{-}CH_2\text{-}NH_2$ (i.e. ethylene diamine), $H_2N$-(*n*-propyl)-$NH_2$ (i.e. trimethylene diamine), $H_2N$-(*n*-butyl)-$NH_2$ (i.e. butamethylene diamine), $H_2N$-(*n*-pentyl)-$NH_2$ (i.e. pentamethylene diamine) and $H_2N$-(*n*-hexyl)-$NH_2$ (i.e. hexamethylene diamine).

Ethylene diamine, pentamethylene diamine and hexamethylene diamine are the most preferred diamine starting materials.

Suitably, the itaconic-based starting material is itaconic acid and the diamine is ethylene diamine. Alternatively, the itaconic-based starting material is itaconic acid and the diamine is pentamethylene diamine. Further alternatively, the itaconic-based starting material is itaconic acid and the diamine is hexamethylene diamine.

Preferably, the molar ratio of diamine to itaconic-based starting material ranges from 1:greater than 1.9, preferably 1 :greaterthan 1.95, more preferably 1:2.0 to 1 :3.0, yet preferably 1:2.0 to 1 :2.5, and most preferably 1:2.0 to 1:2.3.

The distribution and identification of molecular weight peaks, as well as the average molecular weight and polydispersity may be measured via LC-ESI-MS and GPC (as described in detail in the Examples section herein). If not stated otherwise, the weight average molecular weights and the polydispersity values are those determined by GPC against a polyethylene glycol standard, as detailed further in the experimental section.

The first condensate reaction product has a weight average molecular weight ($M_w$) ranging from 200 to 499 g/mol, suitably from 200-450 g/mol, or 250-400 g/mol, or 300-400 g/mol, or 320-380 g/mol. Optionally, the first condensate reaction product and the second condensate reaction product each independently contains no individual compounds having a molecular weight ($M_w$) greater than 1,300 g/mol, and preferably contains no individual compounds having a molecular weight (Mw) greater than 1,250 g/mol (as measured using LC-ESI-MS, as detailed in the experimental section). The first condensate reaction product typically has a polydispersity index ranging from 1 to 1.25, preferably greater than 1 to 1.15, more preferably 1.01 to 1.10, most preferably 1.02 to 1.06.

Where the starting material are itaconic acid and ethylene diamine (in a ratio of 2:1 w/w), the first condensate reaction product may be characterised as having a distribution of peaks with the main peaks in the LC-ESI-MS chromatogram in the region from about 150 m/z to about 600 m/z. By "main peaks" in the context of the present invention is meant the 3 to 5 peaks exhibiting the highest relative abundance. The first condensate reaction product of the present

invention (where the starting material are itaconic acid and ethylene diamine in a ratio of 2:1 w/w), may thus be characterised as exhibiting at least two out of the three peaks in the LC-ESI-MS chromatogram with the highest relative abundance less than 500 g/mol and/or characterised as exhibiting no peaks in the LC-ESI-MS chromatogram above 650 g/mol with relative abundance of at least 10%.

The second condensate reaction product has a weight average molecular weight ($M_w$) ranging from 200 to 499 g/mol, suitably from or 200-450 g/mol, or 250-400 g/mol, or 300-400 g/mol, or 320-380 g/mol. Optionally, the first condensate reaction product and the second condensate reaction product each independently contains no individual compounds having a molecular weight ($M_w$) greater than 1,300 g/mol, and preferably contains no individual compounds having a molecular weight (Mw) greater than 1,250 g/mol (as measured using LC-ESI-MS, as detailed in the experimental section). The second condensate reaction product typically has a polydispersity index ranging from 1 to 1.25, preferably greater than 1 to 1.15, more preferably 1.01 to 1.10, most preferably 1.02 to 1.06.

Where the starting material are itaconic acid and ethylene diamine (in a ratio of 2:1 w/w), the second condensate reaction product may be characterised as having a distribution of peaks with the main peaks in the LC-ESI-MS chromatogram in the region from about 150 m/z to about 600 m/z. As noted above, by "main peaks" in the context of the present invention is meant the 3 to 5 peaks exhibiting the highest relative abundance. The second condensate reaction product of the present invention (where the starting material are itaconic acid and ethylene diamine in a ratio of 2:1 w/w), may thus be characterised as exhibiting at least two out of the three peaks in the LC-ESI-MS chromatogram with the highest relative abundance less than 500 g/mol and/or characterised as exhibiting no peaks in the LC-ESI-MS chromatogram above 650 g/mol with relative abundance of at least 10%.

Typically, the non-hydrolysed condensate reaction product of the present invention comprises one or more compounds with the following chemical structures:

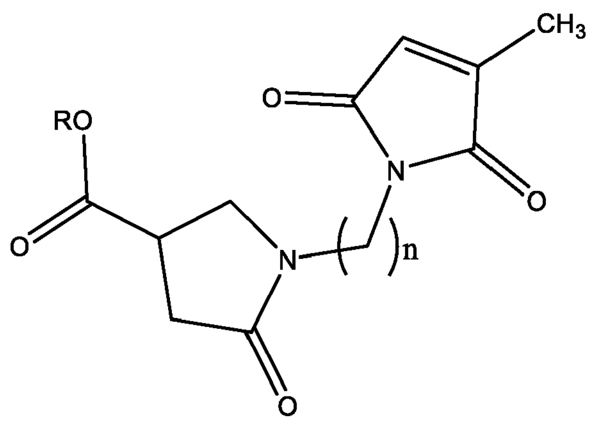

I

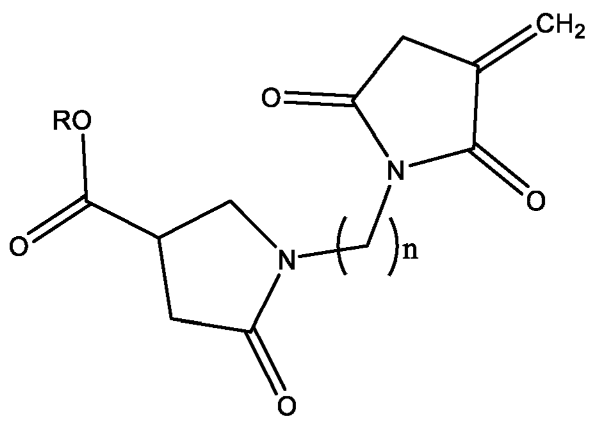

II

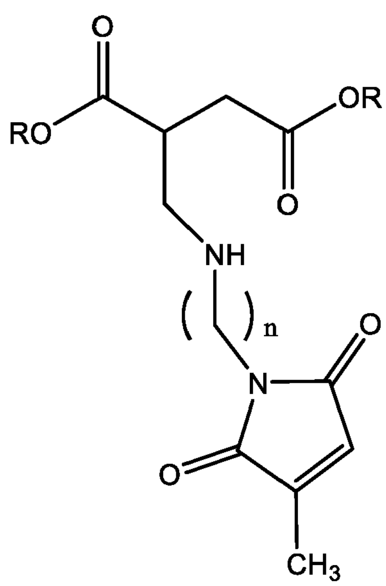

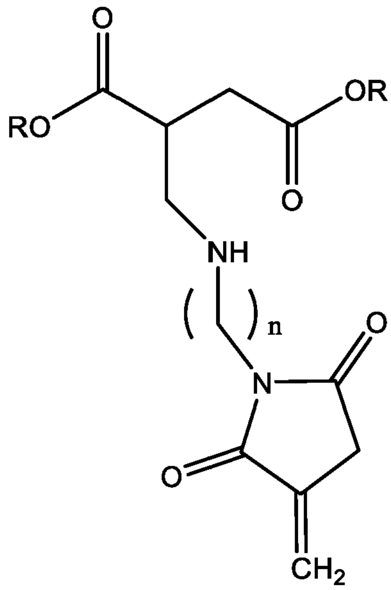

III IV

In all of the above compounds with formula I to IV, n is as defined above in relation to diamine of formula $H_2N(CH_2)_nNH_2$ and all instances of R are either hydrogen or the alkyl group corresponding to the ester of itaconic acid as defined above.

Optionally, the non-hydrolysed condensate reaction product according to the present invention comprises a mixture of at least two of compounds I, II, III and IV; optionally a mixture of at least three of compounds I, II, III and IV. This non-hydrolysed reaction product may comprise a mixture of all compounds I, II, III and IV. The skilled person understands that compounds I and II are isomers, as are compounds III and IV (i.e. in both cases, the position of the double bond in the imide ring changes position, as do the positions of the relevant hydrogen atoms).

When the starting materials are itaconic acid and ethylene diamine, the non-hydrolysed condensate reaction product of the present invention typically comprises one or more compounds with the following chemical structures:

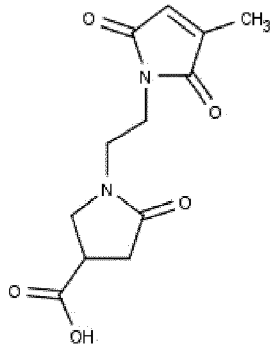

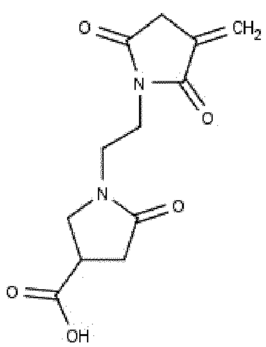

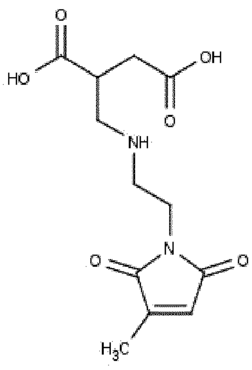

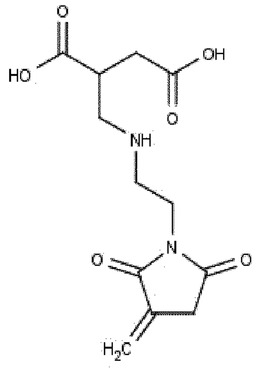

IA IIA IIIA IVA

Compounds IA and IIA have a molecular weight of 266 g/mol and condensate compounds IIIA and IVA have a molecular weight of 284 g/mol. Optionally, this non-hydrolysed condensate reaction product (based on itaconic acid/ethylene diamine) according to the present invention comprises a mixture of at least two of compounds IA, IIA, IIIA and IVA; optionally a mixture of at least three of compounds IA, IIA, IIIA and IVA. This non-hydrolysed reaction product may comprise a mixture of all compounds IA, IIA, IIIA and IVA.

The compounds I to IV, including IA to IVA, are novel and inventive individually (as well as the processes disclosed herein being novel and inventive), so a further aspect of the present invention is the provision of a composition comprising one or more of compounds I to IV, preferably two or more of compounds I to IV. Suitably, the invention relates to a composition comprising three or more, or all, of compounds I to IV. A further aspect of the present invention is also

the provision of a composition comprising one or more of compounds IA to IVA, preferably two or more of compounds IA to IVA. Suitably, the invention relates to a composition comprising three or more, or all, of compounds IA to IVA.

Typically, the hydrolysed condensate reaction product according to the present invention comprises hydrolysed condensate compound V in the free base form shown below or as a salt thereof. As would be understood by the skilled person, the cation of the salt corresponds to the base used in the hydrolysis. For example, when an alkali metal base is used in the hydrolysis, the salt is an alkali metal salt of compound V, such as the sodium or potassium salt. Alternatively, when an alkaline earth metal base is used in the hydrolysis, the salt is an alkaline earth metal salt of compound V, such as the magnesium or calcium salt. Preferably, the salt is the sodium or potassium salt of compound V, more preferably the sodium salt.

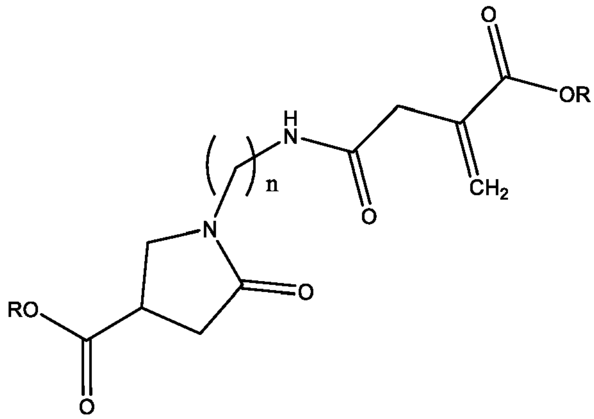

V

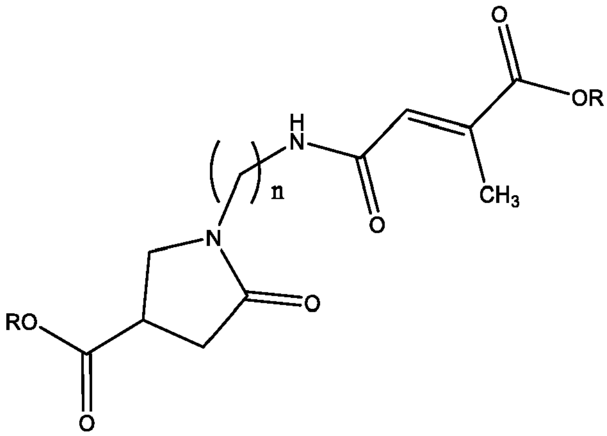

VI

In both of the above compounds with formula V and VI, n is as defined above in relation to diamine of formula $H_2N(CH_2)_nNH_2$ and all instances of R are either hydrogen or the alkyl group corresponding to the ester of itaconic acid as defined above.

The skilled person understands that compounds V and VI are isomers (i.e. the position of the double bond on the acid structural unit changes, as do the positions of the relevant hydrogen atoms). One or both of compounds V and VI may be present in the second reaction product. Typically, both V and VI are present.

When the starting materials are itaconic acid and ethylene diamine, the hydrolysed condensate reaction product according to the present invention typically comprises compounds VA and/or VIA (typically compounds VA and VIA) in the free base form shown below or as a salt thereof. Preferably, the salt is the sodium or potassium salt of compound V, more preferably the sodium salt.

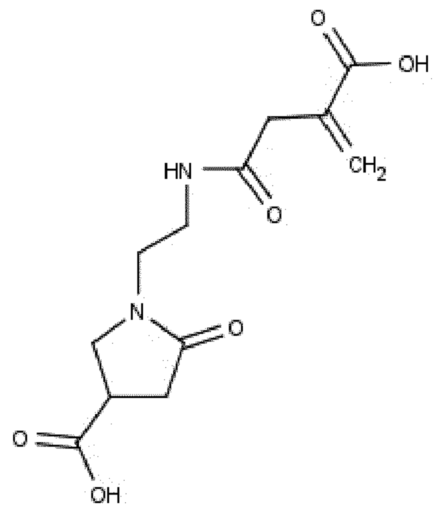

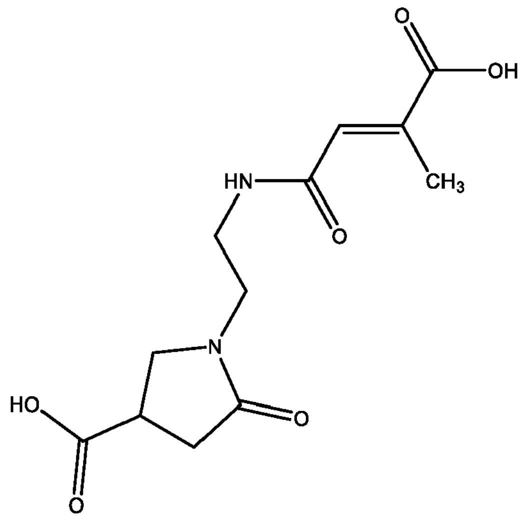

VA VIA

The compounds V and VI, including VA and VIA, are novel and inventive individually (as well as the processes disclosed herein being novel and inventive), so a further aspect of the present invention is the provision of a composition comprising one or both of compounds V and IV. A further aspect of the present invention is also the provision of a composition comprising one or both of compounds VA and VIA.

Optionally, the compounds of formula VII and VIII are present in the second reaction product:

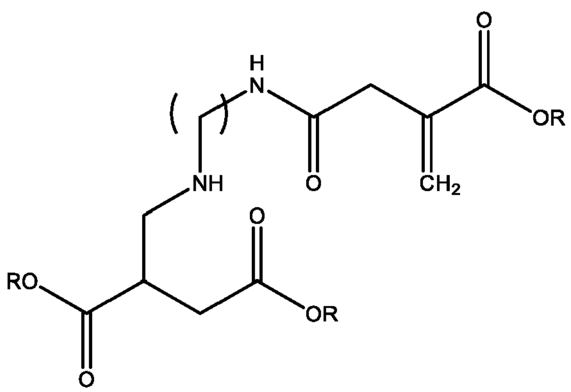

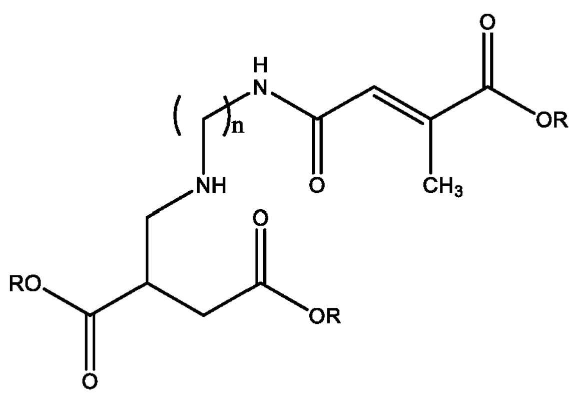

VII VIII

In both of the above compounds with formula VII and VIII, n is as defined above in relation to diamine of formula $H_2N(CH_2)_nNH_2$ and all instances of R are either hydrogen or the alkyl group corresponding to the ester of itaconic acid as defined above.

One or both of compounds VII and VIII may be present in the second reaction product. Typically, both V and VI are present.

When the starting materials are itaconic acid and ethylene diamine, the hydrolysed condensate reaction product according to the present invention typically comprises compounds VIIA and/or VIIIA (typically compounds VIIA and VIIIA) in the free base form shown below or as a salt thereof. Preferably, the salt is the sodium or potassium salt of compound V, more preferably the sodium salt.

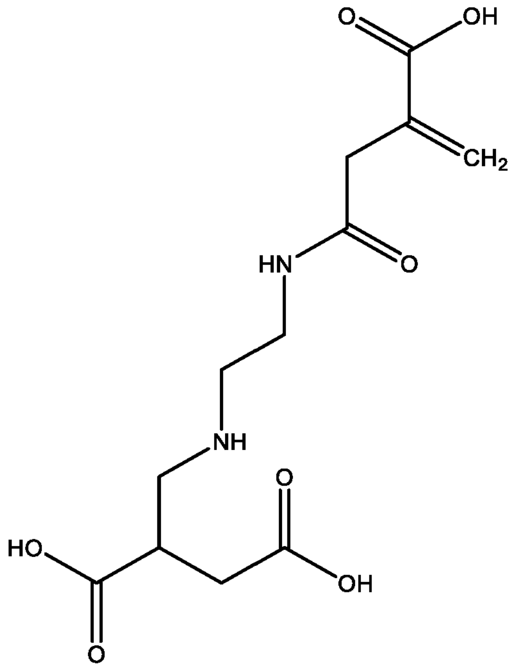

VIIA

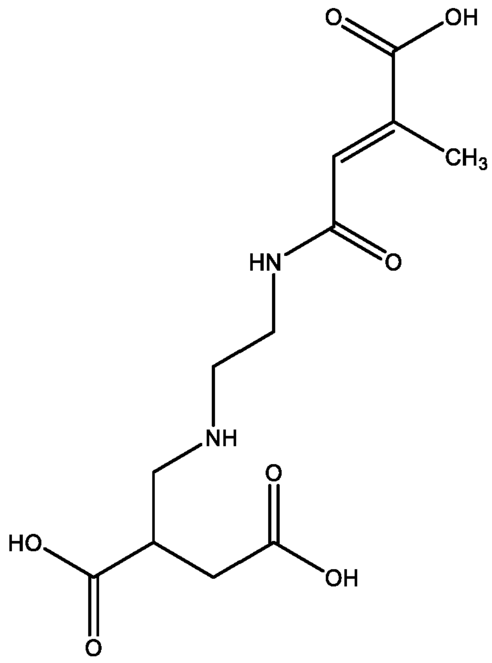

VIIIA

The compounds VII and VIII, including VIIA and VillA, are novel and inventive individually (as well as the processes disclosed herein being novel and inventive), so a further aspect of the present invention is the provision of a composition comprising one or both of compounds VII and VIII. A further aspect of the present invention is also the provision of a composition comprising one or both of compounds VIIA and VIIIA.

The process also forms another aspect of the present invention, i.e. the invention relates to a process for preparing a condensate reaction product comprising:

(i) reacting itaconic acid or an ester thereof with a diamine of formula $H_2N(CH_2)_nNH_2$, where n is an integer ranging from 2 to 12, wherein when n is an integer ranging from 3 to 12, the alkyl chain is a straight chain, in a molar ratio of diamine to itaconic acid or ester thereof ranging from 1:1.8 to 1:3.0 to form a first reaction mixture;
(ii) heating the first reaction mixture to form a first condensate reaction product in a second reaction mixture; and
(iii) optionally hydrolysing the second reaction mixture to form a second condensate reaction product,

wherein the first condensate reaction product and the second condensate reaction product each independently has a weight average molecular weight ($M_w$) ranging from 200 to 499 g/mol.

The features herein disclosed in relation to the products obtainable by the process equally apply to the process. The present invention also provides the products obtained by the processes disclosed herein.

A further aspect of the invention relates to a first condensate reaction product obtainable according to a process comprising:

(i) reacting itaconic acid or an ester thereof with a diamine of formula $H_2N(CH_2)_nNH_2$, where n is an integer ranging from 2 to 12, wherein when n is an integer ranging from 3 to 12, the alkyl chain is a straight chain; and
(ii) heating the first reaction mixture to form the first condensate reaction product

wherein the first condensate reaction product comprises one or more of the following compounds:

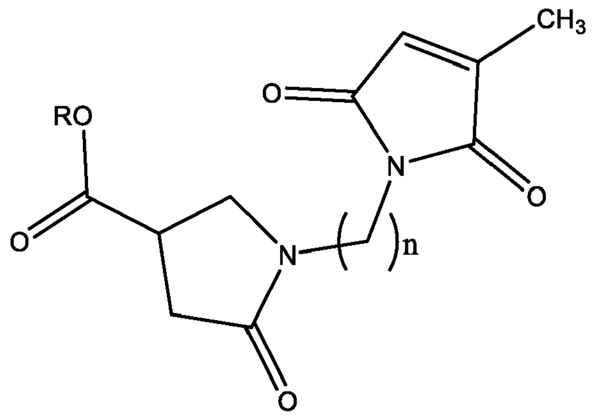

I

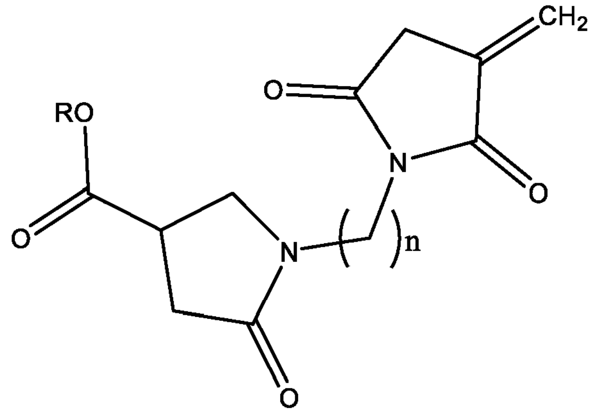

II

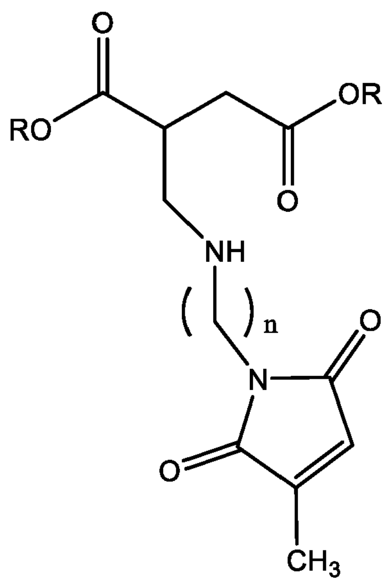

III

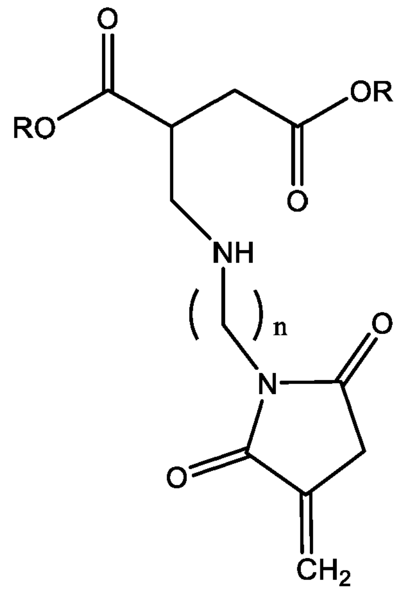

IV

The itaconic-based starting material and the diamine are as disclosed elsewhere herein. Suitably, the starting materials in step (i) are reacted in a molar ratio of diamine to itaconic acid or ester thereof ranging from 1:1.8 to 1:3.0, preferably from 1:1.9 to 1:3.0, more preferably from 1:1.95 to 1:3.0; yet more preferably from 1:2.0 to 1:3.0 and most preferably from 1:2.0 to 1:2.3. The first condensate reaction product typically has a weight average molecular weight ($M_w$) ranging from 200 to 499 g/mol, suitably from 200-450 g/mol, or 250-400 g/mol, or 300-400 g/mol, or 320-380 g/mol. Optionally, the first condensate reaction product contains no individual compounds having a molecular weight ($M_w$) greater than 1,300 g/mol, and preferably contains no individual compounds having a molecular weight (Mw) greater than 1,250 g/mol (as measured using LC-ESI-MS, as detailed in the experimental section). The first condensate reaction product typically has a polydispersity index ranging from 1 to 1.25, preferably greater than 1 to 1.15, more preferably 1.01 to 1.10, most preferably 1.02 to 1.06.

As noted above, the diamine is as disclosed herein. More specifically, the diamine used has the formula $H_2N(CH_2)_nNH_2$, where n is an integer ranging from 2 to 12. When n is greater than or equal to 3, the alkyl chain is a straight chain. Preferably, n is an integer ranging from 2 to 8, more preferably from 2 to 6. Thus, the diamine starting material is preferably selected from the group consisting of $H_2N$-$CH_2$-$CH_2$-$NH_2$ (i.e. ethylene diamine), $H_2N$-(*n*-propyl)-$NH_2$ (i.e. trimethylene diamine), $H_2N$-(*n*-butyl)-$NH_2$ (i.e. butamethylene diamine), $H_2N$-(*n*-pentyl)-$NH_2$ (i.e. pentamethylene diamine) and $H_2N$-(*n*-hexyl)-$NH_2$ (i.e. hexamethylene diamine). Ethylene diamine, pentamethylene diamine and hexamethylene diamine are the most preferred diamine starting materials.

Suitably, the itaconic-based starting material is itaconic acid and the diamine is ethylene diamine. Alternatively, the itaconic-based starting material is itaconic acid and the diamine is pentamethylene diamine. Further alternatively, the itaconic-based starting material is itaconic acid and the diamine is hexamethylene diamine.

When itaconic acid is reacted with ethylene diamine, the compounds of formula IA to IVA have the following structures:

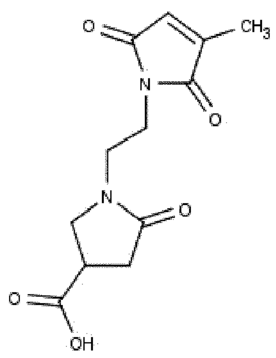
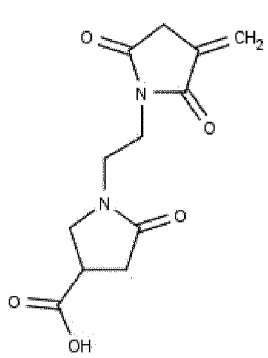
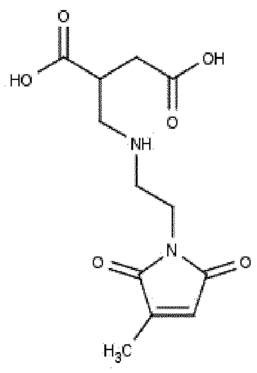
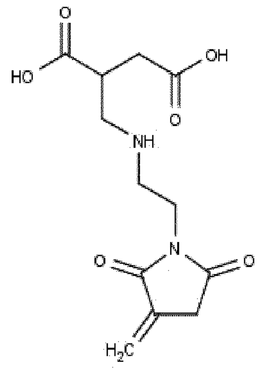

IA IIA IIIA IVA

The process also forms another aspect of the present invention, i.e. the invention relates to a process for preparing a first condensate reaction product comprising:

(i) reacting itaconic acid or an ester thereof with a diamine of formula $H_2N(CH_2)_nNH_2$, where n is an integer ranging from 2 to 12, wherein when n is an integer ranging from 3 to 12, the alkyl chain is a straight chain; and
(ii) heating the first reaction mixture to form the first condensate reaction product

wherein the first condensate reaction product comprises one or more of the following compounds:

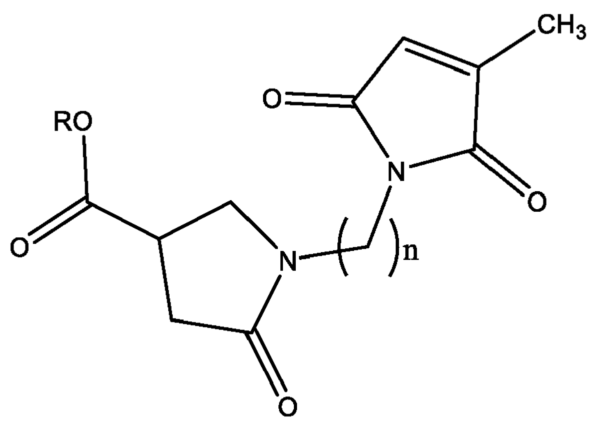
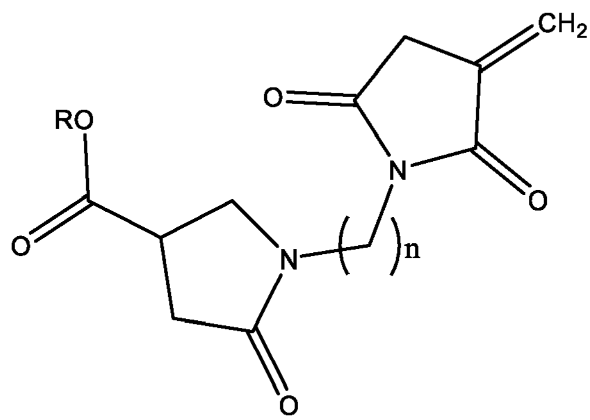

I II

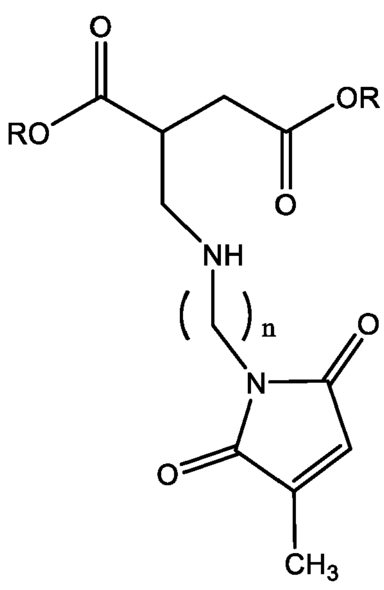

III

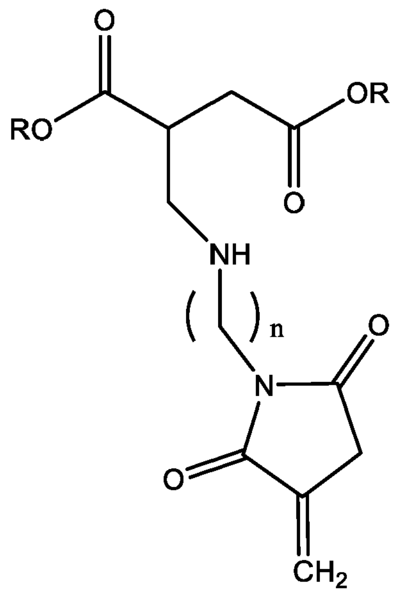

IV

The features herein disclosed in relation to the products obtainable by the process equally apply to the process. The present invention also provides the products obtained by the processes disclosed herein.

A further aspect of the invention relates to a second condensate reaction product obtainable according to a process comprising:

(i) reacting itaconic acid or an ester thereof with a diamine of formula $H_2N(CH_2)_nNH_2$, where n is an integer ranging from 2 to 12, wherein when n is an integer ranging from 3 to 12, the alkyl chain is a straight chain;
(ii) heating the first reaction mixture to form a first condensate reaction product; and
(iii) hydrolysing the product of step (ii) to form the second condensate reaction product,

wherein the second condensate reaction product comprises the following compounds or a salt thereof:

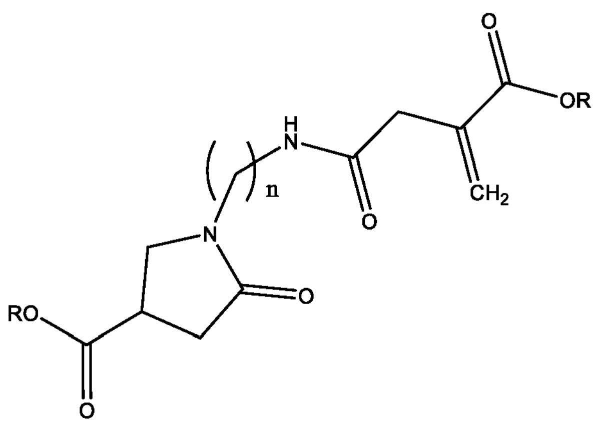

V

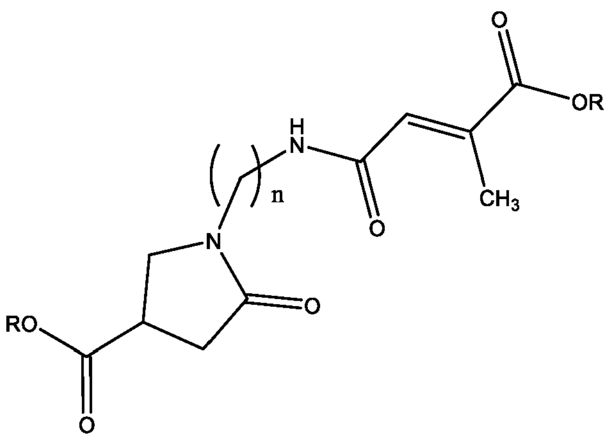

VI

The itaconic-based starting material and the diamine are as disclosed elsewhere herein. Suitably, the starting materials in step (i) are reacted in a molar ratio of diamine to itaconic acid or ester thereof ranging from 1:1.8 to 1:3.0, preferably from 1:1.9 to 1:3.0, more preferably from 1:1.95 to 1:3.0; yet more preferably from 1:2.0 to 1:3.0 and most preferably from 1:2.0 to 1:2.3. The first condensate reaction product typically has a weight average molecular weight ($M_w$) ranging from either 200 to 499 g/mol, suitably from 200-450 g/mol, or 250-400 g/mol, or 300-400 g/mol, or 320-380

g/mol. Optionally, the first condensate reaction product contains no individual compounds having a molecular weight ($M_w$) greater than 1,300 g/mol, and preferably contains no individual compounds having a molecular weight (Mw) greater than 1,250 g/mol (as measured using LC-ESI-MS, as detailed in the experimental section). The first condensate reaction product typically has a polydispersity index ranging from 1 to 1.25, preferably greater than 1 to 1.15, more preferably 1.01 to 1.10, most preferably 1.02 to 1.06.

As noted above, the diamine is as disclosed herein. More specifically, the diamine used has the formula $H_2N(CH_2)_nNH_2$, where n is an integer ranging from 2 to 12. When n is greater than or equal to 3, the alkyl chain is a straight chain. Preferably, n is an integer ranging from 2 to 8, more preferably from 2 to 6. Thus, the diamine starting material is preferably selected from the group consisting of $H_2N$-$CH_2$-$CH_2$-$NH_2$ (i.e. ethylene diamine), $H_2N$-(*n*-propyl)-$NH_2$ (i.e. trimethylene diamine), $H_2N$-(*n*-butyl)-$NH_2$ (i.e. butamethylene diamine), $H_2N$-(*n*-pentyl)-$NH_2$ (i.e. pentamethylene diamine) and $H_2N$-(*n*-hexyl)-$NH_2$ (i.e. hexamethylene diamine). Ethylene diamine, pentamethylene diamine and hexamethylene diamine are the most preferred diamine starting materials.

Suitably, the itaconic-based starting material is itaconic acid and the diamine is ethylene diamine. Alternatively, the itaconic-based starting material is itaconic acid and the diamine is pentamethylene diamine. Further alternatively, the itaconic-based starting material is itaconic acid and the diamine is hexamethylene diamine.

When itaconic acid is reacted with ethylene diamine, the compound of formula V has the following structure VA and the compound of VI has the following structure VIA:

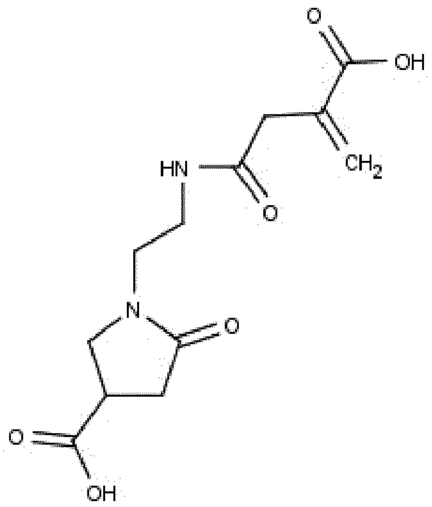

VA

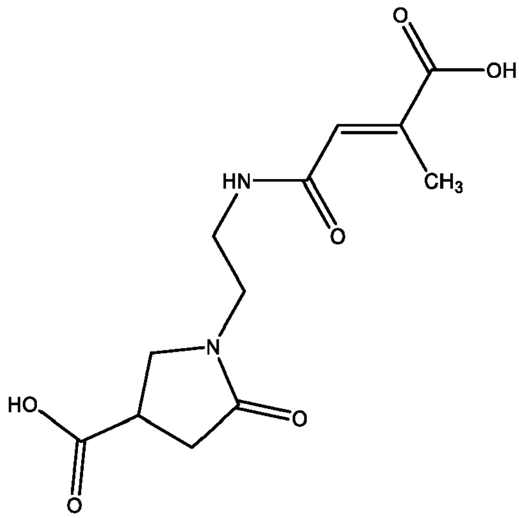

VIA

Further, the compound of formula VII has the following structure VIIA and the compound of VIII has the following structure VIIIA:

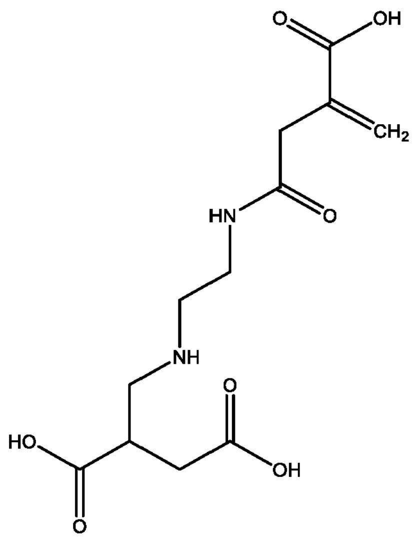

VIIA

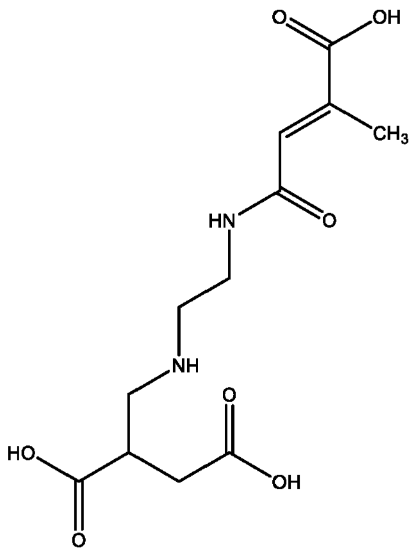

VIIIA

The process also forms another aspect of the present invention, i.e. the invention relates to a process for preparing a second condensate reaction product comprising:

(i) reacting itaconic acid or an ester thereof with a diamine of formula $H_2N(CH_2)_nNH_2$, where n is an integer ranging from 2 to 12, wherein when n is an integer ranging from 3 to 12, the alkyl chain is a straight chain;
(ii) heating the first reaction mixture to form a first condensate reaction product; and
(iii) hydrolysing the product of step (ii) to form the second condensate reaction product,

wherein the second condensate reaction product comprises the following compounds or a salt thereof:

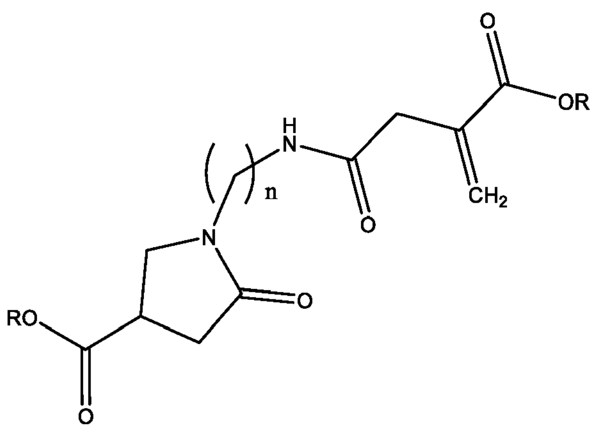

V

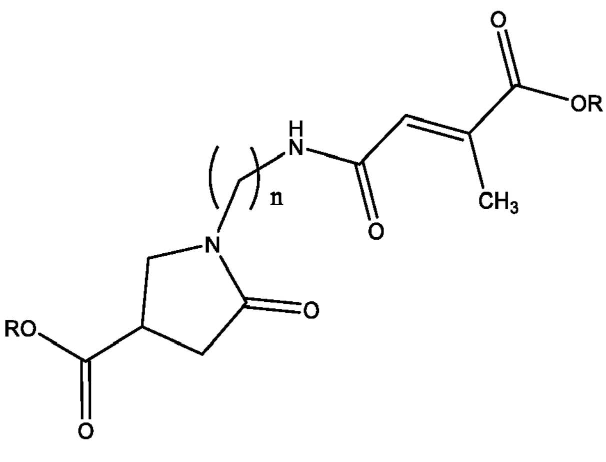

VI

The features herein disclosed in relation to the products obtainable by the process equally apply to the process. The present invention also provides the products obtained by the processes disclosed herein.

Steps (i) and (ii) and/or (iii) as disclosed herein may either be carried out in the absence of a solvent, or in the presence of a solvent. Suitably, the whole reaction (i.e. steps (i) and (ii) where no hydrolysis is carried out, or all of steps (i) to (iii) if hydrolysis is carried out) are carried out either in the absence of a solvent, or in a solvent of water, an alcohol or mixtures thereof. When a solvent is used, either water is the solvent when the starting material is itaconic acid. Alternatively, when an ester of itaconic acid is used, the solvent is the alcohol corresponding to the ester (for example, methanol is used when dimethyl itaconate is the starting material, and ethanol is used when diethyl itaconate is the

starting material). Preferably, no solvent is used.

When the process is carried out without hydrolysis, the first condensate reaction product may be isolated as a solid. Advantageously, when no solvent is used in the process, distillation of the condensed liquid throughout the process results in a solid product upon reaction completion. Alternatively, if the product is present in a solution, the isolation typically comprises removal of the liquid in the reaction mass (suitably by distillation) followed by cooling and optionally drying. The first condensate reaction product may be left in solution and used as such in onward applications, for example as an additive for laundry, adhesive and cosmetic applications.

When the process comprises hydrolysis step (iii), the product of step (ii) may be isolated as a solid before the hydrolysis step (by any of the methods disclosed herein). Preferably, the product of step (ii) is not isolated before the hydrolysis step, so the reaction mass from step (ii) is used directly in hydrolysis step (iii). The second condensate reaction product may then be isolated. Advantageously, when no solvent is used in the process, distillation of the condensed liquid throughout the process results in a solid product upon reaction completion. Alternatively, if the product is present in a solution, the isolation typically comprises removal of the liquid in the reaction mass (suitably by distillation) followed by cooling and optionally drying. The second condensate reaction product may be left in solution and used as such in onward applications, for example as an additive for laundry, adhesive and cosmetic applications.

The present invention also provides the use of a condensate reaction product as disclosed herein as a functional additive. Both the first and second condensate reaction products can be used as functional additives.

The present invention also provides a detergent composition comprising a condensate reaction product as disclosed herein. Both the first and second condensate reaction products can be used as functional additives in detergent compositions.

The present invention also provides a cleaning composition comprising a condensate reaction product as disclosed herein. Both the first and second condensate reaction products can be used as functional additives in cleaning compositions.

The present invention also provides an adhesive composition comprising a condensate reaction product as disclosed herein. Both the first and second condensate reaction products can be used as functional additives in adhesive compositions.

The present invention also provides a cosmetic composition comprising a condensate reaction product as disclosed herein. Both the first and second condensate reaction products can be used as functional additives in cosmetic compositions.

Suitably, the cosmetic composition according to the present invention is in the form of a bleaching powder. Thus, according to another aspect of the invention, there is provided a bleaching powder comprising the first reaction product as disclosed herein, and typically at least one oxidising agent.

According to another aspect of the invention, there is provided a bleaching powder comprising the second reaction product as disclosed herein, and typically at least one oxidising agent. Suitable bleaching powders are disclosed below.

Suitably, the cosmetic composition according to the present invention is in the form of a bleaching paste. Typically, such a cosmetic composition further comprises at least one oxidising agent. Thus, according to another aspect of the invention, there is provided a bleaching paste comprising the first reaction product as disclosed herein, and typically at least one oxidising agent. According to another aspect of the invention, there is provided a bleaching paste comprising the second reaction product as disclosed herein, and typically at least one oxidising agent. Suitable bleaching pastes are further disclosed herein.

Methods for the oxidative lightening and/or dyeing of keratinous fibers are also provided herein. Such methods involve the use of the first or second condensate reaction product disclosed herein, suitably as an additive in a bleaching powder or as an additive in a bleaching paste. Suitable methods are further disclosed herein.

There is also provided by the present invention a multi-component packaging unit (kit-of-parts) for lightening keratin fibres, in particular human hair, containing at least two components packaged separately from one another. Typically, one of the components is one of the bleaching powders disclosed herein or one of the bleaching pastes disclosed herein. Suitable kits are further disclosed herein.

There is further provided by the present invention, a multi-component packaging unit (kit-of-parts) for changing the colour of keratin fibres, in particular human hair, containing at least three components packaged separately from one another. Typically, one of the components is one of the bleaching powders disclosed herein or one of the bleaching pastes disclosed herein. Suitable kits are further disclosed herein.

## BRIEF DESCRIPTION OF THE FIGURES

Figure 1 illustrates the advantages of exemplary reaction products of the present invention with reference to cysteic

acid analysis

Figure 2 illustrates the advantages of exemplary reaction products of the present invention with reference to colour difference analysis

Figure 3 illustrates the advantages of exemplary reaction products of the present invention with reference to lightening effect analysis

Figure 4 illustrates the advantages of exemplary reaction products of the present invention with reference to E-modulus difference analysis

Figure 5 illustrates the advantages of exemplary reaction products of the present invention with reference to stress break analysis

## DETAILED DESCRIPTION

The following detailed description is merely exemplary in nature and is not intended to limit the disclosure or the application and uses of the subject matter as described herein. Furthermore, there is no intention to be bound by any theory presented in the preceding background or the following detailed description.

The present invention generally relates to condensate reaction products obtainable according to a process comprising:

(i) reacting itaconic acid or an ester thereof with a diamine of formula $H_2N(CH_2)_nNH_2$, where n is an integer ranging from 2 to 12, wherein when n is an integer ranging from 3 to 12, the alkyl chain is a straight chain, in a molar ratio of diamine to itaconic acid or ester thereof ranging from 1:1.8 to 1:3.0 to form a first reaction mixture; and

(ii) heating the first reaction mixture to form a first condensate reaction product.

The product of step (ii) may be used in onward applications as such or may be hydrolysed to form a second condensate reaction product, which may also be used as such in onward applications. The inventors have surprisingly found that the use of the specific starting materials, and a particular ratio of those starting materials, results in reaction products that are particularly useful as functional additives in detergent, cosmetic, cleaning or adhesive compositions. In particular, the reaction products are unexpectedly advantageous when incorporated in cosmetic compositions.

From a sustainability perspective, the substances based on itaconic acid as the itaconic-based starting material have the advantage that they consist of >50wt%, in some instances >80wt%, bio-based raw materials. The carbon-based bio determination is also high for the raw materials used in the process (for example, in the case of itaconic acid and pentamethylene diamine, the bio content in terms of carbon is 100%). The skilled person can determine the bio-based content of the products based on the raw materials used in the process. The standard ASTM D6866 may be used for such a determination.

A further advantage of the products according to the invention are that they may be produced without the use of a solvent in the production. Alternatively, if a solvent is used, the solvent may beneficially be water. These features of the production are particularly advantageous from an environmental perspective, as well as a cost perspective, and enable the reaction to take place without the need for extensive clean-up of the production apparatus. As will be evident from the reaction details disclosed herein, no catalysts are used in the process according to the invention. Further, the production of the reaction products according to the present invention is simple and requires no intensive work-up of the products. For example, particularly advantageously, the processes of the present invention do not require any recrystallisation steps and filtration steps in order to purify and/or isolate the products.

The use of the itaconic-based starting material and the diamine starting material result in reaction products made up of mixtures of compounds. The major components of the mixtures are relatively short-chain compounds, as is reflected in the average molecular weight of the reaction products. Typically, the first condensate reaction product and the second condensate reaction product each independently has a weight average molecular weight ($M_w$) ranging from 200 to 499 g/mol.

The itaconic-based starting material and the diamine starting material are described in more detail herein. It will also be understood by the skilled person that itaconic anhydride may be used as the itaconic-based starting material. The preferred itaconic-based starting material is itaconic acid and the preferred diamines are those with 2, 5 and 6 methylene units between the amine moieties.

The condensate reaction products (first and second) of the present invention comprise a mixture of compounds, including one or more condensate compounds. The or each compound independently comprises from 3 to 20 structural units, each unit based on either the itaconic acid/ester starting material or the diamine starting material. The compounds present in the reaction products contain structural units based solely on the itaconic-based starting material and the diamine starting material: the reaction takes place in the absence of other starting materials, for example in the absence of any other monomers/structural unit-forming materials.

Typically, the first and second condensate reaction products comprise one or more condensate compounds

consisting of three structural units: two structural units being based on the itaconic-based starting material and one structural unit being based on the diamine starting material, the diamine-based structural unit being positioned between the two itaconic-based structural units. The structural units based on the itaconic-based starting material may be either cyclic or acyclic. In the non-hydrolysed product, both the structural units based on the itaconic-based starting material may be cyclic. Alternatively, one of the structural units is cyclic and the other is acyclic. As would be understood by the skilled person, the hydrolysis step may involve ring-opening of one or more cyclic moieties in the non-hydrolysed product.

The non-hydrolysed condensate reaction product typically comprises imide and/or lactam compounds. The hydrolysed condensate reaction product typically comprises no imide compounds. Optionally, the hydrolysed condensate reaction product comprises no imide compounds and no lactam compounds.

The hydrolysed reaction product typically comprises one or more compounds consisting of three structural units: two structural units based on the itaconic-based starting material and the one structural unit based on the diamine starting material, with the structural unit based on the diamine starting material being positioned between the two units based on the itaconic-based starting material. The structural units based on the itaconic-based starting material may be either cyclic or acyclic. Suitably, one of the structural units based on the itaconic-based starting material is cyclic. The structural units based on the itaconic-based starting material may be either cyclic or acyclic. Alternatively, one of the structural units is cyclic and the other is acyclic. As would be understood by the skilled person, the hydrolysis step may involve ring-opening of one or more cyclic moieties in the non-hydrolysed product.

The first condensate reaction product and the second condensate reaction product may also each independently comprise further compounds within the reaction product. Suitably, such compounds independently have from 5 to 15 structural units, preferably from 5 to 13 structural units; more preferably from 5 to 11 structural units. The structural units in the individual compounds may be derived from any combination of ratios of diamine-based and itaconic-based units, but are typically a majority of structural units derived from the itaconic-based starting material and a corresponding minority of structural units derived from the diamine starting material.

The first condensate reaction product may comprise one or more compounds having a ratio of structural units derived from itaconic-based starting material: diamine of 3:2, 4:3, 4:4, 5:4, 4:2, 5:3, 6:5 and 7:6 and mixtures thereof.

The second condensate reaction product may comprise one or more compounds having a ratio of structural units derived from itaconic-based starting material: diamine of 3:2, 4:3, 4:4, 5:4, 4:2, 5:3, 6:5 and 7:6 and mixtures thereof.

Steps (i) and (ii) and/or (iii) as disclosed herein may either be carried out in the absence of a solvent, or in the presence of a solvent. As would be understood by the skilled person, the product of step (ii) is the product of a condensation reaction, and either water or an alcohol will be produced, depending on the nature of the itaconic-based starting material. If the starting material is itaconic acid, water is produced during the condensation. If an ester of itaconic acid is used, the corresponding alcohol will be produced during the condensation. Thus, it is preferred to use either no solvent (in which case, the condensation reaction will give rise to either water or an alcohol in the reaction mass) or a solvent of either water or the alcohol corresponding to the alcohol produced (i.e. methanol if the ester is dimethyl itaconate or ethanol if the ester is diethyl itaconate), or a mixture of water and the corresponding alcohol. For cost and environmental reasons, as well as to simplify reaction clean-up, either no solvent is used, or water is the only solvent. Thus, the preferred itaconic-based starting material is itaconic acid.

In step (i) the itaconic-based starting material is preferably added portion-wise to the reaction vessel containing the diamine, for example in portions of equal weight, more preferably the individual portions are 1/2, 1/3, 1/4, 1/5, 1/6, 1/7 of the total weight of itaconic-based starting material to be added. The skilled person would understand the appropriate approach to the number of portions depending on the process carried out.

The heating in step (ii) may be started with the addition (portion-wise or otherwise) of the itaconic-based starting material or may be started once the complete amount has been added. The hydrolysis reaction mixture may be heated to a temperature between about 100°C and about 250°C, preferably to at least about 120°C, more preferably to at least about 140°C, most preferably to about 160°C to about 200°C. The temperature of the reaction vessel may be increased at a rate of about 1 °C/min, preferably about 2.5°C/min, more preferably about 3°C/min, most preferably about 4°C/min. The heating step may be carried out (preferably with stirring) for a period of time ranging from about 30 minutes to about 24 hours, preferably from 30 minutes to about 4 hours, more preferably for 1 to 4 hours, most preferably from 1 to 2 hours. The reaction in steps (i) and (ii) involves condensation (as disclosed herein, the condensed material being water or an alcohol), and the produced liquid may be removed (for example distilled off) during these steps. Preferably, the process involves removal of the condensed liquid. As disclosed herein, the process of the present invention may be carried out without the use of a solvent.

If hydrolysis is carried out, after the heating step has been completed, the obtained precipitate or product is allowed to cool or actively cooled and the second condensate reaction product may be removed from the reaction vessel starting at a temperature of 100°C or less, preferably 70°C or less, more preferably 50°C or less, most preferably, around 25°C.

As disclosed herein, a preferred process involves the use of no solvent. This advantageous feature of the process may be combined with the removal of the condensed liquid during the condensation reaction, i.e. in the step

for preparing the first condensate reaction product. In this particularly preferred process, the first condensate reaction product is produced as a solid. This is particularly advantageous as no work-up of the reaction mass is needed; once the reaction mass has been cooled, the first condensate reaction product is available as a solid. As disclosed herein, the solid may be used as such as a functional additive or may be used as the starting material in a hydrolysis step according to the present invention.

In the hydrolysis step, the reaction mass comprising the first condensate reaction product is adjusted to a pH greater than 7, suitably ranging from 9 to 11, preferably from 9.5 to 10.5, more preferably to a pH of around 10. The adjustment may involve the use of an alkali metal hydroxide (preferably NaOH or KOH, more preferably NaOH) or an alkaline earth metal hydroxide (suitably $Ca(OH)_2$ or $Mg(OH)_2$). As disclosed herein, the second condensate reaction product may be in free base form or in the form of a salt thereof. The cation of the salt corresponds to the base used in the hydrolysis step.

To isolate the second condensate reaction product, liquid may be distilled off and the product dried (suitably in a vacuum). The drying may take place at an elevated temperature of around 50°C to 90°C, preferably 60°C to 90°C, more preferably around 80°C.

An aqueous solution of 25 wt.-% of the non-hydrolysed condensate reaction product in water, based on the total weight of the aqueous solution, typically has a pH-value of 4 to 5. An aqueous solution of 25 wt.-% of the hydrolysed condensate reaction product in water, based on the total weight of the aqueous solution, typically has a pH-value of 9 to 11.

The processes according to the invention lead to water soluble condensate reaction products. In a further preferred embodiment, the condensate reaction products are water soluble. As used herein "water soluble" means that the solubility at 20°C in pure water of pH 7 is at least 50 g/L, preferably above 100 g/L, most preferably above 300 g/L, and may for example be as high as or even above 500 g/L, 600 g/L, 700 g/L or 750 g/l. Solubility may for example be measured according to the OECD Guidelines for the Testing of Chemicals, Section 1, Test No. 105: Water Solubility; adopted by the Council on 27th July 1995.

"About", as used herein in relation to a numerical value, means said numerical value ±5%. About 140°C thus relates to a temperature in the range of 133-147°C.

The compositions of the invention including the condensate reaction products disclosed herein, i.e., the detergent, cosmetic, cleaning or adhesive composition may comprise further components typical for such compositions. Accordingly, the additional components of said compositions are not particularly limited as long as the components do not negatively interact with the condensate reaction products, e.g., undergo a chemical reaction and precipitate, with the exception of adhesive compositions where a reaction with further components may be desired. In a preferred embodiment, all of the foregoing compositions are aqueous compositions. In preferred embodiments the detergent, cosmetic and cleaning compositions further comprise at least one surfactant and/or perfume.

In a still further aspect, the invention encompasses the use of the condensate reaction product according to the invention as a functional additive, preferably as an enzyme activity booster and/or chelating agent for detergent compositions, cosmetic compositions, adhesive composition and/or cleaning compositions, more preferably for improving the cleaning performance of detergent compositions, cosmetic compositions and cleaning compositions or as an adhesion promoter or filler material in adhesive compositions. Also within the scope of the invention are the respective detergent, cosmetic, cleaning and adhesive compositions that comprise the condensate reaction products of the invention.

Detergent composition comprising the reaction product according to the invention, preferably contain the product in an amount of 0.1 to 10 wt.-%, more preferably 0.5 to 5 wt.-%, even more preferred 0.8 to 3 wt.-%, most preferred 1 to 1.5 wt.-%, based on the total weight of the detergent composition.

Cleaning compositions comprising the condensate reaction products according to the invention contain the condensate reaction product preferably in an amount of 0.1 to 10 wt.-%, more preferably 1 to 5 wt.-%, even more preferred 1 to 3 wt.-%, most preferred 1 to 1.5 wt.-%, based on the total weight of the cleaning composition.

Adhesive compositions comprising the condensate reaction products according to the invention preferably contain the condensate reaction product in an amount of 0.1 to 80 wt.-%, 5 to 70 wt.-%, 10 to 60 wt.-%, or 15 to 50 wt.-%, based on the total weight of the adhesive composition. Alternatively and advantageously, the condensate reaction products are incorporated in smaller amounts as additives in adhesive compositions (rather than the main component of such compositions). In this case, the adhesive compositions preferably contain from 0.1 to 20wt%, more preferably from 0.1 to 10wt% of the condensate reaction product, based on the total weight of the adhesive composition.

Cosmetic compositions comprising the condensate reaction products according to the invention, contain the condensate reaction product preferably in an amount of at least 0.001wt%. Suitably, the condensate reaction product is present in an amount ranging from 0.1 to 10 wt.-%, preferably from 0.5 to 10 wt.-%, more preferably 1 to 5 wt.-%, even more preferred 1 to 3 wt.-%, yet more preferred 1 to 2 wt.-%, most preferred 1.4 to 1.8 wt.-% based on the total weight of the cosmetic composition. In one aspect of the invention, there is provided a cosmetic composition comprising the non-hydrolysed condensate reaction product defined herein. In another aspect of the invention, there is provided a cosmetic composition comprising the hydrolysed condensate reaction product defined herein. The non-hydrolysed and/or the hydrolysed condensate reaction product may be provided in the form of a solid (typically in powder form) or in the

form of an aqueous solution. If provided in the form of an aqueous solution, the non-hydrolysed condensate reaction product typically has a pH ranging from 3 to 5, and the hydrolysed condensate reaction product typically has a pH ranging from 9 to 11. Suitable cosmetic compositions in which the condensate reaction products may be incorporated include compositions for the oxidative lightening and/or dyeing of keratinous fibres (in particular human hair), shampoos, hair conditioners, hair styling products and body cleansers (such as shower gels and liquid soaps). Such cosmetic compositions may also comprise suitable additives and/or additional components as would be found in conventional compositions. The skilled person is able to select the most appropriate additives and/or additional components based on the nature of the cosmetic composition.

The inventors have surprisingly found that the condensate reaction products of the present invention exhibit beneficial properties when incorporated into hair products for use in the oxidative lightening and/or dyeing of keratinous fibres - both in terms of their use as chelating agents in suitable hair compositions, and also in terms of mechanical properties exhibited by the treated hair. Without wishing to be bound by theory, the inventors of the present invention believe that the condensate reaction products can be used to complex the copper or iron ions in hair. During hair dyeing and bleaching, significantly less reactive oxygen species (ROS) were detected compared to experiments with conventional complexing agents. Thus, the inventors believe that the advantageous chelating properties of the condensate reaction products could be attributable to high complexation selectivity for Cu and Fe irons. Thus, condensate reaction products of the present invention may be incorporated into a complexing agent for use according to the present invention, and according to the methods of the present invention. The complexing agents may comprise the first condensate reaction product, or the second condensate reaction product or both. The complexing agents may consist of the first condensate reaction product, or the second condensate reaction product or both. Suitably, the complexing agents consist of the first condensate reaction product, or alternatively consist of the second condensate reaction product.

The cosmetic compositions of the present invention may also comprise suitable additives and/or additional components as would be found in conventional compositions for use in the oxidative lightening and/or dyeing of keratinous fibres. The skilled person is able to select the most appropriate additives and/or additional components based on the nature of the cosmetic composition. For example, the cosmetic compositions for use in the oxidative lightening and/or dyeing of keratinous fibres may further comprise oxidising agents, alkalising agents, surfactants, oils, fatty components (particularly fatty components with a melting point in the range of 23 - 110°C) and polymeric thickeners.

A further surprising finding of the inventors is the ability of the condensate reaction products of the present invention to be included in conventional bleaching products (for example pastes and powders) without the need for additional substances and this forms another aspect of the present invention. For example, the present invention provides cosmetic compositions comprising the condensate reaction products according to the invention, wherein no additional additives are present. Such additives include acids, for example succinic acid (or salts thereof), lysine and arginine. Suitably, the cosmetic product of the present invention may consist of solely the condensate reaction product of the present invention either as a solid or in an aqueous solution. The condensate reaction product of the present invention may also be dissolved in an organic solvent or in a solvent mixture.

Bleaching powders incorporating the reaction products of the present invention are disclosed herein.

The terms "powder" or "powder-like" are to be understood, in accordance with the invention, to mean an administration form formed of individual particles which, at 20°C and 1013 mbar, is solid and can be poured, the individual particles having particle sizes in the range of from 0.1 μm to at most 1.6 mm. The particle sizes can be determined preferably by means of laser diffraction measurement in accordance with ISO 13320-1 (2009). As appropriate, the grain size of the particles can be adapted to the requirements of the bleaching powder by physical treatment, such as sieving, pressing, granulation or palletisation, or by the addition of specific auxiliaries, so as to enable for example an improved miscibility of the individual powder constituents or the miscibility of the bleaching powder with a hydrogen peroxide preparation.

Bleaching powders that are preferred in accordance with the invention have a bulk density in the range of from 500 to 1000 g/l (grams/litre), preferably 550 to 900 g/l, particularly preferably 600 to 820 g/l. The bulk density is determined preferably in accordance with EN ISO 60 (version 01/2000) or DIN ISO 697 (version 01/1984).

Unless specified otherwise, all specified temperatures relate to a pressure of 1013 mbar.

The bleaching powder according to the invention contains, as first essential constituent, at least one oxidising agent which is selected from sodium percarbonates and inorganic salts of a peroxysulfuric acid and mixtures thereof. The term sodium percarbonates is understood to mean sodium carbonate-hydrogen peroxide complexes. Commercially conventional sodium percarbonate has the average composition 2 $Na_2CO_3 \cdot 3$ HzOz. Sodium percarbonate is present in the form of a white, water-soluble powder, which easily decays into sodium carbonate and "active" oxygen having a bleaching and oxidising effect. Peroxysulfuric acids are understood to mean peroxydisulfuric acid and peroxymonosulfuric acid (Caro's acid). The at least one inorganic salt of a peroxysulfuric acid is preferably selected from ammonium peroxydisulfate, alkali metal peroxydisulfates, ammonium peroxymonosulfate, alkali metal peroxymonosulfates and alkali metal hydrogen peroxymonosulfates. Ammonium peroxydisulfate, potassium peroxydisulfate, sodium peroxydisulfate and potassium hydrogen peroxymonosulfate are particularly preferred. Within the scope of the works for the present

invention, it has also proven to be particularly preferable if the bleaching powder according to the invention contains at least two different peroxydisulfates. Preferred peroxydisulfates are, here, combinations of ammonium peroxydisulfate and potassium peroxydisulfate and/or sodium peroxydisulfate. Preferred bleaching powders according to the invention contain at least one oxidising agent, which is selected from sodium percarbonates and inorganic salts of a peroxysulfuric acid and mixtures thereof, in a total amount of 5 - 85 % by weight, preferably 10 - 75 % by weight, particularly preferably 15 - 65 % by weight, and extremely preferably 20 - 55 % by weight, in each case in relation to the weight of the bleaching powder.

When the complexing agent comprising the condensate reaction product according to the invention is added to the bleaching powder (rather than to the hydrogen peroxide-containing oxidation composition), the bleaching powder according to the invention contains a complexing agent comprising a condensate reaction product according to the invention and as disclosed herein. The condensate reaction product may be the first condensate reaction product or second the condensate reaction product or a mixture thereof.

Bleaching powders that are preferred in accordance with the invention additionally contain at least one inorganic alkalising agent which is solid at 20°C and 1013 mbar, including at least one sodium silicate or sodium metasilicate having a molar SiOz/NazO ratio of ≥ 2, preferably 2.5-3.5, in a total amount of from 0.1 to 50 % by weight, preferably 5 to 40 % by weight, in each case in relation to the weight of the bleaching powder. Bleaching powders that are preferred in accordance with the invention contain at least one inorganic alkalising agent which is solid at 20°C and 1013 mbar, preferably in a total amount of 1 - 60 % by weight, preferably 5 - 55 % by weight, particularly preferably 10 - 50 % by weight, extremely preferably 15 - 45 % by weight, in each case in relation to the weight of the bleaching powder. Besides the at least one sodium silicate or sodium metasilicate having a molar SiOz/NazO ratio of ≥ 2, preferably 2.5-3.5, in a total amount of from 0.1 to 50 % by weight, preferably 5 to 40 % by weight, in each case in relation to the weight of the bleaching powder, further inorganic alkalising agents which are solid at 20°C and 1013 mbar and which are particularly preferred in accordance with the invention are selected as optional alkalising agent from alkaline earth metal silicates, alkaline earth metal hydroxide carbonates, alkaline earth metal carbonates, alkaline earth metasilicates, alkali metal hydroxides, alkaline earth metal hydroxides, (earth) alkali metal phosphates and (earth) alkali metal hydrogen phosphates, and mixtures of these substances. Besides the at least one obligatory sodium silicate or sodium metasilicate, in each case with a molar SiOz/NazO ratio of ≥ 2, preferably 2.5-3.5, inorganic alkalising agents which are solid at 20°C and 1013 mbar and which are particularly preferred in accordance with the invention are selected from magnesium hydroxide carbonates and mixtures of these alkalising agents. Magnesium hydroxide carbonates which are preferred in accordance with the invention are those with the formula $MgCO_3 \cdot Mg(OH)_2 \cdot 2\,H_2O$ and those with the formula $MgCO_3 \cdot Mg(OH)_2$. Magnesium hydroxide carbonate with the formula $MgCO_3 \cdot Mg(OH)_2$. is particularly preferred in accordance with the invention. Bleaching powders that are particularly preferred in accordance with the invention contain, in each case in relation to their total weight, 0.1 to 50 % by weight, preferably 5 to 40 % by weight, of sodium silicates having a molar SiOz/NazO ratio of ≥ 2, preferably 2.5 to 3.5, and 2 - 20 % by weight, preferably 5 - 15 % by weight, particularly preferably 8 - 25 % by weight, of magnesium hydroxide carbonate as inorganic alkalising agent solid at 20°C and 1013 mbar. Bleaching powders that are extremely preferred in accordance with the invention contain, in each case in relation to their total weight, 0.1 to 50 % by weight, preferably 5 to 40 % by weight, of sodium silicates having a molar SiOz/NazO ratio of ≥ 2, preferably 2.5 to 3.5, and 2 - 20 % by weight, preferably 5 - 15 % by weight, particularly preferably 10 - 13 % by weight, of magnesium hydroxide carbonate with the formula $MgCO_3 \cdot Mg(OH)_2$ as inorganic alkalising agent solid at 20°C and 1013 mbar. Provided the bleaching powder according to the invention or the bleaching powder that is preferred in accordance with the invention contains one or more inorganic carbonates, whether as alkalising agent or as oxidising agent in the form of sodium carbonate-hydrogen peroxide complexes, the content thereof is preferably selected such that the molar $CO_3^2$ total concentration in the mixture for use having the above-discussed oxidation composition (Ox) is at least 0.015 mol/100 grams of mixture for use. Provided the bleaching powder according to the invention or the bleaching powder that is preferred in accordance with the invention contains one or more inorganic carbonates, whether as alkalising agent or as oxidising agent in the form of sodium carbonate-hydrogen peroxide complexes, the content thereof is particularly preferably selected such that the molar $CO_3^2$ total concentration in the mixture for use having the above-discussed oxidation composition (Ox) is mathematically at least four times greater than the total concentration of proton donors. Provided the bleaching powder according to the invention or the bleaching powder that is preferred in accordance with the invention contains one or more inorganic carbonates, whether as alkalising agent or as oxidising agent in the form of sodium carbonate-hydrogen peroxide complexes, the content thereof is extremely preferably selected such that the molar $CO_3^2$ total concentration in the ready-to-use mixture having the above-discussed oxidation composition (Ox) is at least 0.015 mol/100 grams of mixture for use and is mathematically at least four times greater than the total concentration of proton donors.

The bleaching powders according to the invention preferably have a water content of 0 to 15 % by weight, preferably 0.1 to 10 % by weight, more preferably 0.5 to 9 % by weight of water, particularly preferably 0.5 to 3 % by weight of water, in each case in relation to the weight of the bleaching powder. These values relate to the content of free water. What is not considered is the content of molecularly bound water or water of crystallisation, which individual

powder constituents may have. The water content can be determined by means of Karl-Fischer titration, for example on the basis of ISO 4317 (version 2011-12).

In order to dedust the bleaching powders according to the invention, at least one dedusting agent can be added, which in particular is selected from at least one oil, in particular selected from paraffin oil, silicone oil or ester oil, and mixtures of these oils.

Bleaching powders that are preferred in accordance with the invention additionally contain at least one oil in a total amount of 0.1 - 15 % by weight, preferably 0.5 - 10 % by weight, particularly preferably 1 - 8 % by weight, extremely preferably 2 - 6 % by weight, in each case in relation to the weight of the bleaching powder. Oils that are preferred in accordance with the invention are selected from natural and synthetic hydrocarbons, particularly preferably from paraffin oils, $C_{18}$-$C_{30}$ isoparaffins, in particular isoeicosane, polyisobutenes, and polydecenes, further selected from $C_8$-$C_{16}$ isoparaffins, in particular from isodecane, isododecane, isotetradecane, and isohexadecane and mixtures thereof, and also 1 ,3-di-(2-ethyl hexyl)-cyclohexane. Further oils that are preferred in accordance with the invention are selected from the benzoic acid esters of linear or branched C8-C22 alkanols. Benzoic acid C12-C15 alkyl esters are particularly preferred. Further oils that are preferred in accordance with the invention are selected from fatty alcohols having 6-30 carbon atoms, which are unsaturated or branched and saturated or branched and unsaturated. Preferred alcohol oils are 2-hexyldecanol, 2-octyldodecanol, 2-ethylhexyl alcohol, and isostearyl alcohol. Further cosmetic oils that are preferred in accordance with the invention are selected from the triglycerides (= triple esters of glycerol) of linear or branched, saturated or unsaturated, optionally hydroxylated C8-30 fatty acids. The use of natural oils, for example amaranth seed oil, apricot kernel oil, argan oil, avocado oil, babassu oil, cottonseed oil, borage seed oil, camelina oil, thistle oil, peanut oil, pomegranate seed oil, grapefruit seed oil, hemp oil, hazelnut oil, elderberry seed oil, blackcurrant seed oil, jojoba oil, linseed oil, macadamia nut oil, corn oil, almond oil, marula oil, evening primrose oil, olive oil, palm oil, palm kernel oil, para nut oil, pecan nut oil, peach kernel oil, rapeseed oil, castor oil, sea buckthorn pulp oil, sea buckthorn seed oil, sesame oil, soy oil, sunflower oil, grapeseed oil, walnut oil, wild rose oil, wheat germ oil, and the liquid components of coconut oil and the like, can be particularly preferred. Synthetic triglyceride oils are also preferred, however, in particular capric/caprylic triglycerides. Further cosmetic oils that are particularly preferred in accordance with the invention are selected from the dicarboxylic acid esters of linear or branched $C_2$-$C_{10}$ alkanols, in particular diisopropyl adipate, di-n-butyl adipate, di-(2-ethylhexyl) adipate, dioctyl adipate, diethyl/di-n-butyl/dioctyl sebacate, diisopropyl sebacate, dioctyl malate, dioctyl maleate, dicaprylyl maleate, diisooctyl succinate, di-2-ethylhexyl succinate, and di-(2-hexyldecyl)succinate. Further cosmetic oils that are particularly preferred in accordance with the invention are selected from esters of linear or branched, saturated or unsaturated fatty alcohols having 2-30 carbon atoms with linear or branched, saturated or unsaturated fatty acids having 2-30 carbon atoms, which can be hydroxylated. These preferably include 2-hexyldecyl stearate, 2-hexyldecyl laurate, isodecyl neopentanoate, isononyl isononanoate, 2-ethylhexyl palmitate, and 2-ethylhexyl stearate, isopropyl myristate, isopropyl palmitate, isopropyl stearate, isopropyl isostearate, isopropyl oleate, isooctyl stearate, isononyl stearate, isocetyl stearate, isononyl isononanoate, isotridecyl isononanoate, cetearyl isononanoate, 2-ethylhexyl laurate, 2-ethylhexyl isostearate, 2-ethylhexyl cocoate, 2-octyldodecyl palmitate, butyl octanoic acid 2-butyl octanoate, diisotridecyl acetate, n-butyl stearate, n-hexyl laurate, n-decyl oleate, oleyl oleate, oleyl erucate, erucyl oleate, erucyl erucate, ethylene glycol dioleate and ethylene glycol dipalmitate. Further cosmetic oils that are preferred in accordance with the invention are selected from the addition products of 1 to 5 propylene oxide units with mono- or polyvalent $C_{8-22}$ alkanols such as octanol, decanol, decanediol, lauryl alcohol, myristyl alcohol, and stearyl alcohol, e.g. PPG-2 Myristyl Ether and PPG-3 Myristyl Ether. Further cosmetic oils that are preferred in accordance with the invention are selected from addition products of at least 6 ethylene oxide and/or propylene oxide units with mono- or polyvalent $C_{3-22}$ alkanols such as glycerol, butanol, butanediol, myristyl alcohol, and stearyl alcohol, which can be esterified if desired, e.g. PPG-14 Butyl Ether, PPG-9 Butyl Ether, PPG-10 Butanediol, PPG-15 Stearyl Ether, and Glycereth-7 diisonoanoate. Further cosmetic oils that are preferred in accordance with the invention are selected from $C_8$-$C_{22}$ fatty alcohol esters of monovalent or polyvalent $C_2$-$C_7$ hydroxycarboxylic acids, in particular the esters of glycolic acid, lactic acid, malic acid, tartaric acid, citric acid, and salicylic acid, for example $C_{12}$-$C_{15}$ alkyl lactate. Further cosmetic oils that are preferred in accordance with the invention are selected from symmetrical, asymmetrical, or cyclic esters of carbonic acid with $C_{3-22}$ alkanols, $C_{3-22}$ alkanediols, or $C_{3-22}$ alkanetriols, e.g. dicaprylyl carbonate, or the esters according to DE 19756454 A1, in particular glycerol carbonate. Further cosmetic oils that are suitable in accordance with the present invention are selected from the silicone oils that include, for example, decamethylcyclopentasiloxane, dodecamethylcyclohexasiloxane, dimethylpolysiloxane and methylphenylpolysiloxane, but also hexamethyldisiloxane, octamethyltrisiloxane and decamethyltetrasiloxane. Mixtures of the aforementioned oils can be used extremely preferably in accordance with the invention. Preferred bleaching powders according to the invention are characterised in that the cosmetic oil is selected from natural and synthetic hydrocarbons, particularly preferably from paraffin oils, $C_{18}$-$C_{30}$ isoparaffins, in particular isoeicosane, polyisobutenes, and polydecenes, $C_8$-$C_{16}$ isoparaffins, and 1,3-di-(2-ethylhexyl)cyclohexane; benzoic acid esters of linear or branched $C_{8-22}$ alkanols; fatty alcohols having 6-30 carbon atoms, which are unsaturated or branched and saturated or branched and unsaturated; triglycerides of linear or branched, saturated or unsaturated, optionally hydroxylated $C_{8-30}$ fatty acids, in particular natural oils; dicarboxylic acid esters of linear or branched $C_2$-$C_{10}$

alkanols; esters of linear or branched, saturated or unsaturated fatty alcohols having 2-30 carbon atoms with linear or branched, saturated or unsaturated fatty acids having 2-30 carbon atoms, which can be hydroxylated; addition products of 1 to 5 propylene oxide units with mono- or polyvalent $C_{8-22}$ alkanols; addition products of at least 6 ethylene oxide and/or propylene oxide units with mono- or polyvalent $C_{3-22}$ alkanols; $C_8$-$C_{22}$ fatty alcohol esters of monovalent or polyvalent $C_2$-$C_7$ hydroxycarboxylic acids; symmetrical, asymmetrical, or cyclic esters of carbonic acid with $C_{3-22}$ alkanols, $C_{3-22}$ alkanediols, or $C_{3-22}$ alkanetriols; esters of dimers of unsaturated $C_{12}$-$C_{22}$ fatty acids (dimer fatty acids) with monovalent linear, branched, or cyclic $C_2$-$C_{18}$ alkanols or with polyvalent linear or branched $C_2$-$C_6$ alkanols; silicone oils; and mixtures of the aforementioned substances, and preferably in a total amount of 0.1 - 15 % by weight, preferably 0.5 - 10 % by weight, particularly preferably 1 - 8 % by weight, extremely preferably 2 - 6 % by weight, in each case in relation to the weight of the bleaching powder.

Further bleaching powders that are preferred in accordance with the invention contain at least one polymer, which is selected from acrylic acid homo- and copolymers, methacrylic acid homo- and copolymers, itaconic acid homo- and copolymers, polysaccharides which can be chemically and/or physically modified, and mixtures of these polymers, wherein one or more of the aforementioned polymers is particularly preferably contained in a total amount of 0.1 - 6 % by weight, preferably 0.5 - 4 % by weight, particularly preferably 1 - 3.5 % by weight, extremely preferably 2 - 3 % by weight, in each case in relation to the weight of the bleaching powder.

A further subject of the present invention is a method for lightening keratin fibres, in particular human hair, in which a bleaching powder according to the invention or a bleaching powder that is preferred in accordance with the invention as disclosed herein is mixed with an oxidation composition which, in each case in relation to its weight, contains 50 - 96 % by weight, preferably 70 - 93 % by weight, particularly preferably 80 - 90 % by weight of water and 0.5 - 20 % by weight of hydrogen peroxide and also contains at least one pH adjuster in such an amount that the oxidation composition has a pH value in the range of 2.5 to 5.5, measured at 20°C, is applied directly thereafter to the keratin-containing fibres, is left on the fibres for 5 to 60 minutes, and then the fibres are rinsed with water and the mixture is optionally washed out using a surfactant-containing cleansing agent, wherein the bleaching powder (B) and the oxidation composition (Ox) are preferably mixed with one another in a weight-based ratio (B):(Ox) of 0.2 - 1, particularly preferably 0.3 - 0.8, more preferably 0.4 - 0.7, extremely preferably 0.5 - 0.6.

The oxidation composition (Ox) used in the lightening method according to the invention contains fundamentally water and hydrogen peroxide. The concentration of hydrogen peroxide is determined on the one hand by the legal requirements and on the other hand by the desired effect. It is 0.5 - 20 % by weight, preferably 3 - 12 % by weight, particularly preferably 6 - 9 % by weight of hydrogen peroxide (calculated as 100% HzOz), in each case in relation to the weight of the oxidation composition (Ox).

When the complexing agent comprising the condensate reaction product according to the invention is added to this hydrogen peroxide-containing oxidation composition (rather than to the bleaching powder), the oxidation composition used according to the invention contains a complexing agent comprising a condensate reaction product according to the invention and as disclosed herein. The condensate reaction product may be the first condensate reaction product or second the condensate reaction product or a mixture thereof.

The oxidation composition (Ox), in order to stabilise the hydrogen peroxide, preferably has an acidic pH value, in particular a pH value in the range of 2.5 to 5.5, measured at 20°C. To stabilise the hydrogen peroxide, complexing agents, preservatives and/or buffer substances are also preferably contained. The bleaching powder that is preferred in accordance with the invention is of such a composition that the mixture with the aforementioned oxidation composition (Ox), i.e. the colour-changing agent ready for use, in particular bleaching agent, has an alkaline pH value, preferably a pH value of 8 to 11.5, particularly preferably a pH value of 8.5 to 11, extremely preferably a pH value of 9.0 to 10.5, in each case measured at 20°C. Oxidation compositions (Ox) used particularly preferably in accordance with the invention also contain at least one oil and/or at least one fatty component having a melting point in the range of 23 - 110°C, preferably in a total amount of 0.1 - 60 % by weight, particularly preferably 0.5 - 40 % by weight, extremely preferably 2 - 24 % by weight, in each case in relation to the weight of the oxidation composition (Ox) used with particular preference in accordance with the invention. The oils suitable for the oxidation compositions (Ox) preferably used in accordance with the invention are the same oils as those disclosed above as being suitable dedusting agents.

Fatty components with a melting point in the range of 23 - 110°C preferably used in accordance with the invention in the oxidation compositions (Ox) are selected from linear saturated 1-alkanols with 12-30 carbon atoms, preferably in a total amount of 0.1 - 8 % by weight, particularly preferably 3.0 to 6.0 % by weight, in each case in relation to the weight of the oxidation composition (Ox) used in accordance with the invention. The at least one linear saturated 1-alkanol having 12 - 30 carbon atoms is preferably selected from lauryl alcohol, myristyl alcohol, cetyl alcohol, stearyl alcohol, arachidyl alcohol, and behenyl alcohol and also from mixtures of these 1-alkanols, particularly preferably from cetyl alcohol, stearyl alcohol and cetyl alcohol/stearyl alcohol mixtures. Oxidation compositions (Ox) that are used with preference in accordance with the invention also contain, in each case in relation to their weight, at least one linear saturated 1-alkanol having 2-30 carbon atoms in a total amount of 0.1 - 8 % by weight, preferably in a total amount of 2 - 6 % by weight, wherein at least one 1-alkanol selected from cetyl alcohol, stearyl alcohol and cetyl alcohol/stearyl

alcohol mixtures is contained. Further oxidation compositions (Ox) that are used with preference in accordance with the invention contain at least one fatty component having a melting point in a range of 23 - 110°C, which is selected from esters of a saturated, monovalent $C_{16}$-$C_{60}$ alkanol and a saturated $C_8$-$C_{36}$ monocarboxylic acid, in particular cetyl behenate, stearyl behenate and $C_{20}$-$C_{40}$ alkyl stearate, glycerol triesters of saturated linear $C_{12}$-$C_{30}$ carboxylic acids, which can be hydroxylated, candelilla wax, carnauba wax, beeswax, saturated linear $C_{14}$-$C_{36}$ carboxylic acids, and mixtures of the aforementioned substances.

Further oxidation compositions (Ox) that are preferably used in accordance with the invention contain at least one surfactant or at least one emulsifier, preferably in a total amount of 0.5 - 10 % by weight, preferably 1 - 5 % by weight, in each case in relation to the weight of the oxidation composition (Ox) used in accordance with the invention. Surfactants and emulsifiers in the sense of the present application are amphiphilic (bifunctional) compounds that consist of at least one hydrophobic and at least one hydrophilic molecule part. The hydrophobic group is preferably a hydrocarbon chain having 8-28 carbon atoms, which can be saturated or unsaturated, linear or branched. This $C_8$-$C_{28}$ alkyl chain is particularly preferably linear. The basic properties of the surfactants and emulsifiers are oriented absorption at boundary surfaces and also the aggregation to micelles and the formation of lyotropic phases. Anionic, non-ionic and cationic surfactants are particularly suitable in accordance with the invention. However, zwitterionic and amphoteric surfactants are also very suitable in accordance with the invention. All anionic surface-active substances that are suitable for use on the human body are suitable as anionic surfactants in the compositions according to the invention. These are characterised by a water-soluble-making anionic group, such as a carboxylate, sulfate, sulfonate or phosphate group and a lipophilic alkyl group having 8 to 30 C atoms. In addition, glycol or polyglycolether groups, ester, ether and amide groups and also hydroxyl groups can be contained in the molecule. Examples of suitable anionic surfactants are linear and branched fatty acids having 8 to 30 C atoms (soaps), alkylether carboxylic acids, acyl sarcosides, acyl taurides, acyl isethionates, sulfosuccinic acid monoesters and dialkylesters and sulfosuccinic acid mono-alkylpolyoxyethyl esters, linear alkane sulfonates, linear alpha-olefin sulfonates, alkylsulfates and alkylether sulfates and also alkyl and/or alkenyl phosphates. Preferred anionic surfactants are alkyl sulfates, alkylether sulfates and alkylether carboxylic acids each having 10 to 18 C atoms, preferably 12 to 14 C atoms in the alkyl group and up to 12 glycolether groups, preferably 2 to 6 glycol ether groups in the molecule. Examples of such surfactants are the compounds with the INCI names Sodium Laureth Sulfate, Sodium Lauryl Sulfate, Sodium Myreth Sulfate or Sodium Laureth Carboxylate. Surface-active compounds that carry, in the molecule, at least one quaternary ammonium group and at least one carboxylate, sulfonate or sulfate group are referred to as zwitterionic surfactants. Particularly suitable zwitterionic surfactants are what are known as betaines, such as the N-alkyl-N,N-dimethylammonium glycinates, for example coco-alkyl-dimethylammonium glycinate, N-acyl-aminopropyl-N,N-dimethylammonium glycinates, for example coco-acylaminopropyl-dimethylammonium glycinate, and 2-alkyl-3-carboxymethyl-3-hydroxyethyl imidazolines, each having 8 to 18 C atoms in the alkyl or acyl group and also coco-acylaminoethylhydroxyethylcarboxymethyl glycinate. A preferred zwitterionic surfactant is the fatty acid amide derivative known under the INCI name Cocamidopropyl Betaine. Amphoteric surfactants are understood to be surface-active compounds which, in addition to a $C_8$-$C_{24}$ alkyl or acyl group, also contain at least one free amino group and at least one -COOH or -$SO_3$H group in the molecule and are capable of forming inner salts. Examples of suitable amphoteric surfactants are N-alkylglycines, N-alkylpropionic acids, N-alkylaminobutyric acids, N-alkyliminodipropionic acids, N-hydroxyethyl-N-alkylamidopropylglycines, N-alkyltaurines, N-alkylsarcosines, 2-alkyl aminopropionic acids, and alkyl amino acetic acids each having 8 to 24 C atoms in the alkyl group. Particularly preferred amphoteric surfactants are N-coco-alkylaminopropionate, coco-acylaminoethylaminopropionate, and $C_{12}$-$C_{18}$ acyl sarcosine. Non-ionic surfactants contain, as hydrophilic group, for example a polyol group, a polyalkylene glycol ether group or a combination of polyol group and polyglycol ether group. Such compounds are, for example, addition products of 4 to 50 mol ethylene oxide and/or 0 to 5 mol propylene oxide with linear and branched fatty alcohols, with fatty acids, and with alkyl phenols, in each case having 8 to 20 C atoms in the alkyl group, ethoxylated mono-, di- and triglycerides, such as glycerol monolaurate + 20 ethylene oxide, and glycerol monostearate + 20 ethylene oxide, sorbitol fatty acid ester, and addition products of ethylene oxide with sorbitol fatty acid ester, such as Polysorbate (Tween 20, Tween 21, Tween 60, Tween 61, Tween 81), addition products of ethylene oxide with fatty acid alkanolamides and fatty amines, and alkylpolyglycosides. In particular, $C_8$-$C_{22}$ alkylmono- and -oligoglycosides and ethoxylated analogues thereof and also ethylene oxide addition products with saturated or unsaturated linear fatty alcohols each having 2 to 30 mol of ethylene oxide per mol of fatty alcohol are suitable as non-ionic surfactants. Further oxidation compositions used with preference in accordance with the invention are characterised in that the at least one anionic surfactant is selected from alkyl sulfates, alkyl ether sulfates, and alkyl ether carboxylic acids each having 10 to 18 C atoms, preferably 12 to 14 C atoms, in the alkyl group and up to 12 glycolether groups, preferably 2 to 6 glycol ether groups, in the molecule. Further oxidation compositions used with preference in accordance with the invention are characterised in that at least one non-ionic surfactant, selected from ethylene oxide addition products with saturated or unsaturated linear fatty alcohols each having 2 to 30 mol of ethylene oxide per mol of fatty alcohol, and at least one anionic surfactant, selected from alkyl sulfates, alkylether sulfates, and alkyl ether carboxylic acids, each having 10 to 18 C atoms, preferably 12 to 14 C atoms, in the alkyl group and up to 12 glycol ether groups, preferably 2 to 6 glycol ether groups, in the molecule are contained, wherein

the ratio by weight of the totality of all anionic surfactants to the totality of all non-ionic surfactants particularly preferably lies in the range of 5 - 50, preferably 10 - 30. All cationic surface-active substances that are suitable for use on the human body are suitable in principle as cationic surfactants in oxidation compositions (Ox) used with preference in accordance with the invention. These are characterised by at least one water-soluble-making cationic group, such as a quaternary ammonium group, or by at least one water-soluble-making cationisable group, such as an amine group, and also at least one (lipophilically acting) alkyl group having 6 to 30 C atoms or at least one (lipophilically acting) imidazole group or at least one (lipophilically acting) imidazyl alkyl group. Oxidation compositions (Ox) used with particular preference in accordance with the invention contain at least one cationic surfactant, which is preferably selected from quaternary ammonium compounds having at least one C8-C24 alkyl group, esterquats and amidoamines each having at least one C8-C24 acyl group and mixtures hereof. Preferred quaternary ammonium compounds having at least one C8-C24 alkyl group are ammonium halides, in particular chlorides and ammonium alkyl sulfates, such as methosulfates or ethosulfates, such as C8-C24 alkyl trimethyl ammonium chlorides, C8-C24 dialkyl dimethyl ammonium chlorides and C8-C24 trialkyl methyl ammonium chlorides, for example cetyl trimethyl ammonium chloride, stearyl trimethyl ammonium chloride, distearyl dimethyl ammonium chloride, lauryl dimethyl ammonium chloride, lauryl dimethyl benzyl ammonium chloride, and tricetyl methyl ammonium chloride, and the imidazolium compounds known under the INCI names Quaternium-27, Quaternium-83, Quaternium-87 and Quaternium-91. The alkyl chains of the above-mentioned surfactants preferably have 8 to 24 carbon atoms. Esterquats are cationic surfactants which contain both at least one ester function and at least one quaternary ammonium group as structural element and also at least one C8-C24 alkyl group or C8-C24 acyl group. Preferred esterquats are quaternised ester salts of fatty acids with triethanolamine, quaternised ester salts of fatty acids with diethanol alkylamines and quaternised ester salts of fatty acids with 1 ,2-dihydroxypropyldialkylamines. Such products are sold for example under the trade name Stepantex®, Dehyquart® and Armocare®. N.N-Bis(2-Palmitoyloxyethyl)dimethylammonium chloride, Distearoylethyl Dimonium Methosulfate and Distearoylethyl Hydroxyethylmonium Methosulfate are preferred examples of such esterquats. The alkyl amidoamines are usually produced by amidation of natural or synthetic C8-C24 fatty acids and fatty acid cuts with di-(C1-C3)alkyl amino amines. A compound from this substance group which is particularly suitable in accordance with the invention is stearamidopropyl dimethylamine. Oxide compositions (Ox) used with particular preference in accordance with the invention contain at least one cationic surfactant in a total amount of 0.01 - 5 % by weight, preferably 0.1 - 3 % by weight, particularly preferably 0.3 - 2 % by weight, in each case in relation to the weight of the oxidation composition (Ox) used in accordance with the invention.

The present invention also provides a multi-component packaging unit (kit-of-parts) for changing the colour of keratin fibres, in particular human hair, containing at least two or three components packaged separately from one another. The bleaching powder of the present invention is present in one of the parts.

A multi-component packaging unit comprises a plurality of individual components which are packaged separately from one another, and also a common packaging for these components, for example a collapsible box. The components are provided therein, each separated into different containers. Within the scope of the present invention, a container is understood to mean a wrapping which is present in the form of an optionally re-closable bottle, a tube, a can, a bag, a sachet or a similar wrapping. In accordance with the invention, the wrapping material is not subject to any limitations. However, the wrappings are preferably made of glass or plastic. In addition, the packaging unit can comprise application aids, such as combs, hairbrushes or paintbrushes, personal protective clothing, in particular disposable gloves, and a set of instructions.

In a further preferred embodiment of the invention a bleaching powder according to the invention or a bleaching powder that is preferred in accordance with the invention can be combined with an alkalising composition (Alk) and with an oxidation composition (Ox), which suitably forms a lightening or dyeing agent for keratin fibres. The bleaching powder may be packaged together with the oxidising agent. Alternatively, the bleaching powder may be packaged together with the alkalising agent. A further alternative is that the bleaching powder is packaged separately from both the oxidising agent and the from the alkalising agent.

Since, when treating keratin fibres, in particular hair, with oxidising agents, in particular with hydrogen peroxide, the dye melanin, which occurs naturally in the fibres, is destroyed to a certain extent, the fibres/hair are/is inevitably lightened, i.e. the colour thereof changes even without the presence of a dye. The term "colour change" in the sense of the present application therefore includes both the lightening and dyeing.

The alkalising composition (Alk) used in accordance with the invention contains water and at least one alkalising agent, which is selected from ammonia, alkanolamines and mixtures hereof, and which and has a pH value in the range of 8 - 12, preferably 9 - 11, particularly preferably 9.5 - 10.5, in each case measured at 20°C. Preferred alkanolamines are selected from monoethanolamine, 2-amino-2-methylpropanol and triethanolamine and also mixtures hereof, wherein monoethanolamine is particularly preferred. An extremely preferred alkalising agent is ammonia. Ammonia ($NH_3$) in the form of its aqueous solution is usually used. Aqueous ammonia solutions contain ammonia ($NH_3$) often in concentrations of 10 to 32 % by weight. Here, the use of an aqueous ammonia solution which contains 25 % by weight ammonia ($NH_3$) is preferred. Besides ammonia and alkanolamines, at least one further alkalising agent can be contained, which is selected from alkali metal silicates, alkaline earth metal silicates, alkaline earth metal hydroxide carbonates, alkaline

earth metal carbonates, alkali metal metasilicates, alkaline earth metal metasilicates, alkali metal hydroxides, alkaline earth metal hydroxides and mixtures of these substances. Ammonia and/or monoethanolamine are preferably contained in the alkalising compositions used with preference in accordance with the invention in amounts of 0.01 - 10 % by weight, preferably of 0.1 - 7.5 % by weight, more preferably of 0.5 - 5.5 % by weight, and particularly preferably of 1.5 - 4.5 % by weight, in each case in relation to the weight of the alkalising composition.

A further subject of the present invention is a method for changing the colour of keratin fibres, in particular human hair, in which a bleaching powder according to the invention or a bleaching powder that is preferred in accordance with the invention as disclosed herein is mixed with an oxidation composition (Ox) which contains, in each case in relation to its weight, 50 - 96 % by weight, preferably 70 - 93 % by weight, particularly preferably 80 - 90 % by weight of water and 0.5 - 20 % by weight of hydrogen peroxide and also contains at least one pH adjuster in such an amount that the oxidation composition has a pH value in the range of 2.5 to 5.5, measured at 20°C,

and additionally is mixed with an alkalising composition (Alk) which contains water and at least one alkalising agent which is selected from ammonia, alkanolamines and mixtures hereof, and has a pH value in the range of 8 - 12, preferably 9 - 11, particularly preferably of 9.5 - 10.5, in each case measured at 20°C, is applied to the keratin-containing fibres directly thereafter, is left on the fibres for 5 to 60 minutes, and the fibres are then rinsed with water and the mixture is optionally washed out using a surfactant-containing cleansing agent, wherein the bleaching powder (B), the oxidation composition (Ox), and the alkalising composition (Alk) are preferably mixed with one another in a weight-based ratio (B):(Ox):(Alk) of (0.7 - 1.3):(2 - 3):(2 - 3), particularly preferably (0.8 - 1.2):(2.3 - 2.7):(2.3 - 2.7), extremely preferably 1:2:2.

In accordance with the invention, the bleaching powder is preferably composed such that the mixture with the aforementioned oxidation composition (Ox) and with the aforementioned alkalising composition (Alk), i.e. the colour-changing agent ready for use, in particular the bleaching agent, has an alkaline pH value, preferably a pH value from 8 to 11.5, particularly preferably a pH value from 8.5 to 11, extremely preferably a pH value from 9.0 to 10.5, in each case measured at 20°C.

The ready-for-use mixtures of a bleaching powder according to the invention or a bleaching powder that is preferred in accordance with the invention with one of the aforementioned oxidation compositions (Ox) and optionally with one of the aforementioned alkalising compositions (Alk) preferably have a viscosity in the range of 15,000 to 100,000 mPas, particularly preferably 20,000 to 85,000 mPas, in each case measured at 20°C using a Brookfield viscometer, DV-II+ model, spindle 5 with a speed of 4 revolutions per minute. A viscosity in this range means that the ready-for-use agent can be easily applied and also has such a flow behaviour that this guarantees, for the agent, a sufficiently long time of action at the site of action on the keratin fibres.

In order to facilitate the miscibility of the alkalising composition (Alk) used in accordance with the invention with the bleaching powder according to the invention or the bleaching powder preferred in accordance with the invention and the oxidation composition used in accordance with the invention and so as to also improve the use properties of the resultant mixture that is to be used, the alkalising composition used with preference in accordance with the invention preferably contains, in each case in relation to its weight, at least one surfactant in a total amount of 0.5 - 10 % by weight, preferably 2 - 8 % by weight.

The surfactants suitable for the alkalising compositions (Alk) used with preference in accordance with the invention are selected from the same anionic, cationic, non-ionic, amphoteric and zwitterionic surfactants and emulsifiers disclosed further above as surfactants and emulsifiers suitable for the oxidation compositions (Ox) used with preference.

Alkalising compositions (Alk) that are used with particular preference in accordance with the invention also contain at least one oil and/or at least one fatty component having a melting point in the range of 23 - 110°C, preferably in a total amount of 0.1 - 60 % by weight, particularly preferably 0.5 - 40 % by weight, extremely preferably 2 - 24 % by weight, in each case in relation to the weight of the alkalising composition (Alk) used with preference in accordance with the invention. The oils suitable for the alkalising compositions (Alk) used with preference in accordance with the invention are the same oils disclosed further above as suitable dedusting agents.

Fatty components having a melting point in the range of 23 - 110°C and used with preference in the alkalising compositions (Alk) in accordance with the invention are selected from linear saturated 1-alkanols having 12 - 30 carbon atoms, preferably in a total amount of 0.1 - 20 % by weight, particularly preferably 3 - 15 % by weight, extremely preferably 5 - 10 % by weight, in each case in relation to the weight of the alkalising composition used in accordance with the invention.

The at least one linear saturated 1-alkanol having 12-30 carbon atoms is preferably selected from lauryl alcohol, myristyl alcohol, cetyl alcohol, stearyl alcohol, arachidyl alcohol, and behenyl alcohol and also from mixtures of these 1-alkanols, particularly preferably from cetyl alcohol, stearyl alcohol and cetyl alcohol/stearyl alcohol mixtures. Alkalising compositions (Alk) used with preference in accordance with the invention also contain, in each case in relation to their weight, at least one linear saturated 1-alkanol having 12 - 30 carbon atoms in a total amount of 0.1 - 20 % by weight, preferably in a total amount of 3 - 15 % by weight, extremely preferably 5 - 10 % by weight, wherein at least one 1-alkanol, selected from cetyl alcohol, stearyl alcohol and cetyl alcohol/stearyl alcohol mixtures, is contained. Further alkalising compositions (Alk) used with preference in accordance with the invention contain at least one fatty component having a melting point in the range of 23 - 110°C, which is selected from esters of a saturated monovalent $C_{16}$-$C_{60}$

alkanol and a saturated $C_8$-$C_{36}$ monocarboxylic acid, in particular cetyl behenate, stearyl behenate and $C_{20}$-$C_{40}$ alkyl stearate, glycerol triesters of saturated linear $C_{12}$-$C_{30}$ carboxylic acids, which can be hydroxylated, candelilla wax, carnauba wax, beeswax, saturated linear $C_{14}$-$C_{36}$ carboxylic acids, and mixtures of the aforementioned substances.

The bleaching powders according to the invention and/or the bleaching powders that are preferred in accordance with the invention and/or the alkalising compositions used with preference in accordance with the invention can also contain at least one substantive dye. These are dyes which are drawn directly onto the hair and do not require an oxidising process to form the colour. To dull undesirable residual colour impressions caused by melanin degradation products, in particular in the red or blue spectrum, certain substantive dyes of the complementary colours are particularly preferably contained. Substantive dyes are usually nitrophenylenediamines, nitroaminophenols, azo dyes, anthraquinones or indophenols. Substantive dyes can be anionic, cationic or non-ionic. The substantive dyes are each used preferably in an amount of 0.001 to 2 % by weight, in relation to the weight of the bleaching powder or the alkalising composition (Alk).

Preferred anionic substantive dyes are the compounds known under the international names or trade names Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, bromophenol blue and tetrabromophenol blue. Preferred cationic substantive dyes include cationic triphenylmethane dyes, for example Basic Blue 7, Basic Blue 26, Basic Violet 2 and Basic Violet 14, aromatic systems which are substituted with a quaternary nitrogen group, for example Basic Yellow 57, Basic Red 76, Basic Blue 99, Basic Brown 16 and Basic Brown 17, cationic anthraquinone dyes, such as HC Blue 16 (Bluequat B), as well as substantive dyes containing a heterocyclic compound having at least one quaternary nitrogen atom, in particular Basic Yellow 87, Basic Orange 31 and Basic Red 51. The cationic substantive dyes sold under the Arianor trademark are likewise cationic substantive dyes preferred in accordance with the invention. Non-ionic substantive dyes which are suitable are, in particular, non-ionic nitro and quinone dyes and neutral azo dyes. Preferred non-ionic substantive dyes include the compounds known under the international names or trade names HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, as well as 1,4-diamino-2-nitrobenzene, 2-amino-4-nitrophenol, 1,4-bis-(2-hydroxyethyl)amino-2-nitrobenzene, 3-nitro-4-(2-hydroxyethyl)aminophenol, 2-(2-hydroxyethyl)amino-4,6-dinitrophenol, 4-[(2-hydroxyethyl)amino]-3-nitro-1-methylbenzene, 1-amino-4-(2-hydroxyethyl)amino-5-chloro-2-nitrobenzene, 4-amino-3-nitrophenol, 1-(2'-ureidoethyl)amino-4-nitrobenzene, 2-[(4-amino-2-nitrophenyl)amino]benzoic acid, 6-nitro-1,2,3,4-tetrahydroquinoxaline, 2-hydroxy-1,4-naphthoquinone, picramic acid and salts thereof, 2-amino-6-chloro-4-nitrophenol, 4-ethylamino-3-nitrobenzoic acid and 2-chloro-6-ethylamino-4-nitrophenol. A combination of tetrabromophenol blue and Acid Red 92 is contained very particularly preferably in accordance with the invention.

As a further optional ingredient, the alkalising composition used with preference in accordance with the invention contains at least one oxidation dye precursor product, which is preferably selected from one or more developer components and optionally one or more coupler components. At least one oxidation dye precursor product is particularly preferably contained in a total amount of 0.0001 to 10.0 % by weight, preferably 0.001 to 8 % by weight, in each case in relation to the weight of the alkalising composition used with preference in accordance with the invention.

It may be preferred in accordance with the invention to select, as developer component, at least one compound from the group formed from p-phenylenediamine, p-toluylenediamine, 2-(2-hydroxyethyl)-p-phenylenediamine, 2-(1,2-dihydroxyethyl)-p-phenylenediamine, N,N-bis-(2-hydroxyethyl)-p-phenylenediamine, N-(4-amino-3-methylphenyl)-N-[3-(1H-imidazol-1-yl)propyl]amine, N,N'-bis-(2-hydroxyethyl)-N,N'-bis-(4-aminophenyl)-1,3-diaminopropan-2-ol, bis-(2-hydroxy-5-aminophenyl)methane, 1,3-bis-(2,5-diaminophenoxy)propan-2-ol, N,N'-bis-(4-aminophenyl)-1,4-diazacycloheptane, 1,10-bis-(2,5-diaminophenyl)-1,4,7,10-tetraoxadecane, p-aminophenol, 4-amino-3-methylphenol, 4-amino-2-aminomethylphenol, 4-amino-2-(1,2-dihydroxyethyl)phenol, 4-amino-2-(diethylaminomethyl)phenol, 4,5-diamino-1-(2-hydroxyethyl)pyrazole, 2,4,5,6-tetraaminopyrimidine, 4-hydroxy-2,5,6-triaminopyrimidine, 2-hydroxy-4,5,6-triaminopyrimidine, and the physiologically acceptable salts thereof. At least one developer component is preferably contained in a total amount of 0.0001 to 10.0 % by weight, preferably 0.001 to 8 % by weight, in each case in relation to the weight of the alkalising composition used with preference in accordance with the invention.

Coupler components, within the scope of oxidative dyeing, do not alone form any significant colouration, but instead always require the presence of developer components. It is therefore preferred in accordance with the invention for additionally at least one coupler component to be used when at least one developer component is used. Coupler components that are preferred in accordance with the invention are selected from 3-aminophenol, 5-amino-2-methylphenol, N-cyclopentyl-3-aminophenol, 3-amino-2-chloro-6-methylphenol, 2-hydroxy-4-aminophenoxyethanol, 2,6-dimethyl-3-aminophenol, 3-trifluoroacetylamino-2-chloro-6-methylphenol, 5-amino-4-chloro-2-methylphenol, 5-amino-4-methoxy-2-methylphenyl, 5-(2-hydroxyethyl)amino-2-methylphenol, 3-(diethylamino)phenol, N-cyclopentyl-3-aminophenyl, 1,3-dihydroxy-5-(methylamino)benzene, 3-ethylamino-4-methylphenol, 2,4-dichloro-3-aminophenol, 2-(2,4-diaminophenoxy)ethanol, 1,3-bis(2,4-diaminophenoxy)propane, 1-methoxy-2-amino-4-(2-hydroxyethylamino)benzene, 1,3-bis(2,4-diaminophenyl)propane, 2,6-bis(2'-hydroxyethylamino)-1-methylbenzene, 2-({3-[(2-hydroxyethyl)amino]-4-methoxy-5-methylphenyl}amino)ethanol, 2-({3-[(2-hydroxyethyl)amino]-2-methoxy-5-methylphenyl}amino)ethanol, 2-({3-[(2-hydroxyethyl)amino]-4,5-dimethylphenyl}amino)ethanol, 2-[3-morpholin-4-ylphenyl)amino]ethanol, 3-amino-4-(2-methoxyethoxy)-5-methylphenylamine, 1-amino-3-bis-(2-hydroxyethyl)aminobenzene, resorcinol, resorcinol monomethyl ether, 2-methylresorcinol, 5-methylresorcinol, 2,5-dimethylresorcinol, 2-chlororesorcinol, 4-chlororesorcinol, pyrogallol, 1,2,4-trihydroxybenzene, 2,6-dihydroxypyridine, 2-amino-3-hydroxypyridine, 2-amino-5-chloro-3-hydroxypyridine, 3-amino-2-methylamino-6-methoxypyridine, 2,6-dihydroxy-3,4-dimethylpyridine, 2,6-dihydroxy-4-methylpyridine, 2,6-diaminopyridine, 2,3-diamino-6-methoxypyridine, 3,5-diamino-2,6-dimethoxypyridine, 2,6-dihydroxy-3,4-dimethylpyridine, 3,4-diaminopyridine, 2-(2-methoxyethyl)amino-3-amino-6-methoxypyridine, 2-(4'-methoxyphenyl)amino-3-aminopyridine, 1-naphthol, 2-methyl-1-naphthol, 2-hydroxymethyl-1-naphthol, 2-hydroxyethyl-1-naphthol, 1,3-dihydroxynaphthalene, 1,5-dihydroxynaphthalene, 1,6-dihydroxynaphthalene, 1,7-dihydroxynaphthalene, 1,8-dihydroxynaphthalene, 2,7-dihydroxynaphthalene, 2,3-dihydroxynaphthalene, 4-hydroxyindole, 6-hydroxyindole, 7-hydroxyindole, 4-hydroxyindoline, 6-hydroxyindoline, 7-hydroxyindoline, 4-6-diaminopyrimidine, 4-amino-2,6-dihydroxypyrimidine, 2,4-diamino-6-hydroxypyrimidine, 2,4,6-trihydroxypyrimidine, 2-amino-4-methylpyrimidine, 2-amino-4-hydroxy-6-methylpyrimidine and 4,6-dihydroxy-2-methylpyrimidine or mixtures of these compounds or the physiologically acceptable salts thereof. At least one coupler component is preferably contained in a total amount of 0.0001 to 10.0 % by weight, preferably 0.001 to 8 % by weight, in each case in relation to the weight of the alkalising composition used with preference in accordance with the invention. Here, developer components and coupler components are generally used in approximately equimolar amounts to one another. When the equimolar use has also proven to be expedient, a certain excess of individual oxidation dye precursors is not disadvantageous, and therefore developer components and coupler components can be contained in a molar ratio of 0.2 - 2, in particular 0.5 - 1. The time of action is preferably 5 to 60 min, in particular 5 to 50 min, particularly preferably 10 to 45 min. During the time in which the agents act on the fibres, it may be advantageous to assist the lightening or colour-changing process by adding heat. A phase of action at room temperature likewise corresponds to the invention. In particular, the temperature during the time of action is between 20°C and 40°C, in particular between 25°C and 38°C. The agents provide good treatment results even at physiologically acceptable temperatures of less than 45°C. After the end of the colour-changing process, all components located on the keratin fibres are rinsed from the hair using water or a surfactant-containing cleansing agent. Here, commercially available shampoo can be used in particular as cleansing agent, wherein it is then possible in particular to dispense with the cleansing agent and to carry out the rinsing process using mains water when the colour-changing agent has a higher surfactant content.

A further subject of the present invention is the use of a bleaching powder as disclosed herein for reducing damage to keratin fibres, in particular human hair, caused by the treatment of these fibres with a mixture of the bleaching powder and an oxidation composition, which, in each case in relation to its weight, contains 50 - 96 % by weight, preferably 70 - 93 % by weight, particularly preferably 80 - 90 % by weight of water and 0.5 - 20 % by weight of hydrogen peroxide and has a pH value in the range of 2.5 to 5.5, measured at 20°C.

That already said with regard to the bleaching powders according to the invention and the bleaching powders preferred in accordance with the invention also applies, *mutatis mutandis,* to the multi-component packaging units (kits-of-parts) according to the invention and those preferred in accordance with the invention. That already said with regard to the bleaching powders according to the invention and the bleaching powders preferred in accordance with the invention also applies, *mutatis mutandis,* to the methods according to the invention and those preferred in accordance with the invention for lightening and/or changing the colour of keratin fibres. That already said with regard to the oxidation compositions or alkalising compositions according to the invention and the oxidation compositions or alkalising compositions preferred in accordance with the invention also applies, *mutatis mutandis,* to the multi-component packaging units (kits-of-parts) according to the invention and those preferred in accordance with the invention. That already said with regard to the oxidation compositions or alkalising compositions according to the invention and the oxidation compositions or alkalising compositions preferred in accordance with the invention also applies, *mutatis mutandis,* to the methods according to the invention and those preferred in accordance with the invention for lightening and/or changing the colour of keratin fibres. That already said with regard to the bleaching powders according to the invention and the bleaching powders preferred in accordance with the invention also applies, *mutatis mutandis,* to the use according to the invention. That already said with regard to the oxidation compositions or alkalising compositions according to the invention and the oxidation compositions or alkalising compositions preferred in accordance with the invention also applies, *mutatis mutandis,* to the use according to the invention.

Bleaching pastes incorporating the reaction products of the present invention are disclosed herein.

The terms "paste" or "paste-like" are to be understood, in accordance with the invention, to mean an administration form which, at 20°C and 1013 mbar, has a viscosity in the range of 200,000 to 1,600,000 mPas, preferably 250,000 to 1,400,00 mPas, particularly preferably 300,000 to 1,000,000 mPas, exceptionally preferably 400,000 to 750,000 mPas. The paste viscosity is preferably determined by means of Brookfield; apparatus RVDV II+; spindle no. 96, 4 revolutions per minute, at 20°C.

Unless specified otherwise, all specified temperatures relate to a pressure of 1013 mbar.

The bleaching paste according to the invention contains, as first essential constituent, at least one oxidising agent which is selected from sodium percarbonates and inorganic salts of a peroxysulfuric acid and mixtures thereof. The oxidising agents disclosed above in the context of the bleaching powder according to the present invention may also be used in the bleaching pastes according to the present invention. Thus, the at least one oxidising agent may be selected from those described above. Preferred bleaching pastes according to the invention contain at least one oxidising agent, which is selected from sodium percarbonates and inorganic salts of a peroxysulfuric acid and mixtures hereof, in a total amount of 2.5-65 % by weight, preferably 10-60 % by weight, more preferably 20-55 % by weight, particularly preferably 25-50 % by weight, and in particular 30-45 % by weight, in each case in relation to the weight of the bleaching paste.

When the complexing agent comprising the condensate reaction product according to the invention is added to the bleaching paste (rather than to the hydrogen peroxide-containing oxidation composition), the bleaching paste according to the invention contains a complexing agent comprising a condensate reaction product according to the invention and as disclosed herein. The condensate reaction product may be the first condensate reaction product or second the condensate reaction product or a mixture thereof.

The bleaching pastes according to the invention have a water content of 0 to 4 % by weight, preferably 0.1 to 2 % by weight, particularly preferably 0.2 to 0.7 % by weight of water, in each case in relation to the weight of the bleaching paste. These values relate to the content of free water. What is not considered is the content of molecularly bound water or water of crystallization, which individual paste constituents may have. The water content can be determined by means of Karl-Fischer titration, for example on the basis of ISO 4317 (version 2011-12).

Bleaching pastes according to the invention and that are preferred in accordance with the invention contain at least one oil in a total amount of 16-60 % by weight, preferably 20-50 % by weight, particularly preferably 25-45 % by weight, in each case in relation to the weight of the bleaching paste. The oils disclosed above in the context of the bleaching powder according to the present invention may also be used in the bleaching pastes according to the present invention. Thus, the at least one oil, which is contained as carrier medium in the bleaching pastes according to the invention, may be selected from those described above.

Bleaching pastes that are preferred in accordance with the invention additionally contain at least one inorganic alkalising agent which is solid at 20°C and 1013 mbar and which is contained preferably in a total amount of 0.5-15 % by weight, preferably 1-10 % by weight, particularly preferably 2-8 % by weight, exceptionally preferably 3-7 % by weight, in each case in relation to the weight of the bleaching paste. Inorganic alkalising agents that are particularly preferred in accordance with the invention and that are solid at 20°C and 1013 mbar are selected from alkali metal silicates, alkaline earth metal silicates, alkaline earth metal hydroxide carbonates, alkaline earth metal carbonates, alkali metal metasilicates, alkaline earth metal metasilicates, alkali metal hydroxides, alkaline earth metal hydroxides, alkali (earth alkaline) metal phosphates and alkali (earth alkaline) metal hydrogen phosphates and mixtures of these substances. Inorganic alkalising agents that are particularly preferred in accordance with the invention and that are solid at 20°C and 1013 mbar are selected from sodium metasilicates having a molar SiOz/NazO ratio of 0.8-1.2, preferably of 0.9-1.1, exceptionally preferably of 1. Bleaching pastes that are particularly preferred in accordance with the invention contain, in each case in relation to their total weight, 0.5-15 % by weight, preferably 1-10 % by weight, particularly preferably 2-8 % by weight, exceptionally preferably 3-7 % by weight, in each case in relation to the weight of the bleaching paste, of sodium metasilicates having a molar SiOz/NazO ratio of 0.8-1.2, preferably of 0.9-1.1, exceptionally preferably of 1, as inorganic alkalising agent that is solid at 20°C and 1013 mbar.

In order to ensure the most uniform possible, storage-stable suspension of the obligatory constituents and optionally further constituents which are insoluble in the carrier oil, bleaching pastes that are preferred in accordance with the invention contain at least one substance which thickens the oil phase. Preferred thickening agents for the oil phase are selected from copolymer of C2-C4 alkene and styrene, linear saturated 1-alkanols having 12-30 carbon atoms, esters of saturated branched or unbranched alkane carboxylic acids having 12 to 24 C atoms, and saturated branched or unbranched alcohols having 16 to 50 C atoms, wherein the esters have a melting point in the range of 50°C to 110°C, triglycerides of saturated and optionally hydroxylated $C_{12-30}$ fatty acids, wherein the triglycerides have a melting point in the range of 50°C to 110°C, and mixtures of the aforementioned substances. Bleaching pastes that are preferred in accordance with the invention contain at least one substance which thickens the oil phase in a total amount of 1-15 % by weight, preferably 2-10 % by weight, particularly preferably 3-8 % by weight, particularly preferably 4-6.5 % by weight, in each case in relation to the weight of the bleaching paste. Further bleaching pastes that are preferred in accordance with the invention contain at least one substance which thickens the oil phase in a total amount of 1-15 % by weight, preferably 2-10 % by weight, particularly preferably 3-8 % by weight, particularly preferably 4-6.5 % by weight, in each case in relation to the weight of the bleaching paste, wherein the at least one substance which thickens the oil phase is selected from copolymers of C2-C4 alkene and styrene, linear saturated 1-alkanols having 12-30 carbon atoms, esters of saturated branched or unbranched alkane carboxylic acids having 12 to 24 C atoms, and saturated branched or unbranched alcohols having 16 to 50 C atoms, wherein the esters have a melting point in the range of 50°C to 110°C, triglycerides of saturated and optionally hydroxylated $C_{12-30}$ fatty acids, wherein the triglycerides have a melting point

in the range of 50°C to 110°C, and mixtures of the aforementioned substances. Copolymers of C2-C4 alkene and styrene that are preferred in accordance with the invention and that thicken the oil phase are contained in bleaching pastes that are preferred in accordance with the invention in a total amount of 0.1-1.5 % by weight, preferably 0.2-1 % by weight, particularly preferably 0.3-0.8 % by weight, exceptionally preferably 0.4-0.6 % by weight, in each case in relation to the weight of the bleaching paste. Copolymers of C2-C4 alkene and styrene that are preferred in accordance with the invention and that thicken the oil phase are selected from ethylene/propylene/styrene copolymers, butylene/ethylene/styrene copolymers, butylene/propylene/styrene copolymers, and mixtures of these copolymers. The aforementioned copolymers of C2-C4 alkene and styrene preferably are not copolymers in which the monomer units are randomly distributed, but instead block copolymers, particularly preferably diblock copolymers and triblock copolymers. Such block copolymers have "hard" segments formed of polystyrene and "soft" segments formed of ethylene/propylene or ethylene/butylene or propylene/butylene. The individual blocks can comprise, here, 10 to 10000, preferably 50 to 5000, and in particular 100 to 500 monomers. Preferred diblock copolymers are styrene-ethylene propylene (S-EP) and styrene-ethylene butylene (S-EB); preferred triblock copolymers are styrene-ethylene propylene-styrene (S-EP-S) and styrene-ethylene butylene-styrene (S-EB-S). Mixtures of diblock and triblock copolymers are used with particular preference in accordance with the invention, wherein mixtures of styrene-ethylene propylene (S-EP) and styrene-ethylene propylene-styrene (S-EP-S) have proven to be particularly preferred. Here, the proportion of diblock copolymers very particularly preferably contributes to 10 to 90 % by weight, and the proportion of triblock copolymers very particularly preferably contributes to 90 to 10 % by weight, in each case in relation to the weight of the polymer mixture. Bleaching pastes that are preferred in accordance with the invention contain at least one copolymer of C2-C4 alkene and styrene which thickens the oil phase and which is selected from ethylene/propylene/styrene copolymers, butylene/ethylene/styrene copolymers, butylene/propylene/styrene copolymers, and mixtures of these copolymers in a total amount of 0.1-1.5 % by weight, preferably 0.2-1 % by weight, particularly preferably 0.3-0.8 % by weight, exceptionally preferably 0.4-0.6 % by weight, in each case in relation to the weight of the bleaching paste. Bleaching pastes that are particularly preferred in accordance with the invention contain a combination of ethylene/propylene/styrene copolymer and butylene/ethylene/styrene copolymer, particularly preferably in a total amount of 0.1-1.5 % by weight, preferably 0.2-1 % by weight, particularly preferably 0.3-0.8 % by weight, exceptionally preferably 0.4-0.6 % by weight, in each case in relation to the weight of the bleaching paste. Linear saturated 1-alkanols having 12-30 carbon atoms which thicken the oil phase that are preferred in accordance with the invention are contained in bleaching pastes that are preferred in accordance with the invention in a total amount of 0.1-10 % by weight, preferably 0.5-8 % by weight, particularly preferably 1-7 % by weight, exceptionally preferably 2-5 % by weight, in each case in relation to the weight of the bleaching paste. Linear saturated 1-alkanols having 12-30 carbon atoms that are preferred in accordance with the invention are selected from lauryl alcohol, myristyl alcohol, cetyl alcohol, stearyl alcohol, arachidyl alcohol, and behenyl alcohol and also from mixtures of these 1-alkanols, particularly preferably from cetyl alcohol, stearyl alcohol and cetyl alcohol/stearyl alcohol mixtures. Bleaching pastes that are particularly preferred in accordance with the invention contain at least one linear saturated 1-alkanol having 12-30 carbon atoms, selected from lauryl alcohol, myristyl alcohol, cetyl alcohol, stearyl alcohol, arachidyl alcohol, and behenyl alcohol and also from mixtures of these 1-alkanols, particularly preferably from cetyl alcohol, stearyl alcohol and cetyl alcohol/stearyl alcohol mixtures, in a total amount of 0.1-10 % by weight, preferably 0.5-8 % by weight, particularly preferably 1-7 % by weight, exceptionally preferably 2-5 % by weight, in each case in relation to the weight of the bleaching paste. Esters of saturated, branched or unbranched alkane carboxylic acids having 12 to 24 C atoms and saturated branched or unbranched alcohols having 16 to 50 C atoms having a melting point in the range of 50°C to 110°C that are preferred in accordance with the invention and that thicken the oil phase are contained in bleaching pastes that are preferred in accordance with the invention in a total amount of 0.1-5 % by weight, preferably 0.2-4 % by weight, particularly preferably 0.3-2 % by weight, exceptionally preferably 0.4-1 % by weight, in each case in relation to the weight of the bleaching paste. Esters of saturated, branched or unbranched alkane carboxylic acids having 12 to 24 C atoms and saturated branched or unbranched alcohols having 16 to 50 C atoms having a melting point in the range of 50°C to 110°C that are preferred in accordance with the invention and that thicken the oil phase are selected from $C_{16-36}$ alkyl stearates, in particular $C_{20}$-$C_{40}$ alkyl stearates, $C_{18-38}$ alkyl hydroxy stearoyl stearates, $C_{20-40}$ alkyl erucates, cetearyl behenate, cetyl behenate, stearyl behenate, and mixtures of these substances. Triglycerides of saturated and optionally hydroxylated $C_{12-30}$ fatty acids having a melting point in the range of 50°C to 110°C that are preferred in accordance with the invention and that thicken the oil phase are contained in bleaching pastes that are preferred in accordance with the invention in a total amount of 0.1-5 % by weight, preferably 0.2-4 % by weight, particularly preferably 0.3-2 % by weight, exceptionally preferably 0.4-1 % by weight, in each case in relation to the weight of the bleaching paste. Triglycerides in the sense of the present invention are triesters of glycerol, i.e. esters, in which all OH groups of the glycerol are esterified with acid, in the present case with a saturated and optionally hydroxylated $C_{12-30}$ fatty acid. Triglycerides of saturated and optionally hydroxylated $C_{12-30}$ fatty acids having a melting point in the range of 50°C to 110°C that are preferred in accordance with the invention and that thicken the oil phase are selected from hardened triglyceride fats, in particular hydrogenated palm oil, hydrogenated coconut oil, hydrogenated castor oil, glyceryl tribehenate (tribehenin) or glyceryl tri-12-hydroxystearate, as well as mixtures thereof. Hydrogenated castor oil, obtainable for example as commercial product Cutina®

HR, is particularly preferred in accordance with the invention. Bleaching pastes that are particularly preferred in accordance with the invention contain at least one triglyceride of saturated and optionally hydroxylated $C_{12-30}$ fatty acids having a melting point in the range of 50°C to 110°C, selected from hardened triglyceride fats, in particular hydrogenated palm oil, hydrogenated coconut oil, hydrogenated castor oil, glyceryl tribehenate (tribehenin) or glyceryl tri-12-hydroxystearate, as well as mixtures thereof, wherein hydrogenated castor oil is particularly preferred, in a total amount of 0.1-5 % by weight, preferably 0.2-4 % by weight, particularly preferably 0.3-2 % by weight, exceptionally preferably 0.4-1 % by weight, in each case in relation to the weight of the bleaching paste. Bleaching pastes that are particularly preferred in accordance with the invention also contain at least one or more hydrophilic thickeners preferably selected from polysaccharides which can be chemically and/or physically modified, acrylic acid homo- and copolymers, methacrylic acid homo- and copolymers, itaconic acid homo- and copolymers, and mixtures of these polymers. In particular, compounds from the group of polysaccharides are suitable as hydrophilic thickeners. Examples include representatives of the celluloses (cellulose itself and derivatives thereof), alginic acids (and their corresponding physiologically acceptable salts, the alginates), agar agar (with the polysaccharide agarose present as main constituent in agar agar), starch fractions and derivatives such as amylose, amylopectin and dextrins, karaya rubber, locust bean gum, gum arabic, dextrans, guar gum and xanthan gum. Suitable cellulose derivatives are methyl celluloses, ethyl celluloses, hydroxyalkyl celluloses (such as hydroxyethyl cellulose), methylhydroxyalkyl celluloses, and carboxymethyl celluloses (such as those with the INCI name Cellulose Gum) and also their physiologically acceptable salts. From the group of polysaccharides, anionic polysaccharides such as carboxymethyl celluloses, alginic acid and xanthan gum are preferably selected for the thickening of the agent according to the invention. Carboxymethyl celluloses, alginic acids and xanthan gum, in addition to their physiologically acceptable salts, are referred to within the scope of the present invention as anionic polysaccharides, since the carboxylic acid groups present in these polysaccharides necessarily dissociate to a greater or lesser extent in water or aqueous formulation, whereby anionic carboxylate groups are formed, of which the number increases further with rising pH value. In preferred embodiments, carboxymethyl cellulose (preferably carboxymethyl cellulose with the INCI name Cellulose Gum) is contained as hydrophilic thickener in view of a reliable viscosity adjustment and residue-free application to keratin fibres and the scalp. Carboxymethyl cellulose can be contained in a preferred embodiment as the sole hydrophilic thickener. However, in particular a combination of carboxymethyl cellulose and xanthan (preferably xanthan with the INCI name Xanthan Gum) or physiologically acceptable salts thereof is also preferred. The physiologically acceptable salts are understood to mean in particular the sodium salts, but also the potassium salts, and also magnesium and calcium salts. Bleaching pastes that are particularly preferred in accordance with the invention contain at least one hydrophilic thickener in a total amount of 0.1 to 5 % by weight, preferably of 0.5 to 4 % by weight, more preferably of 1 to 3 % by weight, and very particularly preferably of 1.7 to 2.5 % by weight, in each case in relation to the weight of the bleaching paste. In a further preferred embodiment of the present invention the bleaching paste according to the invention contains, in each case in relation to the weight of the paste, 0.1 to 3 % by weight, preferably 0.5 to 2.5 % by weight, more preferably 1 to 2 % by weight, even more preferably 1 to 1.5 % by weight of carboxymethyl cellulose. In a further preferred embodiment of the present invention the bleaching paste according to the invention contains, in each case in relation to the weight of the paste, 0.1 to 3 % by weight, preferably 0.5 to 2.5 % by weight, more preferably 1 to 2 % by weight, even more preferably 1 to 1.5 % by weight of xanthan. Hydrophilic thickeners that are suitable in accordance with the invention include acrylic acid homo- and copolymers, methacrylic acid homo- and copolymers, itaconic acid homo- and copolymers, preferably selected from the group formed by the crosslinked and uncrosslinked homo- or copolymers of acrylic acid, methacrylic acid and salts thereof and alkyl esters, homo- or copolymers of acrylic acid amides and/or methacrylic acid amides, copolymers of acrylic acid and acrylic acid amides and mixtures thereof, copolymers of ethoxylated C1-C6 alkyl esters of methacrylic acid and the sulfonated acrylic acid amides and salts thereof and crosslinked copolymers of methacrylic acid, acrylic acid amides and the sulfonated acrylic acid amides and salts thereof. The above-mentioned polymers and copolymers can be crosslinked or uncrosslinked. Provided the above-mentioned polymers and copolymers do not have any alkyl groups with a chain length of at least 8 carbon atoms, they are preferably crosslinked. Provided the above-mentioned polymers and copolymers have alkyl groups with a chain length of at least 8 carbon atoms, they are preferably uncrosslinked. Examples of polymers that are preferred as hydrophilic thickeners are those known for example under the INCI name Copolymer Ammonium Acryloyldimethyltaurate / Beheneth-25 methacrylate Crosspolymer (trade name: Aristoflex HMB; Clariant), the copolymers known under the INCI name Acrylates/C10-30 Alkyl Acrylate Crosspolymer, and the crosslinked copolymer known under the INCI name Polyacrylate Crosspolymer-11 (trade name: Aristoflex Velvet; Clariant). In addition, bleaching pastes that are preferred in accordance with the invention contain one or more alkoxylated fatty alcohols, in particular ethoxylated fatty alcohols. In particular, fatty alcohols having 12 to 80 ethylene oxide groups, preferably 25 to 50 ethylene oxide groups, are suitable. Ethoxylated fatty alcohols that are suitable in accordance with the invention are those of the following formula (FAEO):

$$RO[CH_2CH_2\text{-}O]_nH \qquad \text{(FAEO)}$$

in which R stands for an unbranched or branched, saturated or unsaturated C10-C24 alkyl group and n stands for an

integer from 12 to 80. R preferably stands for an unbranched, saturated C12-C18 alkyl group or for an unbranched, monounsaturated C12-C18 alkyl group. In the formula (FAEO), n preferably stands for an integer from 20 to 60, and n particularly preferably stands for an integer from 25 to 50. Examples of alkoxylated fatty alcohols of the formula (FAEO) are Laureth-20, Laureth-25, Laureth-30, Laureth-40, Laureth-50, Myreth- 20, Myreth-25, Myreth-30, Myreth-40, Myreth-50, Ceteth-20, Ceteth-25, Ceteth-30, Ceteth-40, Ceteth-50, Steareth-20, Steareth- 25, Steareth-30, Steareth-40, Steareth-50, Ceteareth-20, Ceteareth-25, Ceteareth-30, Ceteareth-40, Ceteareth-50, Oleth-20, Oleth-25, Oleth-30, Oleth-40 and Oleth-50. Bleaching pastes that are particularly preferred in accordance with the invention contain one or more alkoxylated fatty alcohols, in particular ethoxylated fatty alcohols of the above formula (FAEO) in a total amount of 0.2 to 13.0 % by weight, preferably 2.0 to 8.0 % by weight, more preferably of 3.0 to 7.0 % by weight, in each case in relation to the weight of the bleaching paste according to the invention. Bleaching pastes that are particularly preferred in accordance with the invention contain Ceteareth-30 and/or Ceteareth-50 or a combination thereof. In particular, a combination of Ceteareth-30 and Ceteareth-50 is preferred. Here, Ceteareth-30 is preferably contained in an amount of 0.1 to 5 % by weight, more preferably 0.2 to 1 % by weight, even more preferably 0.3 to 0.7 % by weight, and Ceteareth-50 is preferably contained in an amount of 0.1 to 8 % by weight, more preferably 2 to 6 % by weight, even more preferably 3 to 5 % by weight, in each case in relation to the weight of the bleaching paste according to the invention. It has been found that particularly advantageous properties of the bleaching pastes that are particularly preferred in accordance with the invention and of the mixtures for use produced therefrom are attained with an amount of Ceteareth-30 and Ceteareth-50 in the specified ranges, in particular with a combination of Ceteareth-30 and Ceteareth-50.

A further subject of the present invention is a method for lightening keratinic fibres, in particular human hair, in which a bleaching paste according to the invention or a bleaching paste that is preferred in accordance with the invention as disclosed herein is mixed with an oxidation composition (Ox) which, in each case in relation to its weight, contains 50-96 % by weight, preferably 70-93 % by weight, particularly preferably 80-90 % by weight of water and 0.5-20 % by weight of hydrogen peroxide and also contains at least one pH adjuster in such an amount that the oxidation composition has a pH value in the range of 2.5 to 5.5 at 20°C, is applied directly thereafter to the keratin-containing fibres, is left on the fibres for 5 to 60 minutes, and then the fibres are rinsed with water and the bleaching paste is optionally washed out using a surfactant-containing cleansing agent, wherein the bleaching paste (B) and the oxidation composition (Ox) are preferably mixed with one another in a weight-based ratio (B):(Ox) of 0.2-1, particularly preferably 0.3-0.8, more preferably 0.4-0.7, exceptionally preferably 0.5-0.6.

The oxidation composition (Ox) used in the lightening method according to the invention contains fundamentally water and hydrogen peroxide. The concentration of hydrogen peroxide is determined on the one hand by the legal requirements and on the other hand by the desired effect. It is 0.5-20 % by weight, preferably 3-12 % by weight, particularly preferably 6-9 % by weight of hydrogen peroxide (calculated as 100% HzOz), in each case in relation to the weight of the oxidation composition (Ox).

The oxidation composition (Ox), in order to stabilise the hydrogen peroxide, preferably has an acidic pH value, in particular a pH value in the range of 2.5 to 5.5, measured at 20°C. To stabilise the hydrogen peroxide, complexing agents, preservatives and/or buffer substances are also preferably contained.

The bleaching paste that is preferred in accordance with the invention is of such a composition that the mixture with the aforementioned oxidation composition (Ox), i.e. the colour-changing agent ready for use, in particular bleaching agent, has an alkaline pH value, preferably a pH value of 8 to 11.5, particularly preferably a pH value of 8.5 to 11, exceptionally preferably a pH value of 9.0 to 10.5, in each case measured at 20°C.

When the complexing agent comprising the condensate reaction product according to the invention is added to this hydrogen peroxide-containing oxidation composition (rather than to the bleaching paste), the oxidation composition used according to the invention contains a complexing agent comprising a condensate reaction product according to the invention and as disclosed herein. The condensate reaction product may be the first condensate reaction product or second the condensate reaction product or a mixture thereof.

Oxidation compositions (Ox) used particularly preferably in accordance with the invention also contain at least one oil and/or at least one fatty component having a melting point in the range of 23-110°C, preferably in a total amount of 0.1-60 % by weight, particularly preferably 0.5-40 % by weight, exceptionally preferably 2-24 % by weight, in each case in relation to the weight of the oxidation composition (Ox) used with particular preference in accordance with the invention. The oils that are suitable for inclusion in these oxidation compositions (Ox) are the same oils as those described above in the context of the bleaching powders and methods involving the use thereof.

Fatty components preferably used in accordance with the invention in the oxidation compositions (Ox) with a melting point in the range of 23-110°C are present preferably in a total amount of 0.1-8 % by weight, particularly preferably 2.0 to 6.0 % by weight, in each case in relation to the weight of the oxidation composition (Ox) used in accordance with the invention. The fatty components that are suitable for inclusion in these oxidation compositions (Ox) are the same fatty components as those described above in the context of the bleaching powders and methods involving the use thereof.

Further oxidations compositions (Ox) that are preferably used in accordance with the invention contain at least one surfactant or at least one emulsifier, preferably in a total amount of 0.5-10 % by weight, preferably 1-5 % by weight,

in each case in relation to the weight of the oxidation composition (Ox) used in accordance with the invention. The oils surfactants/emulsifiers are suitable for inclusion in these oxidation compositions (Ox) are the same oils as those described above in the context of the bleaching powders and methods involving the use thereof.

The present invention also provides a multi-component packaging unit (kit-of-parts) for changing the colour of keratin fibres, in particular human hair, containing at least two or three components packaged separately from one another. The bleaching paste of the present invention is present in one of the parts.

A multi-component packaging unit comprises a plurality of individual components which are packaged separately from one another, and also a common packaging for these components, for example a collapsible box. The components are provided therein, each separated into different containers. Within the scope of the present invention, a container is understood to mean a wrapping which is present in the form of an optionally re-closable bottle, a tube, a tin, a bag, a sachet or a similar wrapping. In accordance with the invention, the wrapping material is not subject to any limitations. However, the wrappings are preferably made of plastic. In addition, the packaging unit can comprise application aids, such as combs, hairbrushes or paintbrushes, personal protective clothing, in particular disposable gloves, and a set of instructions.

In a further preferred embodiment of the invention a bleaching paste according to the invention or a bleaching paste that is preferred in accordance with the invention can be combined with an alkalising composition (Alk) and with an oxidation composition (Ox), which suitably forms a lightening or dyeing agent for keratin fibres. The bleaching paste may be packaged together with the oxidising agent. Alternatively, the bleaching paste may be packaged together with the alkalising agent. A further alternative is that the bleaching paste is packaged separately from both the oxidising agent and the from the alkalising agent.

Thus, a further subject of the present invention is a multi-component packaging unit (kit-of-parts) for lightening keratinic fibres which contains at least two components packaged separately from one another and which is characterised in that

i) the first component (I) is a bleaching paste according to the invention or is a bleaching paste that is preferred in accordance with the invention,
ii) the second component (II) is an oxidation composition which contains, in each case in relation to its weight, 50-96 % by weight, preferably 70-93 % by weight, particularly preferably 80-90 % by weight of water and 0.5-20 % by weight of hydrogen peroxide and has a pH value in the range of 2.5 to 5.5, measured at 20° C, wherein components (I) and (II) are preferably present in a weight-based ratio to one another (I):(II) of 0.2-1, particularly preferably 0.3-0.8, more preferably 0.4-0.7, exceptionally preferably 0.5-0.6.

A further subject of the present invention is a multi-component packaging unit (kit-of-parts) for changing the colour of keratinic fibres, in particular human hair, containing at least three components packaged separately from one another, wherein

i) the first component (I) is a bleaching paste according to the invention or is a bleaching paste that is preferred in accordance with the invention,
ii) the second component (II) is an oxidation composition which contains, in each case in relation to its weight, 50-96 % by weight, preferably 70-93 % by weight, particularly preferably 80-90 % by weight of water and 0.5-20 % by weight of hydrogen peroxide and has a pH value in the range of 2.5 to 5.5, measured at 20° C,
iii) the third component (III) is an alkalising composition (Alk) which contains water and at least one alkalising agent, which is selected from ammonia, alkanolamines and mixtures hereof and has a pH value in the range of 8-12, preferably of 9-11, particularly preferably of 9.5-10.5, in each case measured at 20°C,

wherein the bleaching paste (B), the oxidation composition (Ox) and the alkalising composition (Alk) are preferably present in a weight-based ratio to one another (B):(Ox):(Alk) of (0.7-1.3):(2-3):(2-3), particularly preferably (0.8-1.2):(2.3:2.7):(2.3-2.7).

A further subject of the present invention is a multi-component packaging unit (kit-of-parts) for changing the colour of keratinic fibres, in particular human hair, containing at least three components packaged separately from one another, wherein

i) the first component (I) is a bleaching paste according to the invention or is a bleaching paste that is preferred in accordance with the invention,
ii) the second component (II) is an oxidation composition which contains, in each case in relation to its weight, 50-96 % by weight, preferably 70-93 % by weight, particularly preferably 80-90 % by weight of water and 0.5-20 % by weight of hydrogen peroxide and has a pH value in the range of 2.5 to 5.5, measured at 20° C,
iii) the third component (III) is an alkalising composition (Alk) which contains water and at least one alkalising agent,

which is selected from ammonia, alkanolamines and mixtures hereof and has a pH value in the range of 8-12, preferably of 9-11, particularly preferably of 9.5-10.5, in each case measured at 20°C,

wherein the bleaching paste (B), the oxidation composition (Ox) and the alkalising composition (Alk) are preferably present in a weight-based ratio to one another (B):(Ox):(Alk) of (0.7-1.3):(2-3):(2-3), particularly preferably (0.8-1.2):(2.3:2.7):(2.3-2.7), exceptionally preferably 1:2:2.

In a further preferred embodiment of the invention a bleaching paste according to the invention or a bleaching paste that is preferred in accordance with the invention can be combined with an alkalising composition and with an oxidation composition to form a lightening or dyeing agent for keratinic fibres.

A further subject of the present invention is a method for changing the colour of keratinic fibres, in particular human hair, in which a bleaching paste according to the invention or a bleaching paste that is preferred in accordance with the invention as disclosed herein is mixed with an oxidation composition (Ox) which contains, in each case in relation to its weight, 50-96 % by weight, preferably 70-93 % by weight, particularly preferably 80-90 % by weight of water and 0.5-20 % by weight of hydrogen peroxide and also contains at least one pH adjuster in such an amount that the oxidation composition has a pH value in the range of 2.5 to 5.5, measured at 20°C, and additionally is mixed with an alkalising composition (Alk) which contains water and at least one alkalising agent which is selected from ammonia, alkanolamines and mixtures hereof and has a pH value in the range of 8-12, preferably 9-11, particularly preferably of 9.5-10.5, in each case measured at 20°C, is applied to the keratin-containing fibres directly thereafter, is left on the fibres for 5 to 60 minutes, and the fibres are then rinsed with water and the bleaching paste is optionally washed out using a surfactant-containing cleansing agent, wherein the bleaching paste (B), the oxidation composition (Ox), and the alkalising composition (Alk) are preferably mixed with one another in a weight-based ratio (B):(Ox):(Alk) of (0.7-1.3):(2-3):(2-3), particularly preferably (0.8-1.2):(2.3-2.7):(2.3-2.7), exceptionally preferably 1:2:2.

In accordance with the invention, the bleaching paste is preferably composed such that the mixture with the aforementioned oxidation composition (Ox) and with the aforementioned alkalising composition (Alk), i.e. the colour-changing agent ready for use, in particular the bleaching agent, has an alkaline pH value, preferably a pH value from 8 to 11.5, particularly preferably a pH value from 8.5 to 11, exceptionally preferably a pH value from 9.0 to 10.5, in each case measured at 20°C.

The ready-for-use mixtures of a bleaching paste according to the invention or a bleaching paste that is preferred in accordance with the invention with one of the aforementioned oxidation compositions (Ox) and optionally with one of the aforementioned alkalising compositions (Alk) preferably have a viscosity in the range of 3000 to 20000 mPas, particularly preferably 6000 to 15000 mPas, in each case measured at 20°C using a Haake cylinder/cylinder viscometer, SV I rotary/measurement system with a cooling time of 5 minutes. In this measurement method the viscosity value is determined at a shear rate of 1/7.2 s. The measurement program operates with the ramp of 0-1/60 s. A viscosity in this range means that the ready-for-use agent can be easily applied and also has such a flow behaviour that this guarantees, for the agent, a sufficiently long time of action at the site of action on the keratinic fibres.

In order to facilitate the miscibility of the alkalising composition used in accordance with the invention with the bleaching paste according to the invention or the bleaching paste preferred in accordance with the invention and the oxidation composition used in accordance with the invention and so as to also improve the use properties of the resultant mixture that is to be used, the alkalising composition used with preference in accordance with the invention preferably contains, in each case in relation to its weight, at least one surfactant in a total amount of 0.5-10 % by weight, preferably 2-8 % by weight. The surfactants suitable for the alkalising compositions (Alk) used with preference in accordance with the invention are selected from the same anionic, cationic, non-ionic, amphoteric and zwitterionic surfactants and emulsifiers disclosed further above as surfactants and emulsifiers suitable for the oxidation compositions (Ox) used with preference.

Alkalising compositions (Alk) that are used with particular preference in accordance with the invention also contain at least one oil and/or at least one fat component having a melting point in the range of 23-110°C, preferably in a total amount of 0.1-60 % by weight, particularly preferably 0.5-40 % by weight, exceptionally preferably 2-24 % by weight, in each case in relation to the weight of the alkalising composition (Alk) used with preference in accordance with the invention. The oils suitable for the alkalising compositions (Alk) used in the bleaching paste according to the invention are the same oils disclosed further above as suitable carrier medium for the bleaching powders.

Fat components having a melting point in the range of 23-110°C and used with preference in the alkalising compositions (Alk) in accordance with the invention are selected from linear saturated 1-alkanols having 12-30 carbon atoms, preferably in a total amount of 0.1-20 % by weight, particularly preferably 3-15 % by weight, exceptionally preferably 5-10 % by weight, in each case in relation to the weight of the alkalising composition used in accordance with the invention. The fatty components suitable for the alkalising compositions (Alk) used in the bleaching pastes according to the invention are the same fatty components as those disclosed above as being suitable for the bleaching powders.

The bleaching pastes according to the invention or the bleaching pastes that are preferred in accordance with the invention and/or the alkalising compositions used with preference in accordance with the invention can also contain

at least one substantive dye. The dyes suitable for the bleaching paste according to the invention are the same dyes disclosed further above as suitable for the bleaching powders.

As further optional ingredient, the alkalising composition used with preference in accordance with the invention contains at least one oxidation dye precursor, which is preferably selected from one or more developer components and optionally one or more coupler components. The developers and/or coupler components suitable for the bleaching paste according to the invention are the same developers and/or coupler components disclosed further above as suitable for the bleaching powders.

That already said with regard to the bleaching pastes according to the invention and the bleaching pastes preferred in accordance with the invention also applies, *mutatis mutandis,* to the multi-component packaging units (kits-of-parts) according to the invention and those preferred in accordance with the invention. That already said with regard to the bleaching pastes according to the invention and the bleaching pastes preferred in accordance with the invention also applies, *mutatis mutandis,* to the methods according to the invention and those preferred in accordance with the invention for lightening and/or changing the colour of keratinic fibres. That already said with regard to the oxidation compositions or alkalising compositions according to the invention and the oxidation compositions or alkalising compositions preferred in accordance with the invention also applies, *mutatis mutandis,* to the multi-component packaging units (kits-of-parts) according to the invention and those preferred in accordance with the invention. That already said with regard to the oxidation compositions or alkalising compositions according to the invention and the oxidation compositions or alkalising compositions preferred in accordance with the invention also applies, *mutatis mutandis,* to the methods according to the invention and those preferred in accordance with the invention for lightening and/or changing the colour of keratinic fibres. That already said with regard to the bleaching pastes according to the invention and the bleaching pastes preferred in accordance with the invention also applies, *mutatis mutandis,* to the use according to the invention. That already said with regard to the oxidation compositions or alkalising compositions according to the invention and the oxidation compositions or alkalising compositions preferred in accordance with the invention also applies, *mutatis mutandis,* to the use according to the invention.

## STATEMENTS OF INVENTION

The present invention may also be defined with reference to the following numbered statements:

1. A condensate reaction product obtainable according to a process comprising:

(i) reacting itaconic acid or an ester thereof with a diamine of formula $H_2N(CH_2)_nNH_2$, where n is an integer ranging from 2 to 12, wherein when n is an integer ranging from 3 to 12, the alkyl chain is a straight chain, in a molar ratio of diamine to itaconic acid or ester thereof ranging from 1:1.8 to 1:3.0 to form a first reaction mixture;
(ii) heating the first reaction mixture to form a first condensate reaction product in a second reaction mixture; and
(iii) optionally hydrolysing the second reaction mixture to form a second condensate reaction product,

wherein the first condensate reaction product and the second condensate reaction product each independently has a weight average molecular weight ($M_w$) ranging from 200 to 499 g/mol.

2. A condensate reaction product according to Statement 1, wherein itaconic acid is reacted with the diamine in step (i).

3. A condensate reaction product according to Statement 1, wherein an ester of itaconic is reacted with the diamine in step (i).

4. A condensate reaction product according to Statement 3, wherein the ester of itaconic acid is dimethyl itaconate or diethylitaconate.

5. A condensate reaction product according to any preceding Statement, wherein n is an integer ranging from 2 to 8, preferably 2 to 6.

6. A condensate reaction product according to any preceding Statement, wherein the diamine is selected from the group consisting of $H_2N\text{-}CH_2\text{-}CH_2\text{-}NH_2$, $H_2N$-(*n*-propyl)-$NH_2$, $H_2N$-(*n*-butyl)-$NH_2$, $H_2N$-(*n*-pentyl)-$NH_2$ and $H_2N$-(*n*-hexyl)-$NH_2$.

7. A condensate reaction product according to Statement 6, wherein the diamine is $H_2N\text{-}CH_2\text{-}CH_2\text{-}NH_2$.

8. A condensate reaction product according to Statement 6, wherein the diamine is $H_2N$-(*n*-propyl)-$NH_2$.

9. A condensate reaction product according to Statement 6, wherein the diamine is $H_2N$-(*n*-butyl)-$NH_2$.

10. A condensate reaction product according to Statement 6, wherein the diamine is $H_2N$-(*n*-pentyl)-$NH_2$.

11. A condensate reaction product according to Statement 6, wherein the diamine is $H_2N$-(*n*-hexyl)-$NH_2$.

12. A condensate reaction product according to Statement 1, wherein in step (i) itaconic acid is reacted with ethylene diamine.

13. A condensate reaction product according to any preceding Statement, wherein the molar ratio of diamine to itaconic acid or ester thereof ranges from 1:greater than 1.9 to 1:3.0.

14. A condensate reaction product according to any preceding Statement, wherein the molar ratio of diamine to itaconic acid or ester thereof ranges from 1:1.95 to 1:3.0.

15. A condensate reaction product according to any preceding Statement, wherein the molar ratio of diamine to itaconic acid or ester thereof ranges from 1:2.0 to 1 :3.0, preferably from 1:2.0 to 1:2.5.

16. A condensate reaction product according to any preceding Statement, wherein the molar ratio of diamine to itaconic acid or ester thereof ranges from 1:2.0 to 1:2.2.

17. A condensate reaction product according to any preceding Statement, wherein the first condensate reaction product has a weight average molecular weight ($M_w$) ranging from 200-450 g/mol.

18. A condensate reaction product according to any preceding Statement, wherein the first condensate reaction product has a weight average molecular weight ($M_w$) ranging from 250-400 g/mol.

19. A condensate reaction product according to any preceding Statement, wherein the first condensate reaction product has a weight average molecular weight ($M_w$) ranging from 300-400 g/mol.

20. A condensate reaction product according to any preceding Statement, wherein the first condensate reaction product has a weight average molecular weight ($M_w$) ranging from 320-380 g/mol.

21. A condensate reaction product according to any preceding Statement, wherein the second condensate reaction product has a weight average molecular weight ($M_w$) ranging from 200-450 g/mol.

22. A condensate reaction product according to any preceding Statement, wherein the second condensate reaction product has a weight average molecular weight ($M_w$) ranging from 250-400 g/mol.

23. A condensate reaction product according to any preceding Statement, wherein the second condensate reaction product has a weight average molecular weight ($M_w$) ranging from 300-400 g/mol.

24. A condensate reaction product according to any preceding Statement, wherein the second condensate reaction product has a weight average molecular weight ($M_w$) ranging from 320-380 g/mol.

25. A condensate reaction product according to any preceding Statement, wherein the first condensate reaction product contains no individual compounds having a molecular weight ($M_w$) greater than 1,300 g/mol, and preferably contains no individual compounds having a molecular weight (Mw) greater than 1,250 g/mol.

26. A condensate reaction product according to any preceding Statement, wherein the second condensate reaction product contains no individual compounds having a molecular weight ($M_w$) greater than 1,300 g/mol, and preferably contains no individual compounds having a molecular weight (Mw) greater than 1,250 g/mol.

27. A condensate reaction product according to any preceding Statement, wherein the first condensate reaction product has a polydispersity index ranging from 1 to 1.25.

28. A condensate reaction product according to any preceding Statement, wherein the first condensate reaction product has a polydispersity index ranging from greater than 1 to 1.15.

29. A condensate reaction product according to any preceding Statement, wherein the first condensate reaction product has a polydispersity index ranging from 1.01 to 1.10.

30. A condensate reaction product according to any preceding Statement, wherein the first condensate reaction product has a polydispersity index ranging from 1.02 to 1.06.

31. A condensate reaction product according to any preceding Statement, wherein the second condensate reaction product has a polydispersity index ranging from 1 to 1.25.

32. A condensate reaction product according to any preceding Statement, wherein the second condensate reaction product has a polydispersity index ranging from greater than 1 to 1.15.

33. A condensate reaction product according to any preceding Statement, wherein the second condensate reaction product has a polydispersity index ranging from 1.01 to 1.10.

34. A condensate reaction product according to any preceding Statement, wherein the second condensate reaction product has a polydispersity index ranging from 1.02 to 1.06.

35. A condensate reaction product according to any preceding Statement, wherein the first condensate reaction product comprises a mixture of compounds including one or more condensate compounds, wherein the or each compound independently comprises from 3 to 20 structural units, each unit based on either the itaconic-based starting material or the diamine starting material.

36. A condensate reaction product according to any preceding Statement, wherein the first condensate reaction product comprises a mixture of compounds including one or more condensate compounds, wherein the or each compound independently comprises from 3 to 15 structural units, each unit based on either the itaconic-based starting material or the diamine starting material.

37. A condensate reaction product according to any preceding Statement, wherein the first condensate reaction product comprises a mixture of compounds including one or more condensate compounds, wherein the or each compound independently comprises from 3 to 8 structural units, each unit based on either the itaconic-based starting material or the diamine starting material.

38. A condensate reaction product according to any preceding Statement, wherein the first condensate reaction product comprises condensate compounds consisting of three structural units: two structural units being based on

the itaconic-based starting material and one structural unit being based on the diamine starting material, with the structural unit based on the diamine starting material being positioned between the two units based on the itaconic-based starting material.

39. A condensate reaction product according to any preceding Statement, wherein the first condensate reaction product comprises imide and/or lactam compounds.

40. A condensate reaction product according to any preceding Statement, wherein the first condensate reaction product comprises one or more compounds with the following chemical structures:

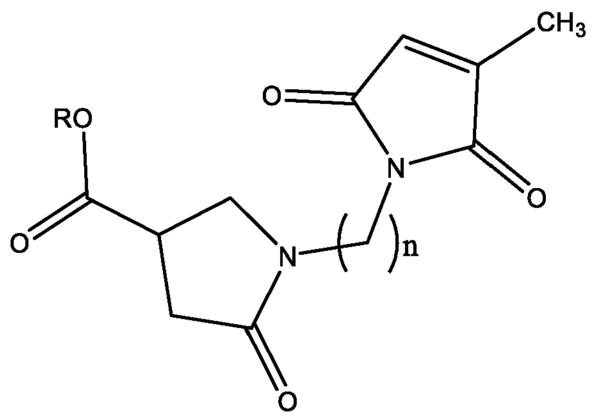

I

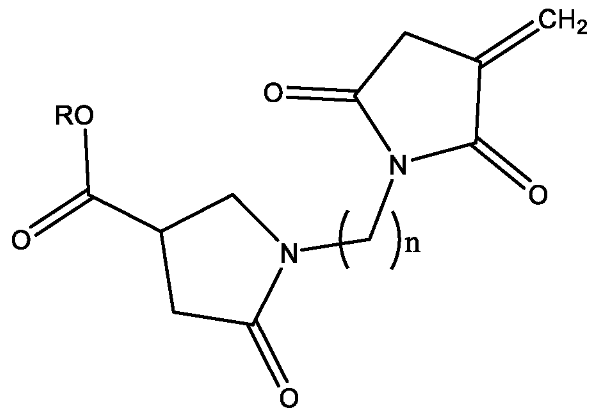

II

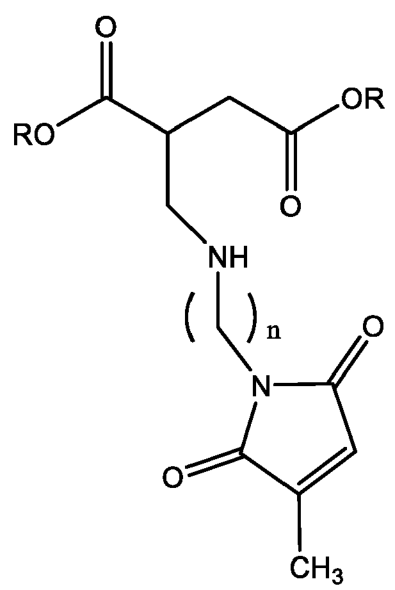

III

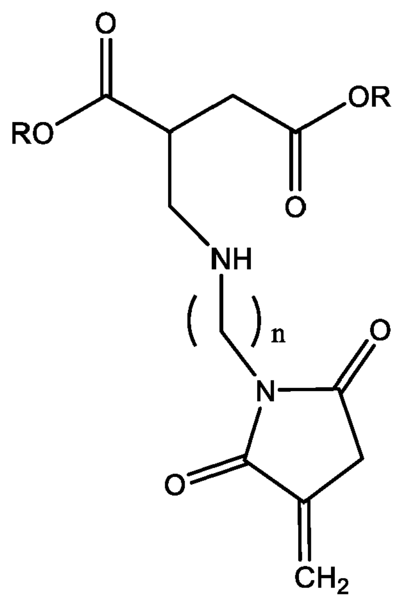

IV

wherein n is as defined in Statement 1 or 5 to 11 in relation to diamine of formula $H_2N(CH_2)_nNH_2$ and all instances of R are either hydrogen or the alkyl group corresponding to the ester of itaconic acid as defined in Statements 1, 3 or 4.

41. A condensate reaction product according to Statement 40, wherein the first condensate reaction product comprises a mixture of two or more compounds with the chemical structures I, II, III and IV.

42. A condensate reaction product according to Statement 40, wherein the first condensate reaction product comprises a mixture of three or more compounds with the chemical structures I, II, III and IV.

43. A condensate reaction product according to Statement 40, wherein the first condensate reaction product comprises a mixture of compounds with the chemical structures I, II, III and IV.

44. A condensate reaction product according to Statement 40, wherein the itaconic-based starting material is itaconic

acid and the diamine is ethylene diamine, and the first condensate reaction product comprises one or more compounds with the following chemical structures:

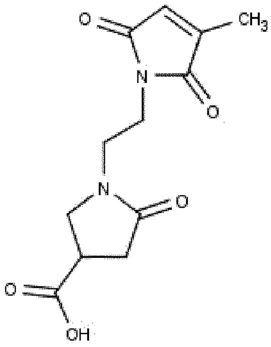

IA

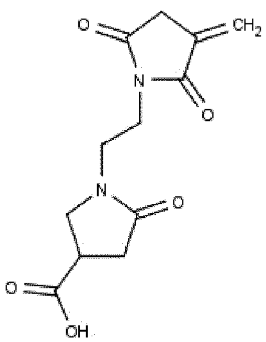

IIA

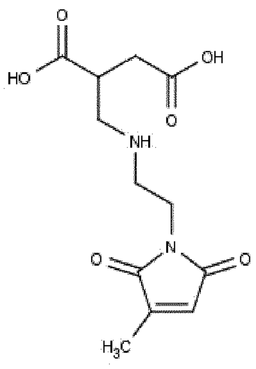

IIIA

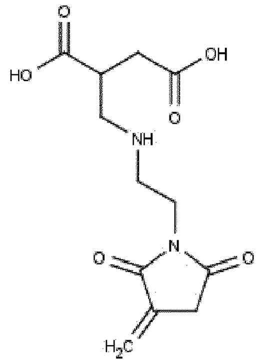

IVA

45. A condensate reaction product according to Statement 44, wherein the first condensate reaction product comprises a mixture of two or more compounds with the chemical structures IA, IIA, IIIA and IVA.
46. A condensate reaction product according to Statement 44, wherein the first condensate reaction product comprises a mixture of three or more compounds with the chemical structures IA, IIA, IIIA and IVA.
47. A condensate reaction product according to Statement 44, wherein the first condensate reaction product comprises a mixture of compounds with the chemical structures IA, IIA, IIIA and IVA.
48. A condensate reaction product according to any preceding Statement, wherein the second condensate reaction product comprises a mixture of compounds including one or more condensate compounds, wherein the or each compound independently comprises from 3 to 20 structural units, each unit based on either the itaconic-based starting material or the diamine starting material.
49. A condensate reaction product according to any preceding Statement, wherein the second condensate reaction product comprises a mixture of compounds including one or more condensate compounds, wherein the or each compound independently comprises from 3 to 15 structural units, each unit based on either the itaconic-based starting material or the diamine starting material.
50. A condensate reaction product according to any preceding Statement, wherein the second condensate reaction product comprises a mixture of compounds including one or more condensate compounds, wherein the or each compound independently comprises from 3 to 8 structural units, each unit based on either the itaconic-based starting material or the diamine starting material.
51. A condensate reaction product according to any preceding Statement, wherein the second condensate reaction product comprises condensate compounds consisting of three structural units: two structural units being based on the itaconic-based starting material and one structural unit being based on the diamine starting material, with the structural unit based on the diamine starting material being positioned between the two units based on the itaconic-based starting material.
52. A condensate reaction product according to any preceding Statement, wherein the second condensate reaction product comprises no imide compounds.
53. A condensate reaction product according to any preceding Statement, wherein the second condensate reaction product comprises compound V and/or compound VI in the free base form or as a salt thereof

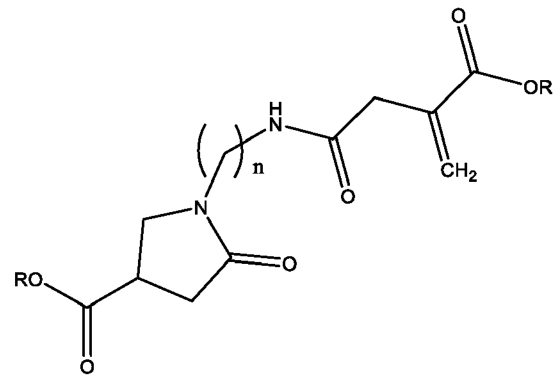

V

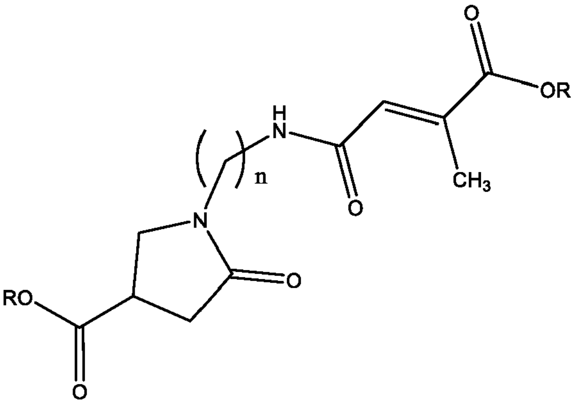

VI

wherein n is as defined in Statement 1 or 5 to 11 in relation to diamine of formula H2N(CH2)nNH2 and all instances of R are either hydrogen or the alkyl group corresponding to the ester of itaconic acid as defined in Statements 1, 3 or 4.

54. A condensate reaction product according to Statement 53, wherein the second condensate reaction product comprises a mixture of both compounds with the chemical structures V and VI.

55. A condensate reaction product according to Statement 53, wherein the itaconic-based starting material is itaconic acid and the diamine is ethylene diamine, and the second condensate reaction product comprises compounds VA and/or VIA in the free base form or as a salt thereof.

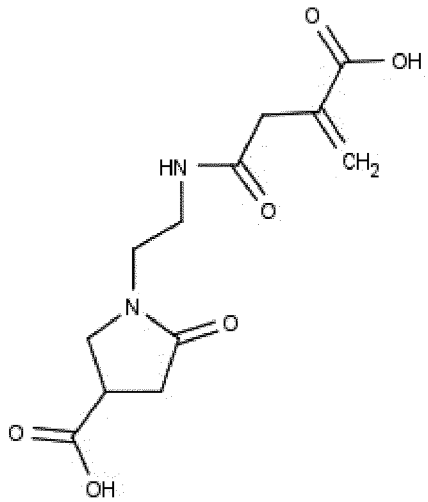

VA

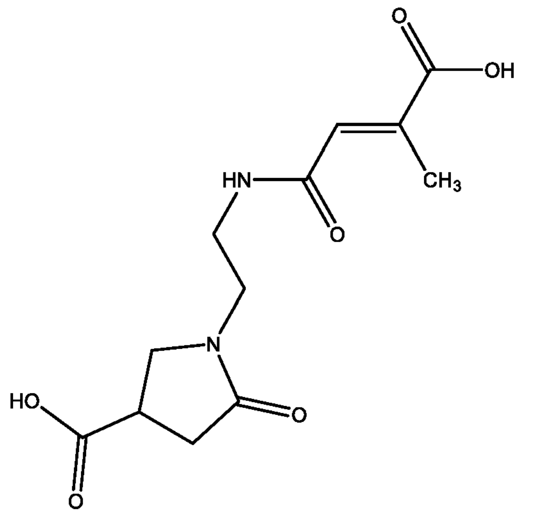

VIA

56. A condensate reaction product according to Statement 55, wherein the second condensate reaction product comprises a mixture of both compounds with the chemical structures VA and VIA.

57. A condensate reaction product according to any one of Statements 53 to 56, wherein the compound of formula V or VA is in the form of the free base and/or the compound of formula VI or VIA is in the form of the free base.

58. A condensate reaction product according to any one of Statements 53 to 56, wherein the compound of formula V or VA is in the form of a salt with an alkali metal, preferably the sodium or potassium salt of compound V and/or the compound of formula VI or VIA is in the form of a salt with an alkali metal, preferably the sodium or potassium salt of compound V.

59. A condensate reaction product according to Statement 58, wherein the salt is the sodium salt.

60. A condensate reaction product according to Statement 58, wherein the salt is the potassium salt.

61. A condensate reaction product according to any one of Statements 53 to 56, wherein the compound of formula V or VA is in the form of a salt with an alkaline earth metal, preferably the magnesium or calcium salt of compound

V and/or the compound of formula VI or VIA is in the form of a salt with an alkaline earth metal, preferably the magnesium or calcium salt of compound V.

62. A condensate reaction product according to Statement 61, wherein the wherein the salt is the magnesium salt.

63. A condensate reaction product according to Statement 61, wherein the salt is the calcium salt.

64. A condensate reaction product according to any one of Statements 35 to 63, wherein the first and/or second condensate reaction product comprises one or more further compounds within the reaction product, the or each compound having from 5 to 15 structural units, the structural units in the or each individual compounds being from a majority of structural units derived from the itaconic-based starting material and a corresponding minority of structural units derived from the diamine starting material.

65. A condensate reaction product according to Statement 64, wherein the first and/or second condensate reaction product comprises one or more further compounds within the reaction product, the or each compound having from 5 to 13 structural units; preferably from 5 to 11 structural units.

66. A condensate reaction product according to Statement 64 or 65, wherein the first condensate reaction product comprises one or more further compounds within the reaction product, the or each compound having one or more compounds having a ratio of structural units derived from itaconic-based starting material:diamine of 3:2, 4:3, 4:4, 5:4, 4:2, 5:3, 6:5 and 7:6 and mixtures thereof.

67. A condensate reaction product according to Statement 64, 65 or 66, wherein the second condensate reaction product comprises one or more further compounds within the reaction product, the or each compound having one or more compounds having a ratio of structural units derived from itaconic-based starting material:diamine of 3:2, 4:3, 4:4, 5:4, 4:2, 5:3, 6:5 and 7:6 and mixtures thereof.

68. A condensate reaction product according to any preceding Statement, wherein steps (i), (ii) and (iii) are carried out in the absence of a solvent.

69. A condensate reaction product according to any one of Statements 1 to 67, wherein steps (i), (ii) and (iii) are carried out in the presence of a solvent selected from water, an alcohol or a mixture thereof.

70. A condensate reaction product according to Statement 69, wherein steps (i), (ii) and (iii) are carried out in the presence of a solvent of solely water.

71. A condensate reaction product according to Statement 69, wherein the itaconic-based starting material is dimethyl itaconate, and steps (i), (ii) and (iii) are carried out in the presence of a solvent of a methanol-water mixture or solely methanol.

72. A condensate reaction product according to any Statement 69, wherein the itaconic-based starting material is diethyl itaconate, and steps (i), (ii) and (iii) are carried out in the presence of a solvent of an ethanol-water mixture or solely ethanol.

73. A condensate reaction product according to any preceding Statement, wherein the process is carried out without hydrolysis step (iii), and the first condensate reaction product is isolated as a solid.

74. A condensate reaction product according to any one of Statements 1 to 72, wherein the process is carried out with the hydrolysis step (iii), and the product of step (ii) is isolated as a solid before the hydrolysis step.

75. A condensate reaction product according to any one of Statements 1 to 72, wherein the process is carried out with the hydrolysis step (iii), and the product of step (ii) is not isolated as a solid before the hydrolysis step.

76. A condensate reaction product according to Statement 74 or 75, wherein the second reaction product is isolated as a solid.

77. A condensate reaction product obtainable according to a process comprising:

(i) reacting itaconic acid or an ester thereof with a diamine of formula $H_2N(CH_2)_nNH_2$, where n is an integer ranging from 2 to 12, wherein when n is an integer ranging from 3 to 12, the alkyl chain is a straight chain; and
(ii) heating the first reaction mixture to form the condensate reaction product,

wherein the condensate reaction product comprises one or more of the following compounds:

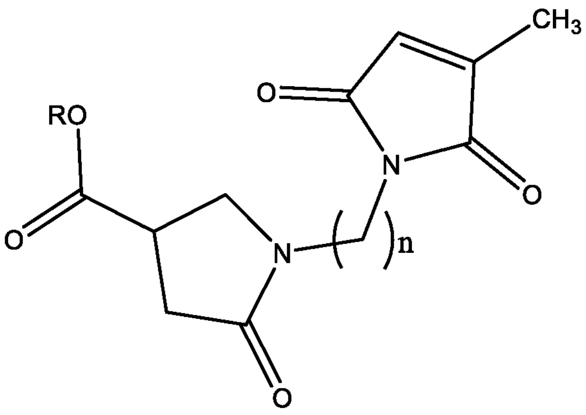

I

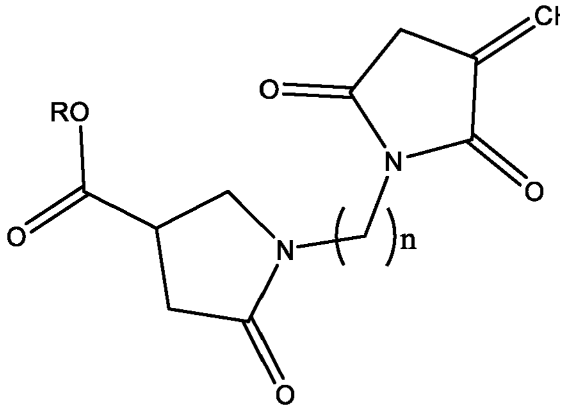

II

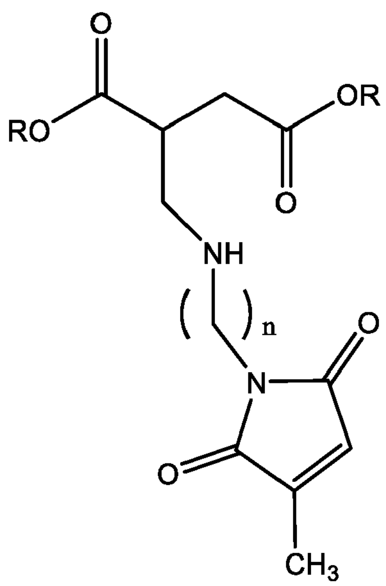

III

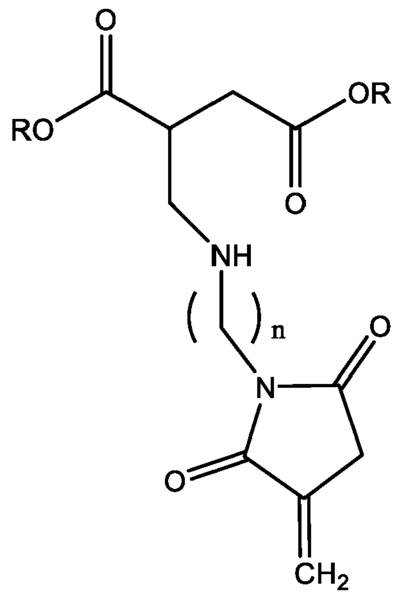

IV

wherein n is as defined in Statement 1 in relation to diamine of formula $H_2N(CH_2)_nNH_2$ and all instances of R are either hydrogen or the alkyl group corresponding to the ester of itaconic acid as defined in Statement 1.

78. A condensate reaction product according to Statement 77, wherein the starting materials in step (i) are reacted in a molar ratio of diamine to itaconic acid or ester thereof ranging from 1:1.8 to 1:3.0, preferably from 1:1.9 to 1:3.0, more preferably from 1:1.95 to 1:3.0; yet more preferably from 1:2.0 to 1:3.0 and most preferably from 1:2.0 to 1:2.3.

79. A condensate reaction product according to Statement 77 or 78 which has a weight average molecular weight (Mw) ranging from 200 to 499 g/mol, or 200-450 g/mol, or 250-400 g/mol, or 300-400 g/mol, or 320-380 g/mol.

80. A condensate reaction product according to any one of Statements 77 to 79, which contains no individual compounds having a molecular weight (Mw) greater than 1,300 g/mol, and preferably contains no individual compounds having a molecular weight (Mw) greater than 1,250 g/mol.

81. A condensate reaction product according to any one of Statements 77 to 80, which has a polydispersity index ranging from 1 to 1.25, preferably greater than 1 to 1.15, more preferably 1.01 to 1.10, most preferably 1.02 to 1.06.

82. A condensate reaction product according to any one of Statements 77 to 81, wherein n is an integer ranging from 2 to 8, more preferably from 2 to 6.

83. A condensate reaction product according to any one of Statements 77 to 82, wherein the diamine starting material is preferably selected from the group consisting of ethylene diamine, trimethylene diamine, butamethylene diamine, pentamethylene diamine and hexamethylene diamine, preferably ethylene diamine, pentamethylene diamine and

hexamethylene diamine.

84. A condensate reaction product according to any one of Statements 77 to 83, wherein the itaconic-based starting material is itaconic acid.

85. A condensate reaction product according to any one of Statements 77 to 83, wherein the itaconic-based starting material is an ester of itaconic acid.

86. A condensate reaction product according to Statement 84, wherein the diamine is ethylene diamine.

87. A condensate reaction product according to Statement 84, wherein the diamine is pentamethylene diamine.

88. A condensate reaction product according to Statement 84, wherein the diamine is hexamethylene diamine.

89. A condensate reaction product according to Statement 86, wherein the compounds of formula I to IV have the following structures:

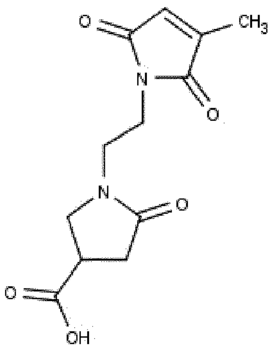
IA

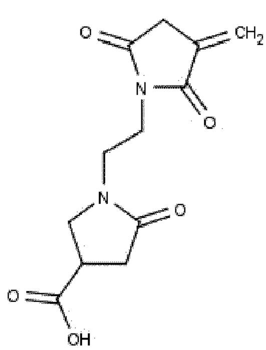
IIA

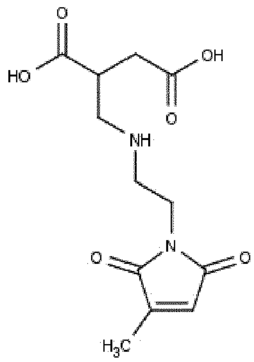
IIIA

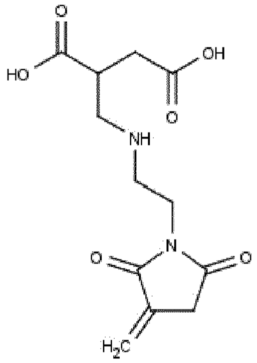
IVA

90. A condensate reaction product according to Statement 89, which comprises a mixture of two or more compounds with the chemical structures IA, IIA, IIIA and IVA.

91. A condensate reaction product according to Statement 89, which comprises a mixture of three or more compounds with the chemical structures IA, IIA, IIIA and IVA.

92. A condensate reaction product according to Statement 89, which comprises a mixture of compounds with the chemical structures IA, IIA, IIIA and IVA.

93. A second condensate reaction product obtainable according to a process comprising:

(i) reacting itaconic acid or an ester thereof with a diamine of formula $H_2N(CH_2)_nNH_2$, where n is an integer ranging from 2 to 12, wherein when n is an integer ranging from 3 to 12, the alkyl chain is a straight chain;
(ii) heating the first reaction mixture to form a first condensate reaction product; and
(iii) hydrolysing the product of step (ii) to form the second condensate reaction product,

wherein the second condensate reaction product comprises compound V and/or compound VI or salts thereof:

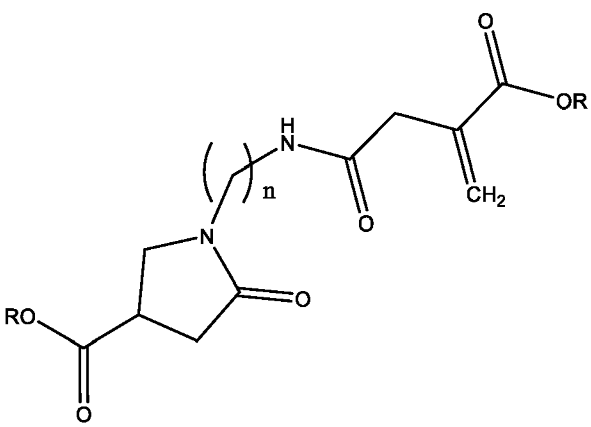
V

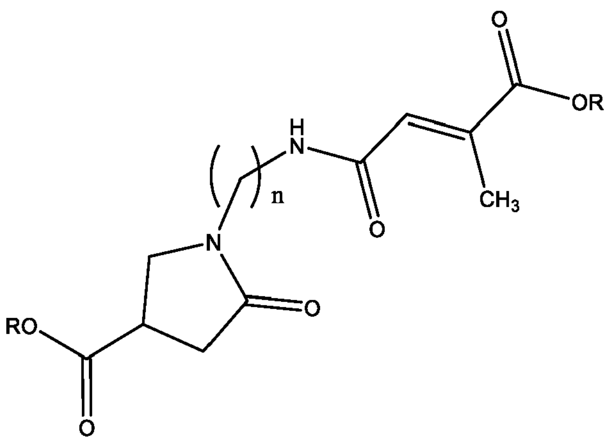
VI

wherein n is as defined in Statement 1 in relation to diamine of formula $H_2N(CH_2)_nNH_2$ and all instances of R are either hydrogen or the alkyl group corresponding to the ester of itaconic acid as defined in Statement 1.

94. A second condensate reaction product according to Statement 93, wherein the starting materials in step (i) are reacted in a molar ratio of diamine to itaconic acid or ester thereof ranging from 1:1.8 to 1:3.0, preferably from 1:1.9 to 1:3.0, more preferably from 1:1.95 to 1:3.0; yet more preferably from 1:2.0 to 1:3.0 and most preferably from 1:2.0 to 1:2.3.

95. A second condensate reaction product according to Statement 93 or 94 which has a weight average molecular weight (Mw) ranging from either 200 to 499 g/mol, suitably from 200-450 g/mol, or 250-400 g/mol, or 300-400 g/mol, or 320-380 g/mol.

96. A second condensate reaction product according to any one of Statements 93 to 95, which contains no individual compounds having a molecular weight (Mw) greater than 1,300 g/mol, and preferably contains no individual compounds having a molecular weight (Mw) greater than 1,250 g/mol.

97. A second condensate reaction product according to any one of Statements 93 to 96, which has a polydispersity index ranging from 1 to 1.25, preferably greater than 1 to 1.15, more preferably 1.01 to 1.10, most preferably 1.02 to 1.06.

98. A second condensate reaction product according to any one of Statements 93 to 97, wherein n is an integer ranging from 2 to 8, more preferably from 2 to 6.

99. A second condensate reaction product according to any one of Statements 93 to 98, wherein the diamine starting material is preferably selected from the group consisting of ethylene diamine, trimethylene diamine, butamethylene diamine, pentamethylene diamine and hexamethylene diamine, preferably ethylene diamine, pentamethylene diamine and hexamethylene diamine.

100. A second condensate reaction product according to any one of Statements 99, wherein the itaconic-based starting material is itaconic acid and the diamine is ethylene diamine.

101. A second condensate reaction product according to any one of Statements 99, wherein the itaconic-based starting material is itaconic acid and the diamine is pentamethylene diamine.

102. A second condensate reaction product according to any one of Statements 99, wherein the itaconic-based starting material is itaconic acid and the diamine is hexamethylene diamine.

103. A second condensate reaction product according to any one of Statements 99, wherein itaconic acid is reacted with ethylene diamine, and the compound of formula V has the following structu re:

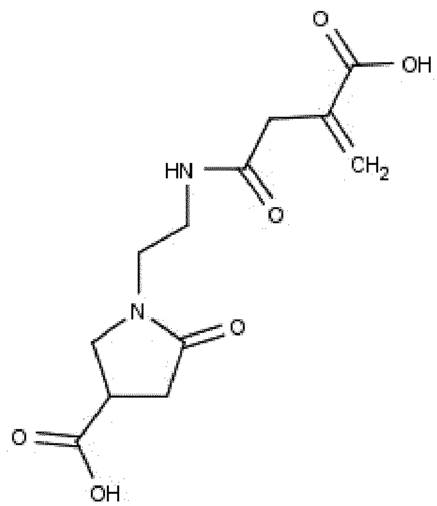

VA

104. A process for preparing a condensate reaction product comprising:

(i) reacting itaconic acid or an ester thereof with a diamine of formula $H_2N(CH_2)_nNH_2$, where n is an integer ranging from 2 to 12, wherein when n is an integer ranging from 3 to 12, the alkyl chain is a straight chain, in a molar ratio of diamine to itaconic acid or ester thereof ranging from 1:1.8 to 1:3.0 to form a first reaction mixture;
(ii) heating the first reaction mixture to form a first condensate reaction product in a second reaction mixture; and
(iii) optionally hydrolysing the second reaction mixture to form a second condensate reaction product,

wherein the first condensate reaction product and the second condensate reaction product each independently has a weight average molecular weight ($M_w$) ranging from 200 to 499 g/mol.

105. A process according to Statement 104, wherein the process is as defined in any one of Statements 1 to 76.

106. A composition comprising a mixture of one or more compounds with the following chemical structures:

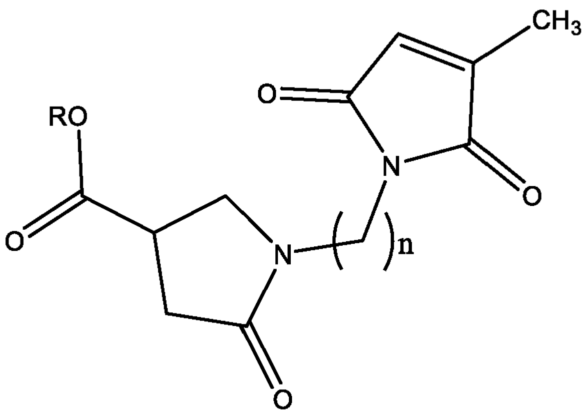

I

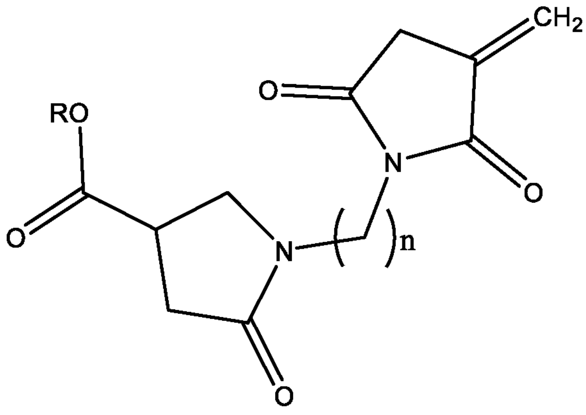

II

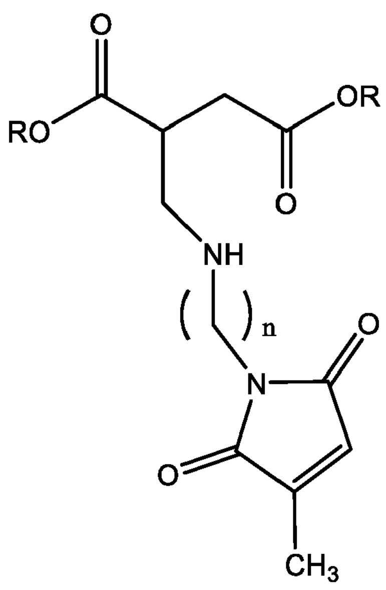

III

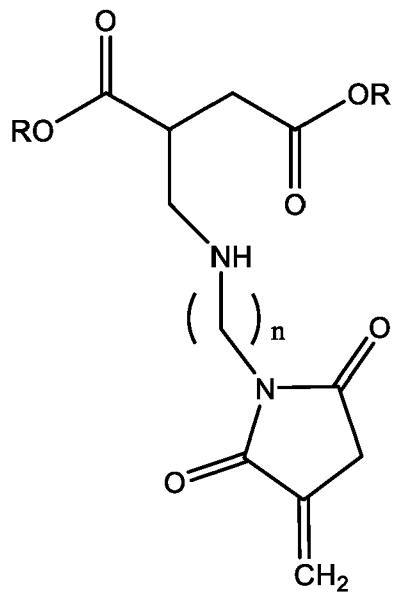

IV

wherein n is an integer ranging from 2 to 12, preferably 2 to 6, wherein when n is an integer ranging from 3 to 12, the alkyl chain is a straight chain, and all instances of R are either hydrogen or methyl or ethyl.

107. The composition according to Statement 106, comprising a mixture of two or more compounds with the chemical structures I, II, III and IV.

108. The composition according to Statement 106, comprising a mixture of three or more compounds with the chemical structures I, II, III and IV.

109. The composition according to Statement 106, comprising a mixture of compounds with the chemical structures I, II, III and IV.

110. The composition according to Statement 106, comprising one or more compounds with the following chemical structures:

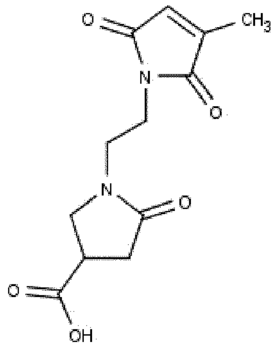 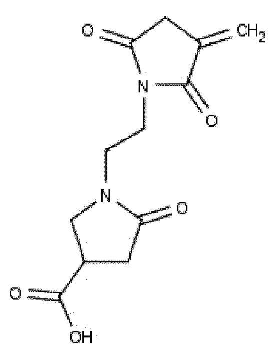 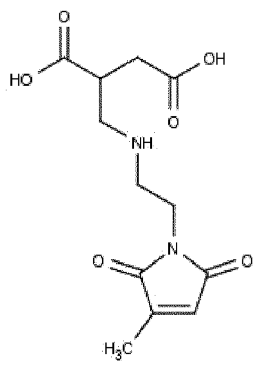 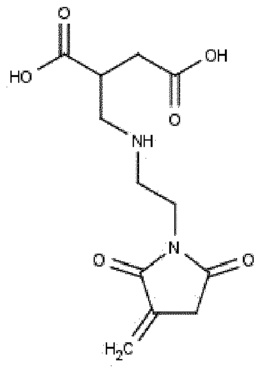

IA IIA IIIA IVA

111. The composition according to Statement 110, comprising a mixture of two or more compounds with the chemical structures IA, IIA, IIIA and IVA.
112. The composition according to Statement 110, comprising a mixture of three or more compounds with the chemical structures IA, IIA, IIIA and IVA.
113. The composition according to Statement 110, comprising a mixture of compounds with the chemical structures IA, IIA, IIIA and IVA.
114. The composition according to any one of Statements 106 to 113, prepared according to steps (i) and (ii) as defined in claim 104, or any embodiment of said process as disclosed herein.
115. The composition according to any one of Statements 106 to 113, prepared according to steps (i) and (ii) as defined in claim 105 or any embodiment of said process as disclosed herein.
116. A composition comprising compound V and/or compound VI, one or both compounds independently being in the free base form or as a salt thereof

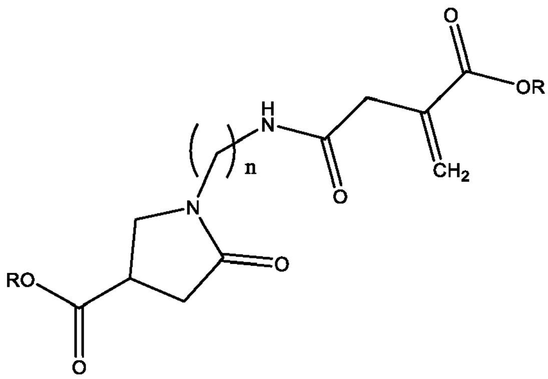 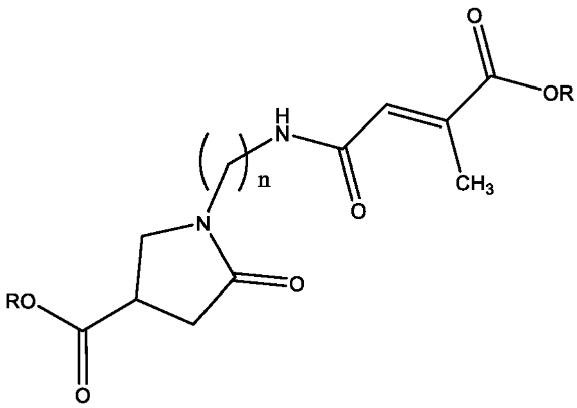

V VI

wherein n is an integer ranging from 2 to 12, preferably 2 to 6, wherein when n is an integer ranging from 3 to 12, the alkyl chain is a straight chain, and all instances of R are either hydrogen or methyl or ethyl.
117. The composition according to Statement 116, comprising a mixture of both compounds with the chemical structures V and VI.
118. The composition according to Statement 116, comprising compounds VA and/or VIA, one or both compounds independently being in the free base form or as a salt thereof.

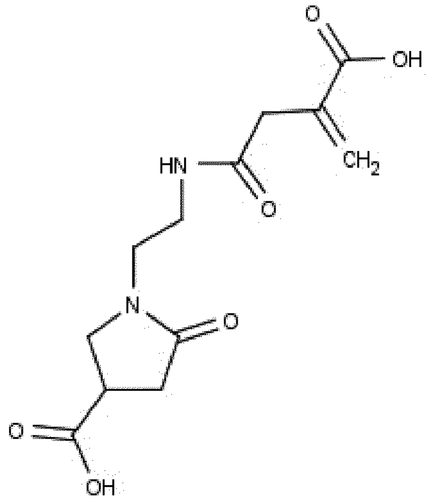

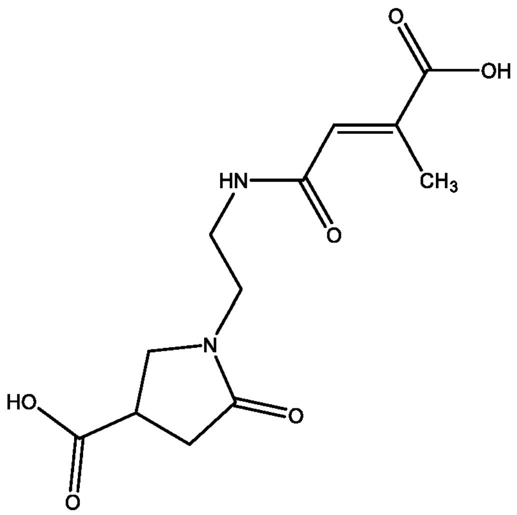

VA VIA

119. The composition according to Statement 116, comprising a mixture of both compounds with the chemical structures VA and VIA.
120. The composition according to any one of Statements 116 to 119, wherein, when present, the compounds of formula V, VA, VI, and VIA are in the form of the free base.
121. The composition according to any one of Statements 116 to 119, wherein, when present, the compounds of formula V, VA, VI and VIA are in the form of a salt with an alkali metal, preferably the sodium or potassium salt.
122. The composition according to Statement 121, wherein the salt is the sodium salt.
123. The composition according to Statement 121, wherein the salt is the potassium salt.
124. The composition according to any one of Statements 116 to 120, wherein, when present, the compounds of formula V, VA, VI, and VIA are in the form of a salt with an alkaline earth metal, preferably the magnesium or calcium salt.
125. The composition according to Statement 124, wherein the salt is the magnesium salt.
126. The composition according to Statement 124, wherein the salt is the calcium salt.
127. The composition according to any one of Statements 116 to 126, comprising no imide products.
128. The composition according to any one of Statements 116 to 127, comprising no imide products and no lactam products.
129. The composition according to any one of Statements 116 to 128, prepared according to steps (i), (ii) and (iii) as defined in claim 104, or any embodiment of said process as disclosed herein.
130. The composition according to any one of Statements 116 to 128, prepared according to steps (i), (ii) and (iii) as defined in claim 105, or any embodiment of said process as disclosed herein.
131. The use of the first condensate reaction product as defined in any one of Statements 1 to 92 or the composition as defined in any one of Statements 106 to 115 as a functional additive.
132. The use of the second condensate reaction product as defined in any one of Statements 1 to 76 or the composition as defined in any one of Statements 116 to 130 as a functional additive.
133. A detergent composition comprising the first condensate reaction product as defined in any one of Statements 1 to 92 or the composition as defined in any one of Statements 106 to 115.
134. A detergent composition comprising the second condensate reaction product as defined in any one of Statements 1 to 76 or as defined in any one of Statements 93 to 103 or the composition as defined in any one of Statements 116 to 130.
135. A cleaning composition comprising the first condensate reaction product as defined in any one of Statements 1 to 92 or the composition as defined in any one of Statements 106 to 115.
136. A cleaning composition comprising the second condensate reaction product as defined in any one of Statements 1 to 76 or as defined in any one of Statements 93 to 103 or the composition as defined in any one of Statements 116 to 130.
137. An adhesive composition comprising the first condensate reaction product as defined in any one of Statements 1 to 92 or the composition as defined in any one of Statements 116 to 130.
138. An adhesive composition comprising the second condensate reaction product as defined in any one of Statements 1 to 76 or as defined in any one of Statements 93 to 103 or the composition as defined in any one of Statements 116 to 130.

139. A cosmetic composition comprising the first condensate reaction product as defined in any one of Statements 1 to 92 or the composition as defined in any one of Statements 106 to 115.

140. A cosmetic composition comprising the second condensate reaction product as defined in any one of Statements 1 to 76 or as defined in any one of Statements 93 to 103 or the composition as defined in any one of Statements 116 to 130.

141. Use of a cosmetic composition according to Statement 114 or 115 or 139 or 140 in a method for the oxidative lightening and/or dyeing of keratinous fibres.

142. A bleaching powder comprising the first condensate reaction product as defined in any one of Statements 1 to 92 or the composition as defined in any one of Statements 106 to 115.

143. A bleaching powder comprising the second condensate reaction product as defined in any one of Statements 1 to 76 or as defined in any one of Statements 93 to 103 or the composition as defined in any one of Statements 116 to 130.

144. A bleaching powder according to Statement 117 or 118, further comprising an oxidising agent.

145. A bleaching powder comprising:

a) at least one oxidising agent selected from sodium percarbonates and inorganic salts of a peroxysulfuric acid, and mixtures thereof, and

b) at least one complexing agent comprising:

(i) the first condensate reaction product as defined in any one of Statements 1 to 92 or the composition as defined in any one of Statements 106 to 115,

(ii) the second condensate reaction product as defined in any one of Statements 1 to 76 or as defined in any one of Statements 93 to 103 or the composition as defined in any one of Statements 116 to 130, or

(iii) mixtures of (i) and (ii).

146. A bleaching powder according to Statement 145, wherein a total amount of the complexing agent is 0.1 - 2.5 % by weight of the powder.

147. A bleaching powder according to Statement 146, wherein a total amount of the complexing agent is 0.1 - 2.0 % by weight of the powder, preferably 0.1 - 1.8% by weight of the powder, more preferably 0.1 - 1.6 % by weight of the powder, yet more preferably 0.5 - 1.6 % by weight of the powder.

148. A bleaching powder according to any one of Statements 145 to 147, wherein the complexing agent is (i).

149. A bleaching powder according to any one of Statements 145 to 147, wherein the complexing agent is (ii).

150. A bleaching powder according to any one of Statements 145 to 147, wherein the complexing agent is (iii).

151. A bleaching powder according to any one of Statements 145 to 150, wherein the complexing agent b) further comprises one or more of the following acids and/or alkali metal salts thereof: 1-hydroxyethane 1,1-diphosphonic acid (HEDP); ethylenediaminetetraacetic acid (EDTA); N-hydroxyethylethylenediaminetriacetic acid; aminotrimethylenephosphonic acid; diethylenetriaminepentaacetic acid; lauroyl ethylenediaminetriacetic acid; nitrilotriacetic acid; iminodisuccinic acid; N-2-hydroxyethyliminodiacetic acid; ethylene glycol-bis-(beta-aminoethyl ether)-N,N-tetraacetic acid; amino trimethylene phosphonic acid, and pentasodium amino trimethylene phosphonate.

152. A bleaching powder according to any one of Statements 145 to 151, wherein the at least one oxidising agent a) further comprises

(i) at least one dicarboxylic acid having 2 to 10 carbon atoms, selected from succinic acid, malic acid, oxalic acid, malonic acid, adipic acid, pimelic acid, suberic acid, azelaic acid, sebacic acid, maleic acid, fumaric acid, D-tartaric acid, L-tartaric acid, meso-tartaric acid, racemic acid, alpha-ketoglutaric acid, beta-ketoglutaric acid, oxaloacetic acid and/or at least one salt of these acids and mixtures of these compounds, wherein the dicarboxylic acid having 2 to 10 carbon atoms is preferably selected from succinic acid, malic acid, maleic acid and the salts of succinic acid, malic acid or maleic acid, and

(ii) at least one amino acid, selected from arginine, lysine, histidine or at least one of the salts of these amino acids.

153. A bleaching paste comprising the first condensate reaction product as defined in any one of Statements 1 to 92 or the composition as defined in any one of Statements 106 to 115.

154. A bleaching paste comprising the second condensate reaction product as defined in any one of Statements 1 to 76 or as defined in any one of Statements 93 to 103 or the composition as defined in any one of Statements 116 to 130.

155. A bleaching paste according to any Statement 152 or 154, further comprising an oxidising agent.

156. A bleaching paste comprising:

a) at least one oxidising agent selected from sodium percarbonates and inorganic salts of a peroxysulfuric acid, and mixtures thereof;
b) at least one complexing agent comprising:

(i) the first condensate reaction product according to any one of Statements 1 to 92 or the composition as defined in any one of Statements 106 to 115,
(ii) the second condensate reaction product according to any one of Statements 1 to 76 or as defined in any one of Statements 93 to 103 or the composition as defined in any one of Statements 116 to 130, or
(iii) mixtures of (i) and (ii);

c) at least one oil; and
d) 0 to 4 % by weight of water, in relation to the weight of the bleaching paste.

157. A bleaching paste according to Statement 156, wherein the at least one oil is present in a total amount of 16-60 % by weight, preferably 20-50 % by weight, particularly preferably 25-45 % by weight, in each case in relation to the weight of the bleaching paste.
158. A bleaching paste according to Statement 156 or 157, wherein the complexing agent b) is (i).
159. A bleaching paste according to any one of Statement 156 or 157, wherein the complexing agent b) is (ii).
160. A bleaching paste according to Statement 156 or 157, wherein the complexing agent b) is (iii).
161. A bleaching paste according to any one of Statements 156 to 160, wherein the complexing agent b) further comprises one or more of the following acids and/or alkali metal salts thereof: 1-hydroxyethane 1,1-diphosphonic acid (HEDP); ethylenediaminetetraacetic acid (EDTA); N-hydroxyethylethylenediaminetriacetic acid; aminotrimethylenephosphonic acid; diethylenetriaminepentaacetic acid; lauroyl ethylenediaminetriacetic acid; nitrilotriacetic acid; iminodisuccinic acid; N-2-hydroxyethyliminodiacetic acid; ethylene glycol-bis-(beta-aminoethyl ether)-N,N-tetraacetic acid; amino trimethylene phosphonic acid, and pentasodium amino trimethylene phosphonate.
162. A bleaching paste according to any one of Statements 156 to 161, wherein the at least one oxidising agent a) further comprises

(iii) at least one dicarboxylic acid having 2 to 10 carbon atoms, selected from succinic acid, malic acid, oxalic acid, malonic acid, adipic acid, pimelic acid, suberic acid, azelaic acid, sebacic acid, maleic acid, fumaric acid, D-tartaric acid, L-tartaric acid, meso-tartaric acid, racemic acid, alpha-ketoglutaric acid, beta-ketoglutaric acid, oxaloacetic acid and/or at least one salt of these acids and mixtures of these compounds, wherein the dicarboxylic acid having 2 to 10 carbon atoms is preferably selected from succinic acid, malic acid, maleic acid and the salts of succinic acid, malic acid or maleic acid, and
(iv) at least one amino acid, selected from arginine, lysine, histidine or at least one of the salts of these amino acids.

163. A method for lightening keratin fibres, in particular human hair, which method is characterised in that

a) a bleaching powder according to any one of Statements 142 to 152 is mixed with an oxidation composition (Ox),
b) said mixture is applied to the keratin-containing fibres directly thereafter, and is left on the fibres for 5 to 60 minutes, and
c) the fibres are then rinsed with water and the mixture is optionally washed out using a surfactant-containing cleansing agent.

164. A method according to Statement 163, wherein the oxidation composition (Ox) which contains, in each case in relation to its weight, 50 - 96 % by weight, preferably 70 - 93 % by weight, particularly preferably 80 - 90 % by weight of water and 0.5 - 20 % by weight of hydrogen peroxide and also at least one pH adjustor in such an amount that the oxidation composition has a pH value in the range of 2.5 to 5.5, measured at 20°C.
165. A method according to Statement 162 or 163, wherein the bleaching powder (B) and the oxidation composition (Ox) are preferably mixed with one another in a weight-based ratio (B):(Ox) of 0.2 - 1, particularly preferably 0.3 - 0.8, more preferably 0.4 - 0.7, extremely preferably 0.5 - 0.6.
166. A multi-component packaging unit (kit-of-parts) for lightening keratin fibres, in particular human hair, containing at least two components packaged separately from one another, characterised in that

(i) the first component (I) is a bleaching powder according to any one of Statements 142 to 152, and
(ii) the second component (II) is an oxidation composition (Ox).

167. A multi-component packaging unit (kit-of-parts) according to Statement 166 which contains, in each case in relation to its weight, 50 - 96 % by weight, preferably 70 - 93 % by weight, particularly preferably 80 - 90 % by weight of water and 0.5 - 20 % by weight of hydrogen peroxide and has a pH value in the range of 2.5 to 5.5, measured at 20°C, wherein the components (I) and (II) are preferably present in a weight-based ratio to one another (I):(II) of 0.2 - 1, particularly preferably 0.3 - 0.8, more preferably 0.4 - 0.7, extremely preferably 0.5 - 0.6.
168. A method for changing the colour of keratin fibres, in particular human hair, in which

a) a bleaching powder according to any one of Statements 142 to 152, is mixed with an oxidation composition (Ox),
b) said mixture is applied to the keratin-containing fibres directly thereafter, is left on the fibres for 5 to 60 minutes, and
c) the fibres are then rinsed with water and the mixture is optionally washed out using a surfactant-containing cleansing agent.

169. A method according to Statement 168, wherein the oxidation composition (Ox) contains, in each case in relation to its weight, 50 - 96 % by weight, preferably 70 - 93 % by weight, particularly preferably 80 - 90 % by weight of water and 0.5 - 20 % by weight of hydrogen peroxide and also at least one pH adjustor in such an amount that the oxidation composition has a pH value in the range of 2.5 to 5.5, measured at 20°C.
170. A method according to Statement 168 and 169, wherein the oxidation composition (Ox) and the bleaching powder are additionally mixed with an alkalising composition (Alk) which contains water and at least one alkalising agent which is selected from ammonia, alkanolamines and mixtures thereof and has a pH value in the range of 8 - 12, preferably 9 - 11, particularly preferably of 9.5 - 10.5, in each case measured at 20°C.
171. A method according to Statement 170, wherein the bleaching powder (B), the oxidation composition (Ox), and the alkalising composition (Alk) are preferably mixed with one another in a weight-based ratio (B):(Ox):(Alk) of (0.7 - 1.3):(2 - 3):(2 - 3), particularly preferably (0.8 - 1.2):(2.3 - 2.7):(2.3 - 2.7), extremely preferably 1:2:2.
172. A multi-component packaging unit (kit-of-parts) for changing the colour of keratin fibres, in particular human hair, containing at least three components packaged separately from one another, characterised in that

i) the first component (I) is a bleaching powder according to any one of Statements 142 to 152
ii) the second component (II) is an oxidation composition (Ox), and
iii) the third component (III) is an alkalising composition (Alk).

173. A multi-component packaging unit (kit-of-parts) according to Statement 172, wherein the oxidation composition (Ox) contains, in each case in relation to its weight, 50 - 96 % by weight, preferably 70 - 93 % by weight, particularly preferably 80 - 90 % by weight of water and 0.5 - 20 % by weight of hydrogen peroxide and has a pH value in the range of 2.5 to 5.5, measured at 20°C, and the alkalising composition (Alk) contains water and at least one alkalising agent, which is selected from ammonia, alkanolamines and mixtures thereof, and has a pH value in the range of 8 - 12, preferably of 9 - 11, particularly preferably of 9.5 - 10.5, in each case measured at 20°C,
wherein the bleaching powder (B), the oxidation composition (Ox) and the alkalising composition (Alk) are preferably present in a weight-based ratio to one another (B):(Ox):(Alk) of (0.7 - 1.3):(2 - 3):(2 - 3), particularly preferably (0.8 - 1.2):(2.3 - 2.7):(2.3 - 2.7), extremely preferably 1:2:2.
174. Use of a complexing agent comprising:

(i) the first condensate reaction product according to any one of Statements 1 to 92 or the composition as defined in any one of Statements 106 to 115,
(i) the second condensation reaction product according to any one of Statements 1 to 76 or any one of Statements 93 to 103 or the composition as defined in any one of Statements 116 to 130, or
(ii) mixtures of (i) and (ii)

in a bleaching powder which further contains at least one oxidising agent, for reducing damage to keratinic fibres, in particular human hair, caused by the treatment of these fibres with the bleaching paste.
175. A method for lightening keratinic fibres, in particular human hair, which method is characterised in that

a) a bleaching paste according to any one of Statements 153 to 162 is mixed with an oxidation composition (Ox),
b) said mixture is applied to the keratin-containing fibres directly thereafter, and is left on the fibres for 5 to 60 minutes, and
c) the fibres are then rinsed with water and the bleaching paste is optionally washed out using a surfactant-containing cleansing agent.

176. A method according to Statement 175, wherein the oxidation composition (Ox) contains, in each case in relation to its weight, 50-96 % by weight, preferably 70-93 % by weight, particularly preferably 80-90 % by weight of water and 0.5-20 % by weight of hydrogen peroxide and also at least one pH adjustor in such an amount that the oxidation composition has a pH value in the range of 2.5 to 5.5, measured at 20°C,
177. A method according to Statement 175 or 176, wherein the bleaching paste (B) and the oxidation composition (Ox) are mixed with one another in a weight-based ratio (B):(Ox) of 0.2-1, particularly preferably 0.3-0.8, more preferably 0.4-0.7, exceptionally preferably 0.5-0.6.
178. A multi-component packaging unit (kit-of-parts) for lightening keratinic fibres, in particular human hair, containing at least two components packaged separately from one another, characterised in that

i) the first component (I) is a bleaching paste according to any one of Statements 153 to 162, and
ii) the second component (II) is an oxidation composition (Ox).

179. A multi-component packaging unit (kit-of-parts) according to Statement 178 which contains, in each case in relation to its weight, 50-96 % by weight, preferably 70-93 % by weight, particularly preferably 80-90 % by weight of water and 0.5-20 % by weight of hydrogen peroxide and has a pH value in the range of 2.5 to 5.5, measured at 20° C, wherein the components (I) and (II) are present in a weight-based ratio to one another (I):(II) of (0.7-1.3):(2-3), particularly preferably (0.8-1.2):(2.3:2.7), exceptionally preferably 1:2.
180. A method for changing the colour of keratinic fibres, in particular human hair, in which

a) a bleaching paste according to any one of Statements 153 to 162, is mixed with an oxidation composition (Ox),
b) said mixture is applied to the keratin-containing fibres directly thereafter, is left on the fibres for 5 to 60 minutes, and
c) the fibres are then rinsed with water and the mixture is optionally washed out using a surfactant-containing cleansing agent.

181. A method according to Statement 180, wherein the oxidation composition (Ox) contains, in each case in relation to its weight, 50-96 % by weight, preferably 70-93 % by weight, particularly preferably 80-90 % by weight of water and 0.5-20 % by weight of hydrogen peroxide and also at least one pH adjustor in such an amount that the oxidation composition has a pH value in the range of 2.5 to 5.5, measured at 20°C.
182. A method according to Statement 180 or 181, wherein the bleaching paste and the oxidation composition (Ox) are additionally mixed with an alkalising composition (Alk) which contains water and at least one alkalising agent which is selected from ammonia, alkanolamines and mixtures hereof and has a pH value in the range of 8-12, preferably 9-11, particularly preferably of 9.5-10.5, in each case measured at 20°C.
183. A method according to Statement 182, wherein the bleaching paste (B), the oxidation composition (Ox), and the alkalising composition (Alk) are preferably mixed with one another in a weight-based ratio (B):(Ox):(Alk) of (0.7-1.3):(2-3):(2-3), particularly preferably (0.8-1.2):(2.3-2.7):(2.3-2.7), exceptionally preferably 1:2:2.
184. A multi-component packaging unit (kit-of-parts) for changing the colour of keratinic fibres, in particular human hair, containing at least three components packaged separately from one another, characterised in that

(i) the first component (I) is a bleaching paste according to any one of Statements 153 to 162,
(ii) the second component (II) is an oxidation composition (Ox), and
(iii) the third component (III) is an alkalising composition (Alk).

185. A multi-component packaging unit (kit-of-parts) according to Statement 184, wherein the oxidation composition (Ox) contains, in each case in relation to its weight, 50-96 % by weight, preferably 70-93 % by weight, particularly preferably 80-90 % by weight of water and 0.5-20 % by weight of hydrogen peroxide and has a pH value in the range of 2.5 to 5.5, measured at 20° C, and the alkalising composition (Alk) contains water and at least one alkalising agent, which is selected from ammonia, alkanolamines and mixtures hereof and has a pH value in the range of 8-12, preferably of 9-11, particularly preferably of 9.5-10.5, in each case measured at 20°C, wherein the bleaching paste (B), the oxidation composition (Ox) and the alkalising composition (Alk) are preferably present in a weight-based ratio to one another (B):(Ox):(Alk) of (0.7-1.3):(2-3):(2-3), particularly preferably (0.8-1.2):(2.3:2.7):(2.3-2.7), exceptionally preferably 1:2:2.
186. Use of at least one complexing agent, selected from:

(ii) the first condensate reaction product according to any one of Statements 1 to 92 or the composition as defined in any one of Statements 106 to 115, and
(iii) the second condensation reaction product according to any one of Statements 1 to 76 or any one of Statements 93 to 103 or the composition as defined in any one of Statements 116 to 130,

in a bleaching paste which further contains at least one oxidising agent, for reducing damage to keratinic fibres, in particular human hair, caused by the treatment of these fibres with the bleaching paste.

## EXAMPLES

### Analvtical methods

#### *Molecular weight*

Molecular weight analysis was carried out using both Gel Permeation Chromatography (GPC) and Liquid Chromatography Electrospray Ionization Mass Spectrometry (LC-ESI-MS).

Aqueous gel permeation chromatography (GPC) was used to determine relative molecular weight averages and molecular weight distribution curves. The product samples were dissolved in distilled water and underwent routine filtration. The elution solvent was an aqueous solution of disodium hydrogen phosphate and chromatographed with an RI (Refractive Index) detector at 30°C. The calibration standard for the analysis was polyethylene glycol.

For LC-ESI-MS (Liquid Chromatography Electrospray Ionization Mass Spectrometry), a sample was dissolved in water/acetonitrile. The scanning range was set from 100 to 1250 Da.

As is well understood by the skilled person, the GPC measurement is a relative technique and calibration against the standard is required. Further, as the molecular weight values obtained are not absolute, the skilled person understands that the values determined may require conversion in order to characterise absolute values for weight average molecular weight (Mw) and number average molecular weight (Mn). This conversion is well within the ability of the skilled person. Additional molecular weight analysis (such as LC-ESI-MS) can support this conversion.

For example, in the characterisation of the products of the present invention where the starting materials were itaconic acid and ethylene diamine (in a ratio of 2:1 w/w), relative Mw values in the range of 1,000 to 1,100 were identified, and Mn values in the range of 950 to 1,050 were identified.

The polydispersity is a ratio (Mw/Mn, as is well understood by the skilled person) so can be used to provide an absolute value of a feature of a product using the relative technique of GPC. For example, in the present case, the inventors identified a polydispersity index ranging from about 1.01 to about 1.04 for the hydrolysed and non-hydrolysed products when the starting materials were itaconic acid and ethylene diamine (in a ratio of 2:1 w/w).

This relatively low polydispersity value indicates that the reaction product of the present invention is not a typical mixture of polymers (which would usually exhibit a higher polydispersity index (for example of at least 1.5; typically at least 1.7.

LC-ESI-MS analysis is a technique that characterises the distribution of absolute molecular weight values. The distribution of peaks in the LC-ESI-MS chromatogram supports the relatively low polydispersity index. The analysis of the products of the present invention (both the non-hydrolysed and the hydrolysed) where the starting materials were itaconic acid and ethylene diamine (in a ratio of 2:1 w/w) identified a distribution of peaks with the main peaks in the LC-ESI-MS chromatogram in the region from about 150 m/z to about 600 m/z. By "main peaks" in the context of the present invention is meant the 3 to 5 peaks exhibiting the highest relative abundance. The products of the present invention may thus be characterised as exhibiting at least two out of the three peaks in the LC-ESI-MS chromatogram with the highest relative abundance less than 500 g/mol. LC-ESI-MS also showed a relatively small number of peaks above 500 g/mol, and particularly few above 650 g/mol. For the peaks in the LC-ESI-MS chromatogram at these higher g/mol values, the relative abundance was very low, i.e. less than 10%. Thus, the reaction products of the present invention may be characterised as exhibiting no peaks in the LC-ESI-MS chromatogram above 650 g/mol with relative abundance of at least 10%.

By comparison with LC-ESI-MS values for absolute molecular weight values, the inventors applied a conversion of 1/3 to the relative molecular weight values identified in the GPC (i.e. the scaling down of the GPC values by 1/3).

#### *FTIR*

A sample of the products (in solid, powder form) was used directly for the FTIR measurement.

The measurement was conducted with a Spectrum 100 FTIR from the company Perkin Elmer. Measurements were conducted on the UATR-unit with 8 scans; resolution 4 $cm^{-1}$.

## Testing of hair samples

***Material***

Hair samples: Kerling international European Natural Hair 7/0 (Backnang, Germany)
Hair clamps: plastic tabs, code 900.0320 (Dia-Stron Ltd, UK)/hair clamped with liquid epoxy resin
Devices: Universal-Dimensions-Measuring-Device UDM 5000A, (Zimmer GmbH, Darmstadt, Germany) Stress-Strain-System MTT 680 with control unit UV 1000 (Dia-Stron Ltd, UK)
Software: UvWin 1.32.1000 (Dia-Stron Ltd, UK)

*Treatment*

50 single hair fibers (length between clamps 3cm) were used for each product and for the reference.

The bleaching was performed twice on single hair fiber under the following conditions: 30g of bleaching powder were mixed with 60g developer solution (9% $H_2O_2$).

The hair was soaked in the bleaching mixture for 45 min at 32°C. Afterwards the fibers were rinsed with tap water for 120 seconds. Finally, the fibers were blow-dried for 60 minutes. This procedure was repeated once.

The treated hair fibers were stored for at least 48 hours.

***Measurement** of **hair thickness***

At the beginning of the test the mean cross-sectional area of each single hair was determined at a temperature of 22°C and a relative humidity of 50%. Data thus obtained were used for the stress calculation before and after product application.

***Determination** of **Difference in** E-Modulus **and** break **stress***

The Young's modulus also known as elastic modulus (E-modulus) is defined as the ratio of stress over strain in the Hookean region. Hookean's law of elasticity states that the longitudinal change of a material body (the strain) is linearly related to the force causing the deformation (the stress). For wet hair this region lies between a strain of approximately 0 and 2%. The Young's Modulus is a measure for the strength of a fiber (the higher the Young's Modulus the stronger the fiber).

Before the application of the products: all the hair fibres were soaked in water for at least 1 hour before they were stretched with a constant speed rate of 10 mm/min within the elastic phase (0-1.5% elongation). Afterwards the E-Modulus (= Young's Modulus) was calculated.

After the application of the products: The hair fibers were soaked in water for at least 1 hour. Afterwards they were stretched with a constant speed rate of 10 mm/min within the elastic phase (0-1.5% elongation). The E-Modulus (= Young's Modulus) was calculated. Afterwards they were stretched with a constant speed rate of 10 mm/min up to the break point (i.e. the stress at fibre break point).

The difference in E-modulus before bleaching and after bleaching was calculated. A small difference, i.e. a low E-modulus means that the elasticity of the hair has been less disturbed by bleaching, i.e. the hair has been less damaged. The lower the difference values, the better.

***Cysteic acid test***

In order to measure hair damage induced by the bleaching treatment, the amount of cysteic acid on each treated hair strand was determined by quantitative NIR spectroscopy.

The spectra were recorded with an MPA ™ FT-NIR Spectrometer from Bruker Optik GmbH. The infrared range including the wavenumber range from 12,500 $cm^{-1}$ to 4,000 $cm^{-1}$ was used to characterize the overtone and combination vibrations of e.g. CH, OH and NH groups.

The measurement was performed at six different positions on each hair strand with the integration sphere module in diffuse reflection. For the analysis of the measured NIR spectra, the wavenumber range from 7,300 $cm^{-1}$ to 4,020 $cm^{-1}$ was chosen.

The NIR spectra of cysteine show characteristic absorption bands in the wavenumber range from 6,200 $cm^{-1}$ to 5,500 $cm^{-1}$. If hair is oxidatively damaged (i.e., the level of cysteic acid in the hair increases), the cysteic acid bands at 5020 $cm^{-1}$ to 4020 $cm^{-1}$ in the NIR spectrum will be shown.

Three hair strands were treated and measured for each bleaching procedure (each formulation). The average value was calculated from the eighteen measurements for each formulation. The quantitative evaluation of the spectra was carried out by computer.

### *Colour difference*

To assess the colour loss caused by shampooing, the colour difference ΔE measured on the respective strands was determined. The colour difference, also referred to as dE or ΔE, can readily be determined by colourimetry by employing a colourimeter, via which the colours in the L*,a*,b* colour space were measured, a colourimeter from Datacolour, Type Spectraflash 450 in Firma X-right, Typ exact, for example.
The L*,a*,b* colour space means the CIELAB colour space. The L-value denotes the lightness of the colour (black-white axis); the higher the value for L, the lighter the colour. The a-value denotes the red-green axis of the system; the higher this value, the more the colour is shifted into the red. The b-value denotes the yellow-blue axis of the system; the higher this value, the more the colour is shifted into the yellow.
The colour shift ΔE, i.e. the colour difference between two (hair) colours, for which a L*,a*,b* value combination was determined in each case, is calculated according to the following formula:

$$\Delta E = (\Delta L^2 + \Delta a^2 + \Delta b^2)0.5$$

The higher the value for ΔE, the more pronounced the colour difference, i.e. the higher the amount of colour washed out and the lower the fastness to washing of the dye.

Example 1: Non-hydrolysed condensate reaction product synthesis according to the invention: itaconic acid/ethylene diamine, molar ratio 2:1 itaconic acid:diamine, no solvent; no hydrolysis

Ethylene diamine (20.0 g = 0.34 Mol) was introduced and itaconic acid (88.8 g = 0.70 Mol) was added in portions (very light exothermia). After approximately 60 g of acid was added, a lump was formed in the flask (a paste-like mass). The substance was then gradually heated to 180°C (bath temperature, oil bath), residual acid was added and the reaction mixture stirred for two hours - while distilling off water; then cooled to room temperature and removed from the flask.
Product: 80.1 g yellow, crystalline substance, very hygroscopic and highly water-soluble over the entire pH range. The molecular weight analysis using LC-ESI-MS indicated the absence of any individual products with a molecular weight higher than 1,200 g/mol. FTIR analysis indicated the presence of imide and/or lactam groups (peaks at 1628, 1706 and 1721 $cm^{-1}$) in the compounds contained within the reaction product.

Example 2: Hydrolysed condensate reaction product synthesis according to the invention: itaconic acid/ethylene diamine, molar ratio 2:1 itaconic acid: ED, no solvent: hydrolysis

The product from Example 1 was adjusted to pH=10 with solid NaOH in water and hydrolysed. Water was then distilled off and the substance dried overnight at 80°C in a vacuum. The molecular weight analysis indicated the absence of any individual products with a molecular weight higher than 1,200 g/mol. FTIR analysis indicated the presence of amide groups (peaks at 1563 and 1650 $cm^{-1}$), and the absence of imide groups (no peaks/shoulders around/greater than 1700 $cm^{-1}$) in the compounds contained within the reaction product.

Example 3: Non-hydrolysed condensate reaction product synthesis according to the invention, itaconic acid/pentamethylene diamine ($H_2N(CH_2)_5NH_2$), molar ratio 2:1 itaconic acid: C5-diamine, no solvent; no hydrolysis

Using the same protocol as in Example 1, the non-hydrolysed condensate reaction product was prepared and analysed using molecular weight analysis and FTIR. FTIR analysis indicated the presence of imide and/or lactam groups (peaks at 1628, 1703 and 1721 $cm^{-1}$) in the compounds contained within the reaction product.

Example 4: Hydrolysed condensate reaction product synthesis according to the invention: itaconic acid/pentamethylene diamine, molar ratio 2:1 itaconic acid: C5-diamine, no solvent; hydrolysis

The product from Example 3 was used to prepare the corresponding hydrolysed condensation product, using the same protocol as in Example 2. The resulting product was analysed using molecular weight analysis and FTIR indicated the presence of amide groups (peaks at 1571 and 1656 $cm^{-1}$), and the absence of imide groups (no peaks/shoulders around/greater than 1700 $cm^{-1}$) in the compounds contained within the reaction product.

Example 5: Non-hydrolysed condensate reaction product synthesis according to the invention, itaconic acid/hexamethylene diamine ($H_2N(CH_2)_6NH_2$), molar ratio 2:1 itaconic acid:C6-diamine, no solvent: no hydrolysis

Using the same protocol as in Example 1, the non-hydrolysed condensate reaction product was prepared and analysed using molecular weight analysis and FTIR. FTIR analysis indicated the presence of imide and/or lactam groups (peaks at 1629, 1706 and 1722 $cm^{-1}$) in the compounds contained within the reaction product.

Example 6: Hydrolysed condensate reaction product synthesis according to the invention: itaconic acid/hexamethylene diamine, molar ratio 2:1 itaconic acid:C6-diamine, no solvent: hydrolysis

The product from Example 5 was used to prepare the corresponding hydrolysed condensation product, using the same protocol as in Example 2. The resulting product was analysed using molecular weight analysis and FTIR indicated the presence of amide groups (peaks at 1573 and 1659 $cm^{-1}$), and the absence of imide groups (no peaks/shoulders around/greater than 1700 $cm^{-1}$) in the compounds contained within the reaction product.

Example 7: Formulation test

The condensate reaction products were incorporated in bleaching compositions (a mixture of a Blonde powder and a developer) and various properties thereof tested. The compositions of the present invention were compared with those incorporating conventional complexing agents (EDTA, EDDS and IDS), as well as with a composition comprising a complexing agent consisting of EDTA in combination with a hair damage repair agent.

Developer formulation:

| **Ingredient** | wt% |
|---|---|
| Aqua (Water, Eau) | 69.0 |
| Paraffinum Liquidum (Mineral Oil) | 17.0 |
| Hydrogen Peroxide | 9.0 |
| Cetearyl Alcohol | 3.5 |
| PEG-40 Castor Oil | 0.7 |
| Sodium Cetearyl Sulfate | 0.3 |
| Etidronic Acid | 0.18 |
| Potassium Hydroxide | 0.1 |
| Disodium Pyrophosphate | 0.09 |
| 2,6-Dicarboxypyridine | 0.09 |
| Sodium Benzoate | 0.03 |
| Sodium Sulfate | 0.01 |

Blonde powder:

| **Ingredient** | wt% |
|---|---|
| Potassium Persulfate | 31,70 |
| Sodium Silicate | 27,00 |
| Magnesium Carbonate Hydroxide | 12,40 |
| Ammonium Persulfate | 9,90 |

(continued)

| Ingredient | wt% |
|---|---|
| Aqua (Water, Eau) | 9,00 |
| Paraffinum Liquidum (Mineral Oil) | 4,30 |
| Cellulose Gum | 2,00 |
| Reaction product of the invention or comparative agent | 1,60 |
| Acrylates Copolymer | 1,00 |
| Silica | 0,65 |
| Sodium Hexametaphosphate | 0,20 |
| Potassium Sulfate | 0,20 |
| Ammonium Sulfate | 0,05 |

The following formulations were prepared using a 2:1 (w/w) mixture of the above developer and the above Blonde powder:

Inventive formulation 1: where the complexing agent is the non-hydrolysed product from Example 1
Inventive formulation 2: where the complexing agent is the hydrolysed product from Example 2
Inventive formulation 3: where the complexing agent is the hydrolysed product from Example 4
Inventive formulation 4: where the complexing agent is the hydrolysed product from Example 6
Comparative formulation 1: where the complexing agent is EDTA
Comparative formulation 2: where the complexing agent is EDTA plus a hair damage repair agent
Comparative formulation 3: where the complexing agent is IDS
Comparative formulation 4: where the complexing agent is EDDS

The resulting product has the following composition:

| Ingredient | wt% |
|---|---|
| Aqua (Water, Eau) | 49,00 |
| Paraffinum Liquidum (Mineral Oil) | 12.74 |
| Potassium Persulfate | 10,60 |
| Sodium Silicate | 9,00 |
| Hydrogen Peroxide | 6,00 |
| Magnesium Carbonate Hydroxide | 4,13 |
| Ammonium Persulfate | 3,30 |
| Cetearyl Alcohol | 2,33 |
| Cellulose Gum | 0,67 |
| Reaction product of the invention or comparative agent | 0,53 |
| PEG-40 Castor Oil | 0,47 |
| Acrylates Copolymer | 0,33 |
| Silica | 0,22 |
| Sodium Cetearyl Sulfate | 0,20 |
| Etidronic Acid | 0,12 |
| Potassium Hydroxide | 0,06 |
| Potassium Sulfate | 0,07 |

(continued)

| Ingredient | wt% |
|---|---|
| Sodium Hexametaphosphate | 0,06 |
| 2,6-Dicarboxypyridine | 0,06 |
| Disodium Pyrophosphate | 0,06 |
| Sodium Benzoate | 0,02 |
| Ammonium Sulfate | 0,02 |
| Sodium Sulfate | 0,01 |

Results and Discussion:

With reference to Figure 1, based on the cysteic acid content, the hair strand appears to be significantly less damaged than the formulation with EDDS or IDS with the application of the inventive formulations.

With reference to Figure 2, the colour shift in comparison to the product comprising the hair damage repair agent (using Comparative Formulation 2) cannot be detected by untrained eyes (i.e. the colour shift value is less than 2).

With reference to Figure 3, the lightening effect (L-value) due to the use of the new substances is surprisingly comparable to the product comprising the hair damage repair agent (using Comparative Formulation 2).

Example 8:

The mechanical properties of the hair were measured after bleaching with Inventive formulations 1 and 2, and with Comparative Example 1 (EDTA).

The results in Figure 4 show that the stronger the damage to the hair, the lower the E-modulus value is. So, the hair loses stability through bleaching. The non-hydrolysed product (Inventive formulation 1) shows the best result.

Figure 5 shows the result of the tear. Here the tension has been measured that the hair needs until it is torn. This means that the higher the value, the stronger is the hair. The non-hydrolysed product (Inventive formulation 1) shows the best result.

It will be appreciated that the invention may be modified within the scope of the appended claims.

## Claims

**1.** An adhesive composition comprising a condensate reaction product obtainable according to a process comprising:

(i) reacting itaconic acid or an ester thereof with a diamine of formula $H_2N(CH_2)_nNH_2$, where n is an integer ranging from 2 to 12, wherein when n is an integer ranging from 3 to 12, the alkyl chain is a straight chain, in a molar ratio of diamine to itaconic acid or ester thereof ranging from 1:1.8 to 1:3.0 to form a first reaction mixture;
(ii) heating the first reaction mixture to form a first condensate reaction product in a second reaction mixture; and
(iii) optionally hydrolysing the second reaction mixture to form a second condensate reaction product,

wherein the first condensate reaction product and the second condensate reaction product each independently has a weight average molecular weight ($M_w$) ranging from 200 to 499 g/mol.

**2.** An adhesive composition according to claim 1, wherein either:

itaconic acid is reacted with the diamine in step (i); or
an ester of itaconic acid is reacted with the diamine, wherein the ester of itaconic acid is dimethyl itaconate or diethylitaconate.

**3.** An adhesive composition according to any preceding claim, wherein the diamine is selected from the group consisting of $H_2N-CH_2-CH_2-NH_2$, $H_2N$-(*n*-propyl)-$NH_2$, $H_2N$-(*n*-butyl)-$NH_2$, $H_2N$-(*n*-pentyl)-$NH_2$ and $H_2N$-(*n*-hexyl)-$NH_2$.

**4.** An adhesive composition according to any preceding claim, wherein the molar ratio of diamine to itaconic acid or ester thereof ranges from 1:2 to 1:3.

5. An adhesive composition according to any preceding claim, wherein the first condensate reaction product and/or the second condensate reaction product contains no individual compounds having a molecular weight ($M_w$) greater than 1,300 g/mol.

6. An adhesive composition according to any preceding claim, wherein the first condensate reaction product and/or the second condensate reaction product has a polydispersity index ranging from 1 to 1.25, preferably from 1.01 to 1.10.

7. An adhesive composition according to any preceding claim, wherein the first condensate reaction product and/or the second condensate reaction product comprises a mixture of compounds including one or more condensate compounds, wherein the or each compound independently comprises from 3 to 20 structural units, each unit based on either the itaconic-based starting material or the diamine starting material.

8. An adhesive composition according to any preceding claim, wherein the first and/or second condensate reaction product comprises one or more further compounds within the reaction product, the or each compound having from 5 to 13 structural units.

9. An adhesive composition according to any preceding claim, wherein the first condensate reaction product comprises imide and/or lactam compounds.

10. An adhesive composition according to any preceding claim, wherein the first condensate reaction product comprises one or more compounds with the following chemical structures:

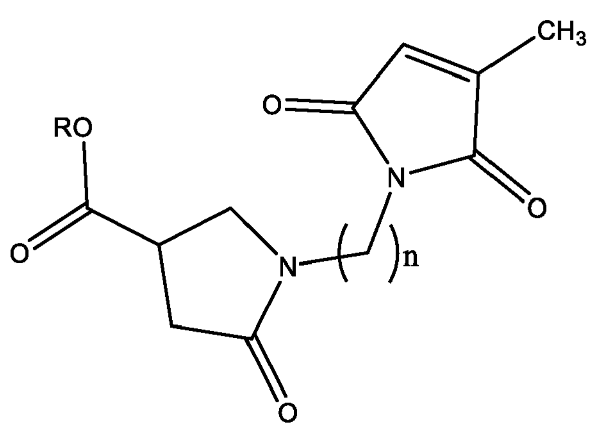

I

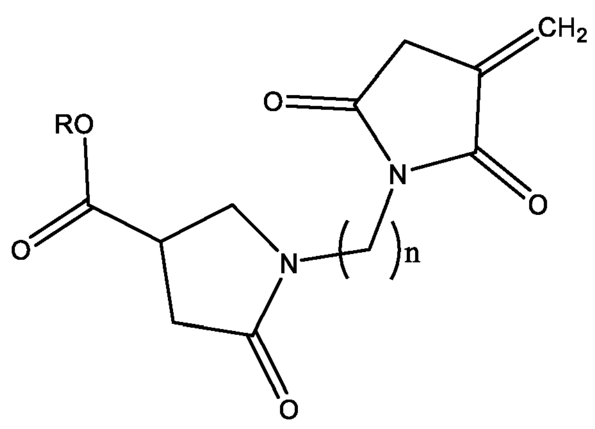

II

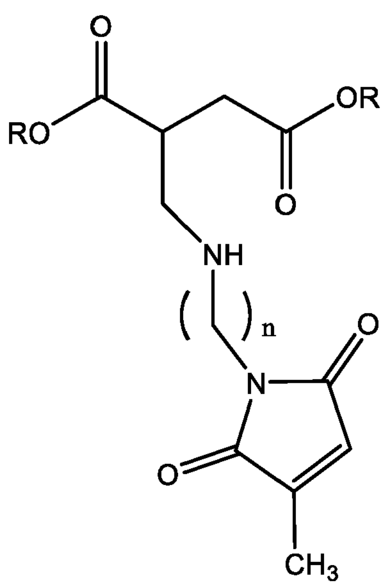

III

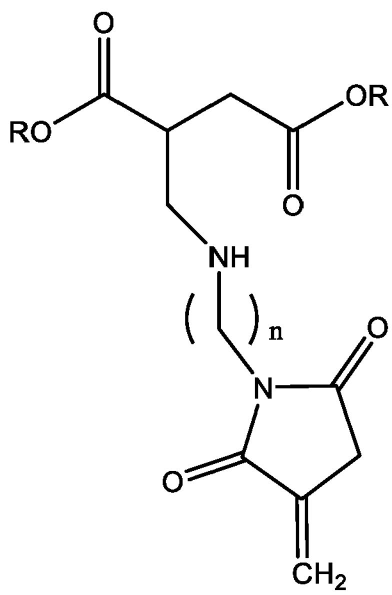

IV

wherein n is as defined in claims 1 or 4 in relation to diamine of formula $H_2N(CH_2)_nNH_2$ and all instances of R are either hydrogen or the alkyl group corresponding to the ester of itaconic acid as defined in claims 1, 2 or 3.

**11.** An adhesive composition according to any one of claims 1 to 10, wherein the second condensate reaction product comprises no imide compounds.

**12.** An adhesive composition according to any one of claims 1 to 10, wherein the second condensate reaction product comprises compounds V and/or VI in the free base form of formula V or as a salt thereof

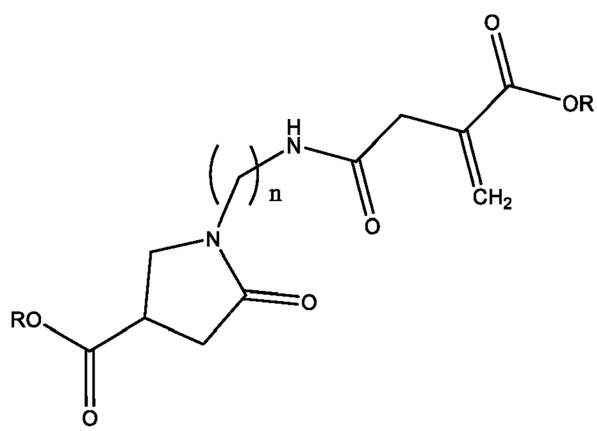

V

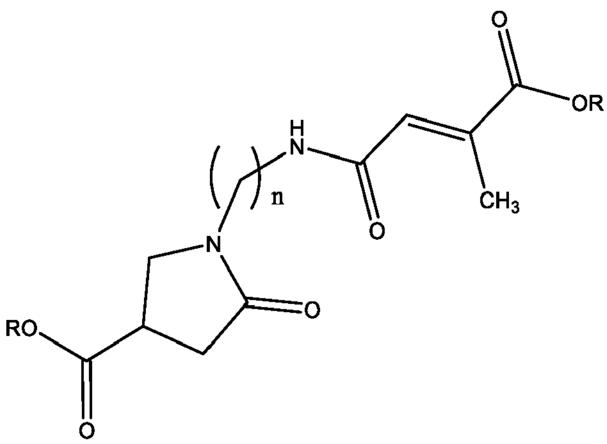

VI

wherein n is as defined in claims 1 or 4 in relation to diamine of formula $H_2N(CH_2)_nNH_2$ and all instances of R are either hydrogen or the alkyl group corresponding to the ester of itaconic acid as defined in claims 1, 2 or 3.

**13.** An adhesive composition according to any preceding claim comprising the first condensate reaction product.

**14.** An adhesive composition according to any preceding claim comprising the second condensate reaction product.

Fig. 1

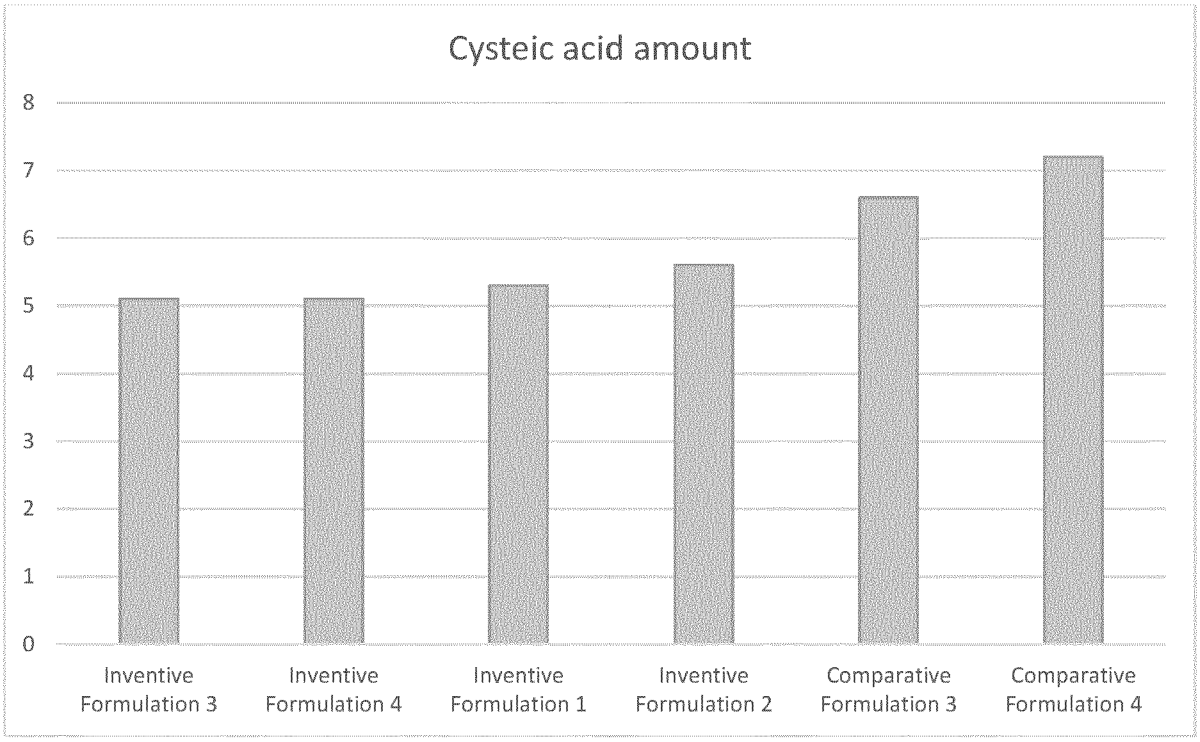
Cysteic acid amount
8
7
6
5
4
3
2
1
0
Inventive Formulation 3
Inventive Formulation 4
Inventive Formulation 1
Inventive Formulation 2
Comparative Formulation 3
Comparative Formulation 4

Fig. 2

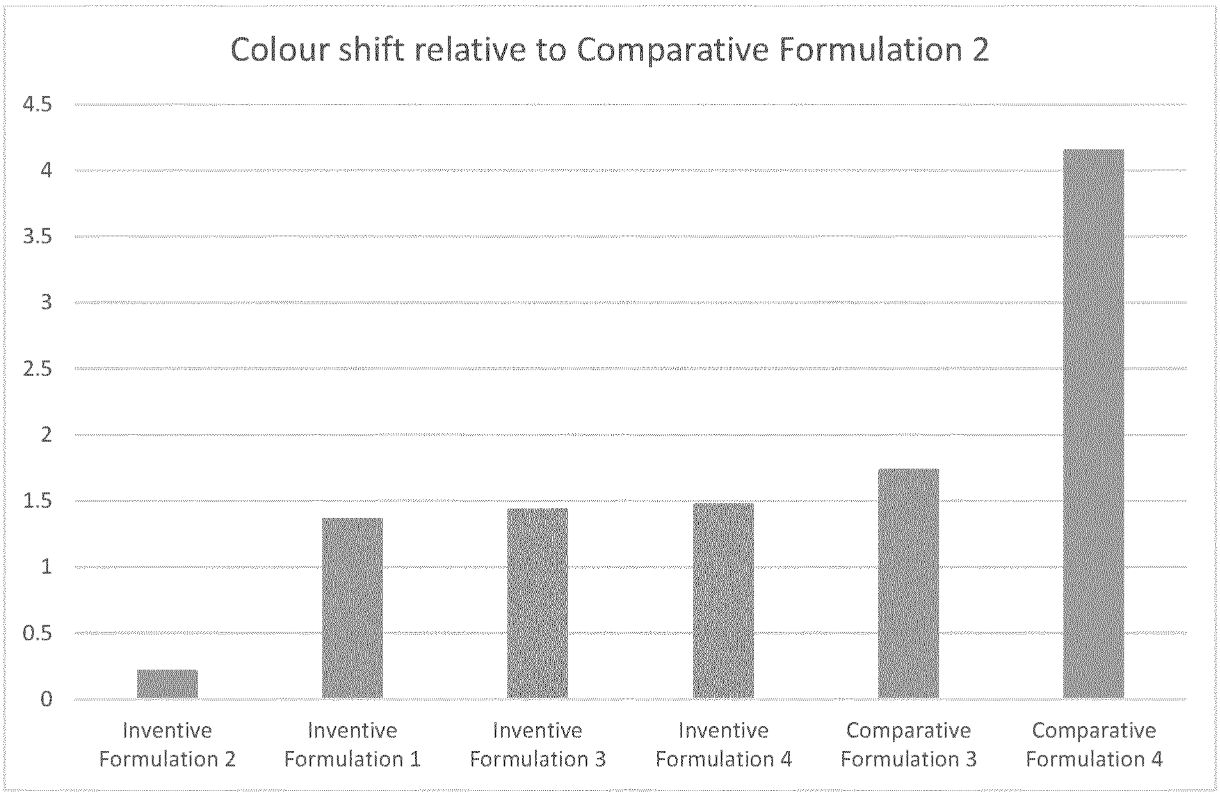
Colour shift relative to Comparative Formulation 2
4.5
4
3.5
3
2.5
2
1.5
1
0.5
0
Inventive Formulation 2
Inventive Formulation 1
Inventive Formulation 3
Inventive Formulation 4
Comparative Formulation 3
Comparative Formulation 4

Fig. 3

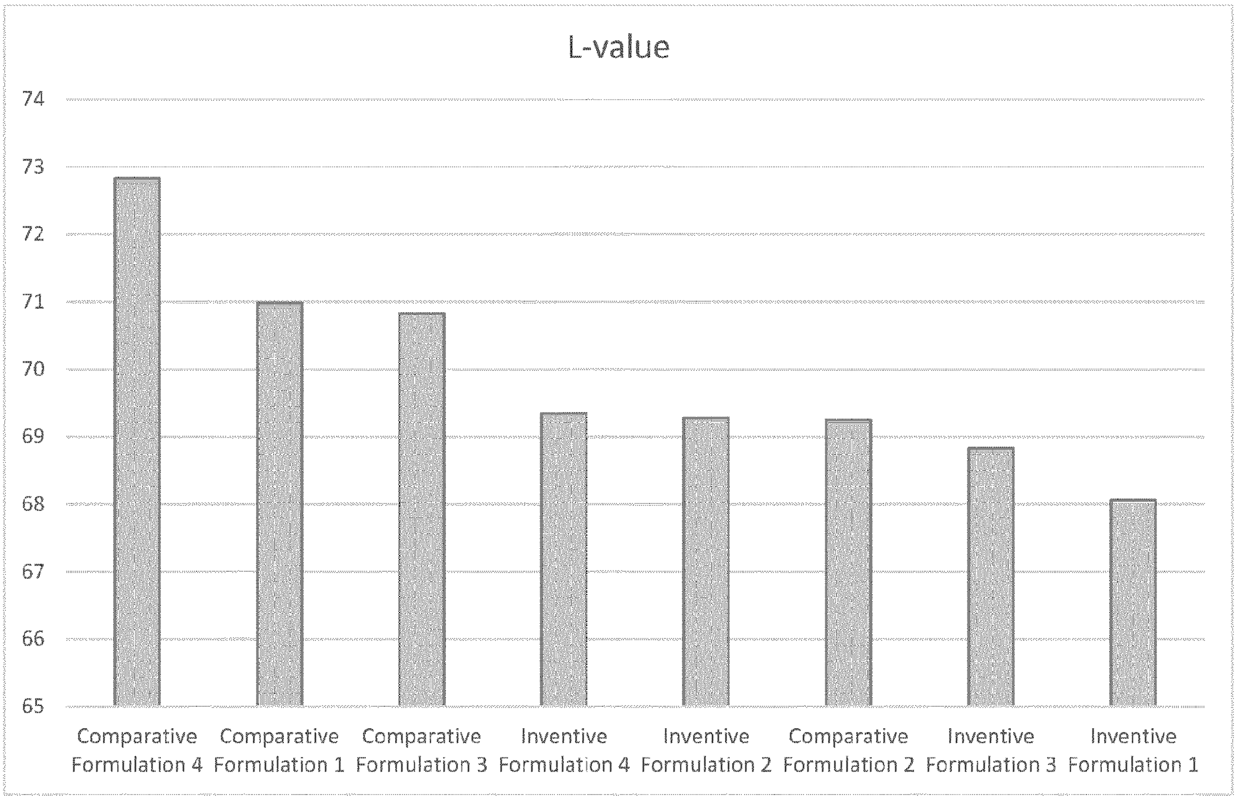
L-value
74
73
72
71
70
69
68
67
66
65
Comparative Formulation 4
Comparative Formulation 1
Comparative Formulation 3
Inventive Formulation 4
Inventive Formulation 2
Comparative Formulation 2
Inventive Formulation 3
Inventive Formulation 1

Fig. 4

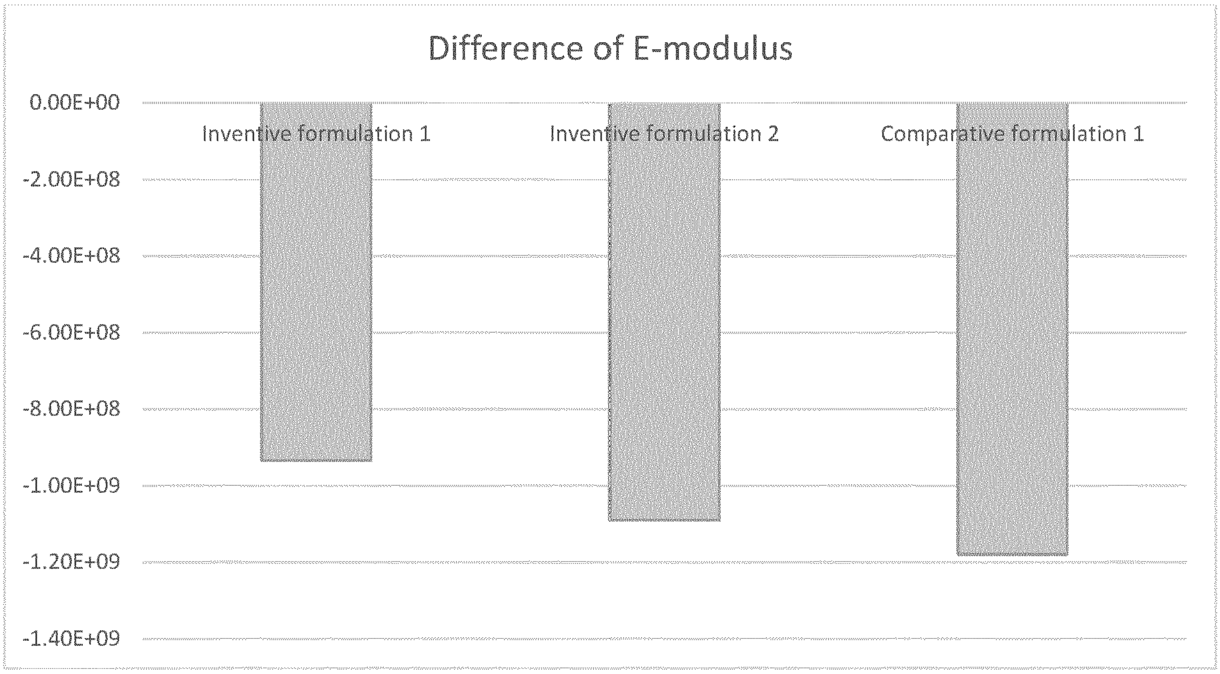
Difference of E-modulus
0.00E+00
-2.00E+08
-4.00E+08
-6.00E+08
-8.00E+08
-1.00E+09
-1.20E+09
-1.40E+09
Inventive formulation 1
Inventive formulation 2
Comparative formulation 1

Fig. 5

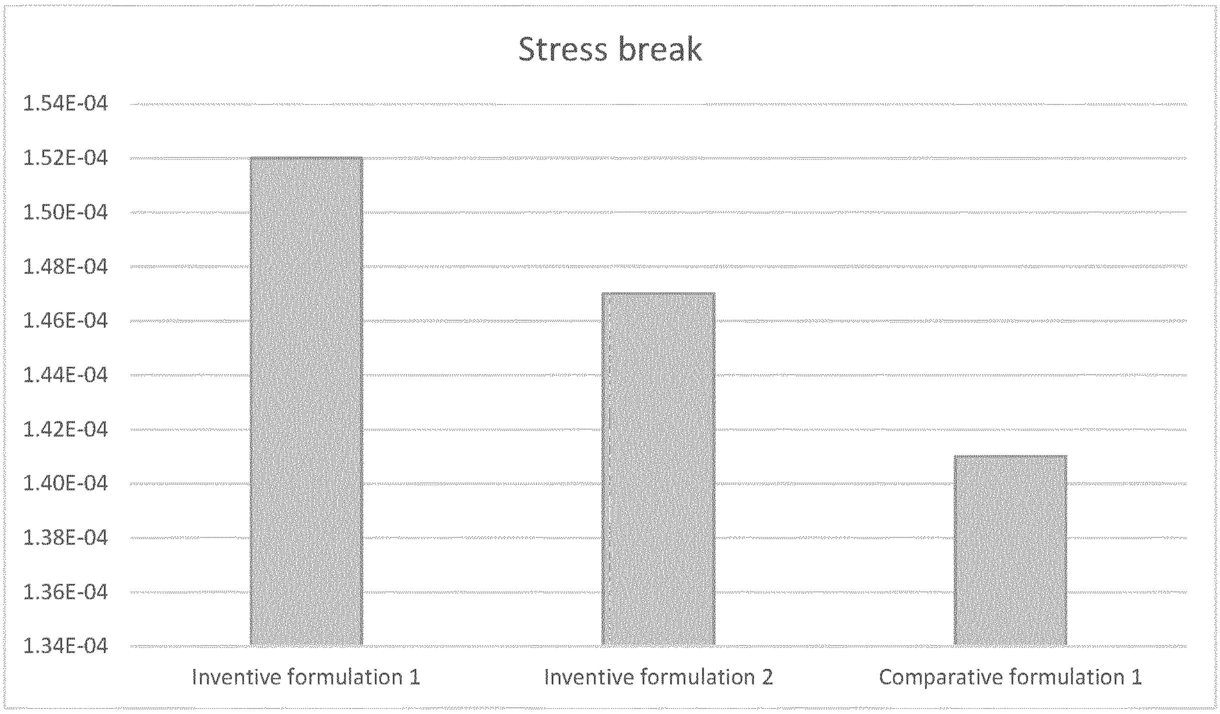
Stress break
1.54E-04
1.52E-04
1.50E-04
1.48E-04
1.46E-04
1.44E-04
1.42E-04
1.40E-04
1.38E-04
1.36E-04
1.34E-04
Inventive formulation 1
Inventive formulation 2
Comparative formulation 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015164598 A1
- WO 2015164601 A1
- WO 2016040962 A1
- WO 9822478 A1
- DE 19756454 A1